# EUROPEAN PATENT SPECIFICATION

(11) **EP 1 688 418 B1**
(45) Date of publication and mention of the grant of the patent: **15.03.2017**
(21) Application number: 06004401.3
(22) Date of filing: 13.11.2002
(51) Int. Cl.: C07D 491/04, C07D 497/04, C07D 223/20, C07D 313/12, C07D 337/12, A61P 1/00, A61K 31/4353, A61K 31/55, C07D 313/00, C07D 221/00, C07D 471/04, C07D 223/00, C07D 519/00, C07D 491/00, C07D 513/04, C07D 337/00, C07D 451/00

(54) **Chemokine receptor antagonists and methods of use thereof**
Chemokine Rezeptor Antagonisten und Methoden zu deren Anwendung
Antagonistes de récepteur de chemokine et leurs procédés d'utilisation

(30) Priority: 21.11.2001 US 989086
(43) Date of publication of application: 09.08.2006
(62) Divisional of application: 02789725.5
(73) Proprietor: MILLENNIUM PHARMACEUTICALS, INC., Cambridge, MA 02139 (US); Kyowa Hakko Kirin Co., Ltd., Tokyo 100-8185 (JP)
(72) Inventor: Luly, Jay R., Wellesley, MA 02481 (US); Nakasato, Yoshisuke, Susono-shi Shizuoka (JP); Ohshima, Etsuo, Nagareyama-shi Chiba (JP); Harriman, Geraldine C. B., Brookline, Massachusetts 02445 (US); Carson, Kenneth G., Princeton, New Jersey 08540 (US); Ghosh, Shomir, Brookline, MA 02446 (US); Elder, Amy M., Arlington, MA 02474 (US); Mattia, Karen M., Marlborough, MA 01752 (US)
(74) Representative: Keen, Celia Mary

(56) References cited:
- WO-A-01/09138
- WO-A-96/31469
- WO-A-99/37651

## Description

### BACKGROUND OF THE INVENTION

Chemoattractant cytokines or chemokines are a family of proinflammatory mediators that promote recruitment and activation of multiple lineages of leukocytes and lymphocytes. They can be released by many kinds of tissue cells after activation. Continuous release of chemokines at sites of inflammation mediates the ongoing migration of effector cells in chronic inflammation. The chemokines characterized to date are related in primary structure. They share four conserved cysteines, which form disulfide bonds. Based upon this conserved cysteine motif, the family is divided into two main branches, designated as the C-X-C chemokines (α-chemokines), and the C-C chemokines (β-chemokines), in which the first two conserved cysteines are separated by an intervening residue, or adjacent respectively (Baggiolini, M. and Dahinden, C. A., Immunology Today, 15:127-133 (1994)).

The C-X-C chemokines include a number of potent chemoattractants and activators of neutrophils, such as interleukin 8 (IL-8), PF4 and neutrophil-activating peptide-2 (NAP-2). The C-C chemokines include RANTES (Regulated on Activation, Normal T Expressed and Secreted), the macrophage inflammatory proteins 1α and 1β (MIP-1α and MIP-1β), eotaxin and human monocyte chemotactic proteins 1-3 (MCP-1, MCP-2, MCP-3), which have been characterized as chemoattractants and activators of monocytes or lymphocytes but do not appear to be chemoattractants for neutrophils. Chemokines, such as RANTES and MIP-1α, have been implicated in a wide range of human acute and chronic inflammatory diseases including respiratory diseases, such as asthma and allergic disorders.

The chemokine receptors are members of a superfamily of G protein-coupled receptors (GPCR) which share structural features that reflect a common mechanism of action of signal transduction (Gerard, C. and Gerard, N.P., Annu Rev. Immunol, 12:775-808 (1994); Gerard, C. and Gerard, N. P., Curr. Opin. Immunol., 6:140-145 (1994)). Conserved features include seven hydrophobic domains spanning the plasma membrane, which are connected by hydrophilic extracellular and intracellular loops. The majority of the primary sequence homology occurs in the hydrophobic transmembrane regions with the hydrophilic regions being more diverse. The first receptor for the C-C chemokines that was cloned and expressed binds the cheinokines MIP-1α and RANTES. Accordingly, this MIP-1α/RANTES receptor was designated C-C chemokine receptor 1 (also referred to as CCR-1; Neote, K., et al., Cell, 72:415-425 (1993); Horuk, R et al., WO 94/11504, May 26, 1994; Gao, J.-I. et al., J Exp. Med., 177:1421-1427 (1993)). Three receptors have been characterized which bind and/or signal in response to RANTES: CCR3 mediates binding and signaling of chemokines including eotaxin, RANTES, and MCP-3 (Ponath et al., J. Exp. Med., 183:2437 (1996)), CCR4 binds chemokines including RANTES, MIP-1α, and MCP-1 (Power, et al., J. Biol. Chem., 270:19495 (1995)), and CCR5 binds chemokines including MIP-1α, RANTES, and MIP-1β (Samson, et al., Biochem. 35: 3362-3367 (1996)). RANTES is a chemotactic chemokine for a variety of cell types, including monocytes, eosinophils, and a subset of T-cells. The responses of these different cells may not all be mediated by the same receptor, and it is possible that the receptors CCR1, CCR4 and CCR5 will show some selectivity in receptor distribution and function between leukocyte types, as has already been shown for CCR3 (Ponath *et al.*). In particular, the ability of RANTES to induce the directed migration of monocytes and a memory population of circulating T-cells (Schall, T. et al., Nature, 347:669-71 (1990)) suggests this chemokine and its receptor(s) may play a critical role in chronic inflammatory diseases, since these diseases are characterized by destructive infiltrates of T cells and monocytes.

Many existing drugs have been developed as antagonists of the receptors for biogenic amines, for example, as antagonists of the dopamine and histamine receptors. No successful antagonists have yet been developed to the receptors for the larger proteins such as chemokines and C5a. Small molecule antagonists of the interaction between C-C chemokine receptors and their ligands, including RANTES and MIP-1α, would provide compounds useful for inhibiting harmful inflammatory processes "triggered" by receptor ligand interaction, as well as valuable tools for the investigation of receptor-ligand interactions.
WO 01/09138 A2 describes a series of compounds which are useful for treating a disease associated with aberrant leukocyte recruitment and/or activation. In this regard, the compounds represent a class of small organic molecules which are antagonists of chemokine receptor function and which can inhibit leukocyte activation and/or recruitment.
WO 96/31469 describes N-substituted azaheterocyclic carboxcyclic acids and esters thereof in which a substituted alkyl chain forms part of the N-substituent or salts thereof. Such compounds are said to be useful in the clinical treatment of painful, hyperalgesic and/or inflammatory conditions in which C-fibres play a pathopsychological role by eliciting neurogenic pain or inflammation.

### SUMMARY OF THE INVENTION

It has now been found that a class of small organic molecules are antagonists of chemokine receptor function and can inhibit leukocyte activation and/or recruitment. An antagonist of chemokine receptor function is a molecule which can inhibit the binding and/or activation of one or more chemokines, including C-C chemokines such as RANTES, MIP-1α, MCP-2, MCP-3 and MCP-4 to one or more chemokine receptors on leukocytes and/or other cell types. As a consequence, processes and cellular responses mediated by chemokine receptors can be inhibited with these small organic molecules. Based on this discovery, the treatment of a disease associated with aberrant leukocyte recruitment and/or activation is disclosed as well the treatment of a disease mediated by chemokine receptor function. The treatment comprises administering to a subject in need an effective amount of a compound or small organic molecule which is an antagonist of chemokine receptor function. Compounds or small organic molecules which have been identified as antagonists of chemokine receptor function are discussed in detail hereinbelow, and can be used for the manufacture of a medicament for treating or for preventing a disease associated with aberrant leukocyte recruitment and/or activation. In one aspect, the compound has the formula: 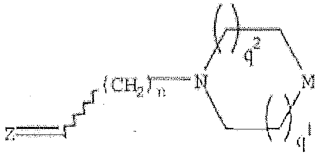 or a physiologically acceptable salt thereof, wherein Z, n, M, q¹ and q² are as described herein.

The invention also relates to the disclosed compounds and small organic molecules for use in treating or preventing a disease associated with aberrant leukocyte recruitment and/or activation. The invention also includes pharmaceutical compositions comprising one or more of the compounds or small organic molecules which have been identified herein as antagonists of chemokine function and a suitable pharmaceutical carrier. The invention further relates to novel compounds which can be used to treat an individual with a disease associated with aberrant leukocyte recruitment and/or activation and methods for their preparation.

Thus, the present invention provides a compound having the formula: 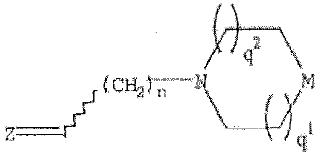 or physiologically acceptable salt thereof, wherein:
n is an integer from one to four;
M is >CR¹R²;
q¹ is zero;
q² is one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, a halogen, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group,
-O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a nonaromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is:

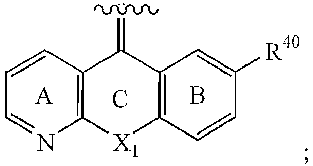

X₁ is -S-, -CH₂-, -CH₂-CH₂-, -CH₂-S-, -S-CH₂-, -O-CH₂-, -CH₂-O-, -NR_{c}-CH₂-, -CH₂-NR_{c}-, -SO-CH₂-, -CH₂-SO-, -S(O)₂-CH₂-, -CH₂-S(O)₂-, -CH=CH-, -NR_{c}-CO-, a bond, -O-, or -CO-NR_{c}-;
R_{c} is -H, an aliphatic group, a substituted aliphatic group, an aromatic group, a substituted aromatic group, a benzyl group or a substituted benzyl group;
said acyl group is an aliphatic carbonyl, aromatic carbonyl, aliphatic sulfonyl or aromatic sulfonyl
said aliphatic group is a C₁-C₆ alkyl, alkenyl or alkynyl;
said aromatic group is selected from the group consisting of phenyl, 1-naphthyl, 2-naphthyl, 1-anthracyl, 2-anthracyl, *N*-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl, 5-oxazolyl, tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 1-isoquinolinyl, 3-quinolinyl, 1-isoindolyl, 3-isoindolyl, acridinyl, 3-benzisoxazolyl, benzocyclopentyl, and benzocyclohexyl;
said non-aromatic heterocyclic group is a five to eight-membered nonaromatic ring which contains one or more heteroatoms independently selected from the group consisting of nitrogen, oxygen and sulfur;
said substituted aliphatic group is substituted with one or more substituents selected from the group consisting of oxo group, epoxy group, non-aromatic heterocyclic ring, benzyl group, substituted benzyl group, aromatic group or substituted aromatic group, electron withdrawing group, halo, azido, -CN, -CONR²⁴R²⁵, -NR²⁴R²⁵, -OS(O)₂NR²⁴R²⁵, -S(O)₂NR²⁴R^{25,} -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(nonaromatic heterocyclic group) and -Q-(CH₂)ₚ-(non-aromatic heterocyclic group);
said substituted non-aromatic heterocyclic ring is substituted with one or more substituents selected from the group consisting of =O, =S, electron withdrawing group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said substituted aromatic group and substituted benzyl group are substituted with one or more substituents selected from the group consisting of electron withdrawing group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)p-(non-aromatic heterocyclic group);
said electron withdrawing group is alkylimine, alkylsulfonyl, carboxamido, carboxylic alkyl esters, -CH=NH, or -NO₂;
Q is -O-, -S-, -S(O)-, -S(O)₂-, -OS(O)₂-, -C(O)-, -OC(O)-, -C(O)O-, -C(O)C(O)-O-, -O-C(O)C(O)-, -NHC(O)-, -OC(O)NH-, -NH-C(O)-NH-, -S(O)₂NH-, -NHS(O)₂-, -C(NR²³)NHNH-, -NHNHC(NR²³)-, -NR²⁴C(O)- or -NR²⁴S(O)₂-;
R²⁰, R²¹ and R²² are independently -H, an aliphatic group, an aromatic group, a non-aromatic heterocyclic group, -NHC(O)-O-(aliphatic group), -NHC(O)-O-(aromatic group) or -NHC(O)-O-(non-aromatic heterocyclic group) or R²¹ and R²², taken together with the nitrogen atom to which they are bonded, form a substituted or unsubstituted non-aromatic heterocyclic ring;
R²³ is -H, an aliphatic group, a benzyl group, an aryl group or non-aromatic heterocyclic group;
R²⁴ and R²⁵ are independently -H, -OH, an aliphatic group, a substituted aliphatic group, a benzyl group, an aryl group, non-aromatic heterocyclic group or R²⁴ and R²⁵ taken together with the nitrogen atom to which they are bonded can form a substituted or unsubstituted non-aromatic heterocyclic ring;
R⁶⁰ is a -H, -OH, -NH₂, an aromatic group or a substituted aromatic group;
t is zero to three;
u is zero to three;
p is one to five; and
R⁴⁰ is selected from the group consisting of

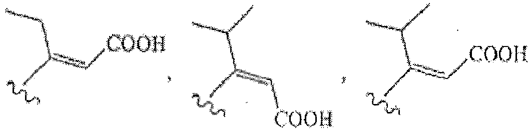

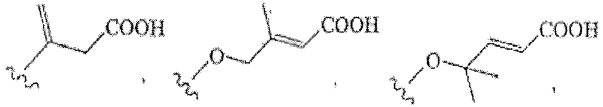

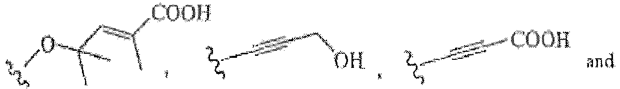

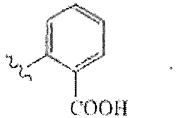

Preferably, R¹ is -H or -OH and R² is a substituted aromatic group. It is further preferred that R² is phenyl substituted with a halogen. It is additionally preferred that R² is 4-chlorophenyl. It is in particular preferred that n is 2, X₁ is -CH₂-O-, and R¹ is -OH.

Also provided is a pharmaceutical composition comprising a compound of the invention and a physiologically acceptable carrier.

Further provided is a compound of the invention for use in treating a disease selected from the group consisting of arthritis, atherosclerosis, arteriosclerosis, restenosis, ischemia/reperfusion injury, diabetes mellitus, psoriasis, multiple sclerosis, inflammatory bowel diseases, rejection of a transplanted organ or tissue, graft versus host disease, allergy and asthma.

### BRIEF DESCRIPTION OF THE DRAWINGS

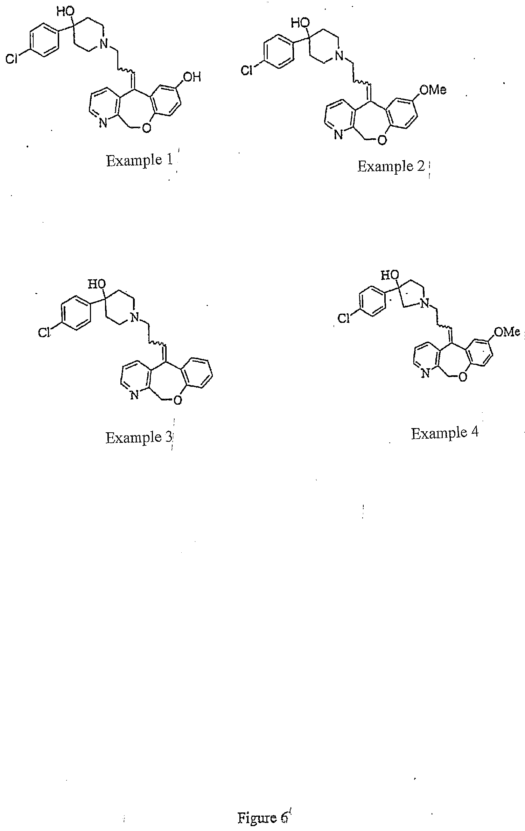

Figure 1 is a schematic showing the preparation, of the compounds represented by Structural Formula (I).
Figure 2 is a schematic showing the preparation of the compounds represented by Compound (VI-b).
Figure 3 is a schematic showing the preparation of the compounds represented by Structural Formula (I)
Figure 4 is a schematic showing the preparation of the compounds represented by Structural Formula (I), wherein
   Ring A and/or Ring B in Z is substituted with R⁴⁰.
Figure 5 is a schematic showing the preparation of the compounds represented by Structural Formula (I),
   wherein Ring A and/or Ring B in Z is substituted with -(O)ᵤ-(CH₂)ₜ-COOR²⁰, -(O)ᵤ-(CH₂)₁-OC(O)R²⁰, -(O)ᵤ-(CH₂)ₜ-C(Q)-NR²¹R²² or -(O)ᵤ-(CH₂)ₜ-NHC(O)O-R²⁰
Figure 6 shows the structures of exemplary compounds.
Figure 7 shows the preparation of compounds represented by Structural Formula (I), wherein Ring
   A or Ring B in Z is substituted with R⁴⁰.
Figure 8A is a schematic showing the preparation of 4-(4-chlorophenyl)-4-fluoropiperidine.
Figure 8B is a schematic showing the preparation of 4-4-azido-4-(4-chlorophenyl)piperidine.
Figure 8C is a schematic showing the preparation of 4-(4-chlorophenyl)-4-methylpiperidine.
Figure 9A is a schematic showing the preparation of compounds
   wherein R¹ is an amine.
Figure 9B is a schematic showing the preparation of compounds
   wherein R¹ is an alkylamine.
Figure 9C is a schematic showing the preparation of 2-(4-chlorophenyl)-1-(*N-*methyl)ethylamine.
Figure 9D is a schematic showing the preparation of 3-(4-chlorophenyl)-3-chloro-1-hydroxypropane.
Figure 9E is a schematic showing the preparation of 3-(4-chlorophenyl)-1-*N-*methylaminopropane.
Figure 10A is a schematic showing the preparation of 3-(4-chlorophenyl)-3-hydroxyl-3-methyl-1-*N*-methylaminoprapane.
Figure 10B is a schematic showing the preparation of 1-(4-chlorobenzoyl)-1,3-propylenediamine.
Figure 10C is a schematic showing three procedures for the preparation of compounds represented by Structural Formula (VII)
   wherein Ring A or Ring B
   in Z is substituted with R⁴⁰. In Figure 10C, R⁴⁰ is represented by -(O)ᵤ-(CH₂)ₜ-C(O)-NR²¹R²², u is one, t is zero.
Figure 10D is a schematic showing the preparation of 4-(4-chlorophenyl)-4-pyridine.
Figure 11 shows the structure of an exemplary compound.
Figure 12 is a schematic showing preparation of compounds of formula (VIc).
Figure 13 is a schematic showing preparation of compounds of formula (VIe).
Figure 14 is a schematic showing a procedure for the preparation of compounds of formula (I-f).
Figure 15 is a schematic showing a procedure for the preparation of Examples 5, 17, 6 and 10.

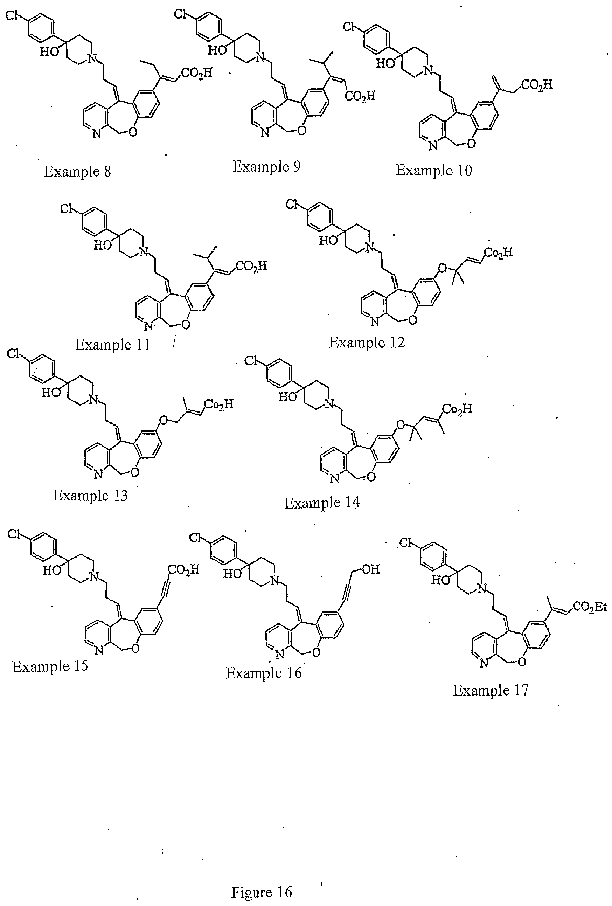

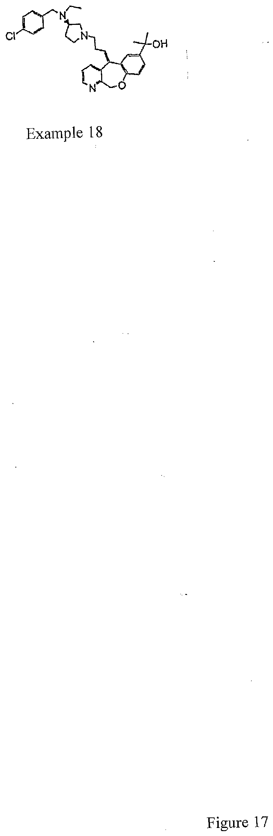

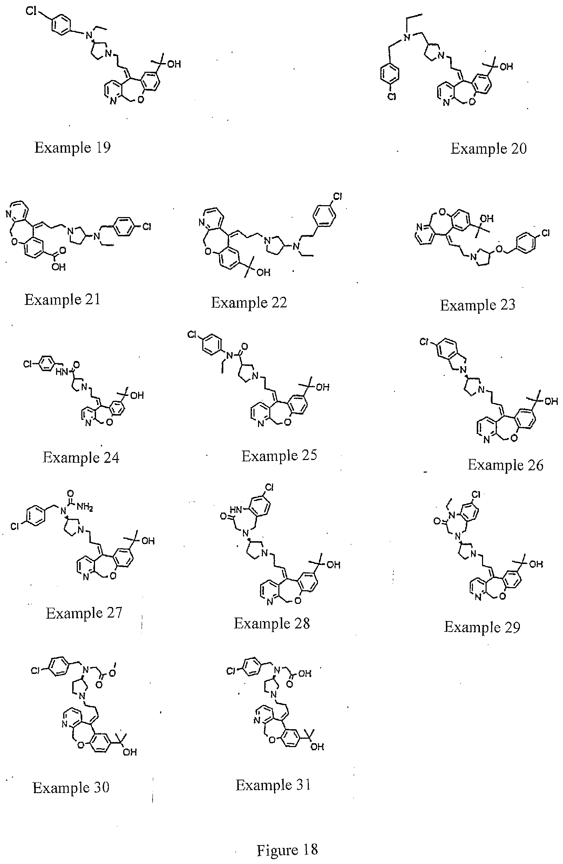

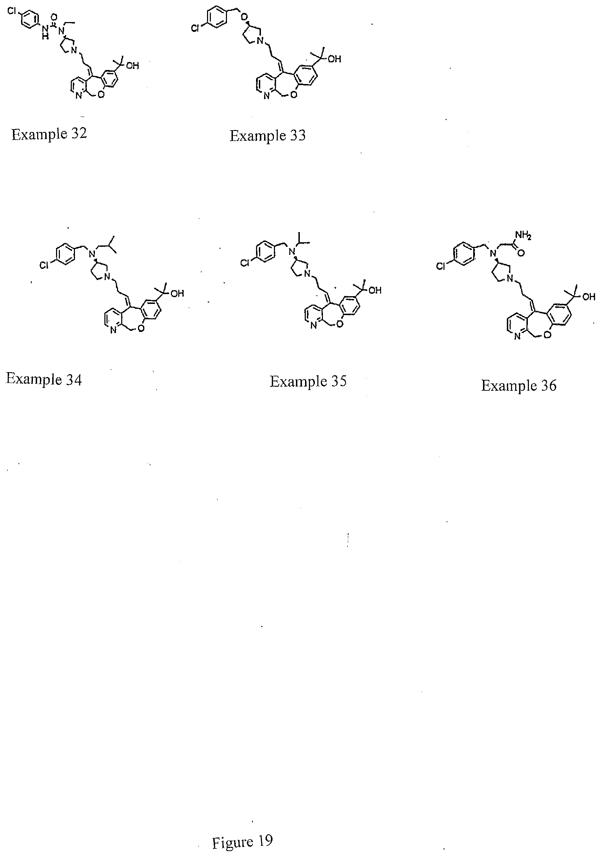

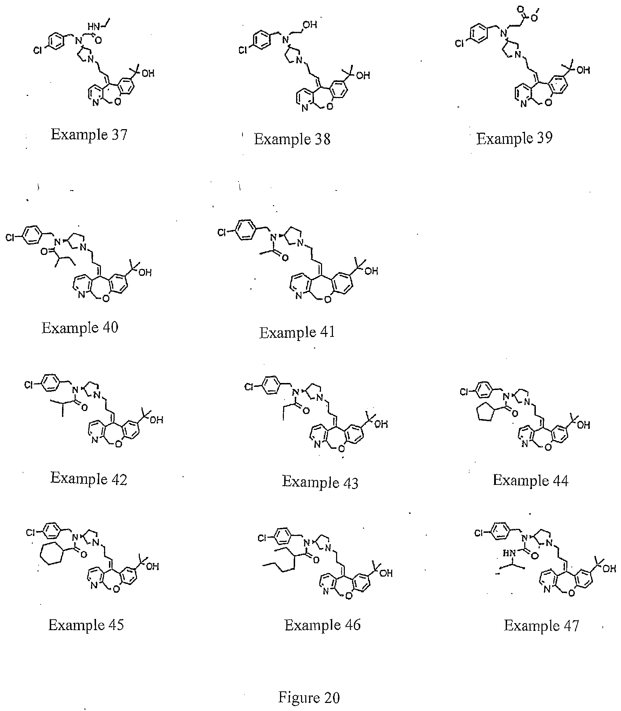

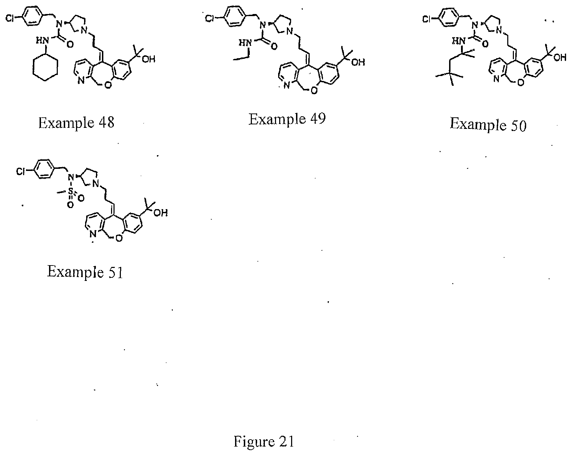

Figures 16-21 show the structures of exemplary compounds.

### DETAILED DESCRIPTION OF THE INVENTION

The present invention relates to small molecule compounds which are modulators of chemokine receptor function. In a preferred embodiment, the small molecule compounds are antagonists of chemokine receptor function. Accordingly, processes or cellular responses mediated by the binding of a chemokine to a receptor can be inhibited (reduced or prevented, in whole or in part), including leukocyte migration, integrin activation, transient increases in the concentration of intracellular free calcium [Ca⁺⁺]ᵢ, and/or granule release of proinflammatory mediators.

The invention further relates to compounds for the treatment including prophylatic and therapeutic treatments, of a disease associated with aberrant leukocyte recruitment and/or activation or mediated by chemokines or chemokine receptor function, including chronic inflammatory disorders characterized by the presence of RANTES, MIP-1α, MCP-2, MCP-3 and/or MCP-4 responsive T cells, monocytes and/or eosinophils, including but not limited to diseases such as arthritis (e.g., rheumatoid arthritis), atherosclerosis, arteriosclerosis, restenosis, ischemia/reperfusion injury, diabetes mellitus (e.g., type 1 diabetes mellitus), psoriasis, multiple sclerosis, inflammatory bowel diseases such as ulcerative colitis and Crohn's disease, rejection of transplanted organs and tissues (i.e., acute allograft rejection, chronic allograft rejection), graft versus host disease, as well as allergies and asthma. Other diseases associated with aberrant leukocyte recruitment and/or activation which can be treated (including prophylactic treatments) with the methods disclosed herein are inflammatory diseases associated with Human Immunodeficiency Virus (HIV) infection, e.g., AIDS associated encephalitis, AIDS related maculopapular skin eruption, AIDS related interstitial pneumonia, AIDS related enteropathy, AIDS related periportal hepatic inflammation and AIDS related glomerulo nephritis. The method comprises administering to the subject in need of treatment an effective amount of a compound (i.e., one or more compounds) which inhibits chemokine receptor function, inhibits the binding of a chemokine to leukocytes and/or other cell types, and/or which inhibits leukocyte migration to, and/or activation at, sites of inflammation.

The invention further relates to compounds for use in methods of antagonizing a chemokine receptor, such as CCR1, in a mammal comprising administering to the mammal a compound as described herein.

According to the method, chemokine-mediated chemotaxis and/or activation of pro-inflammatory cells bearing receptors for chemokines can be inhibited. As used herein, "pro-inflammatory cells" includes but is not limited to leukocytes, since chemokine receptors can be expressed on other cell types, such as neurons and epithelial cells.

While not wishing to be bound by any particular theory or mechanism, it is believed that compounds of the invention are antagonists of the chemokine receptor CCR1, and that therapeutic benefits derived from the method of the invention are the result of antagonism of CCR1 function. Thus, the method and compounds of the invention can be used to treat a medical condition involving cells which express CCR1 on their surface and which respond to signals transduced through CCR1, as well as the specific conditions recited above.

In one embodiment, the antagonist of chemokine activity can be represented by Structural Formula (VII): 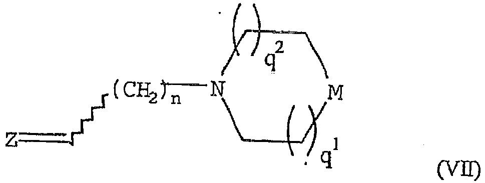 and physiologically acceptable salts thereof.
q¹ is zero, and q² is one. The ring containing M can be substituted or unsubstituted.
Z is represented by Structural Formula (VI): 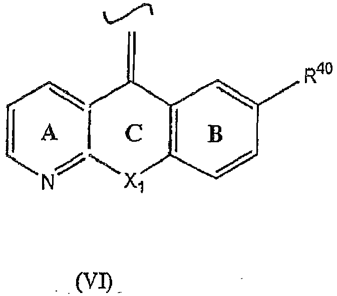
   X₁ is a bond, -O-, -S-, -CH₂ , -CH₂-CH₂-, -CH₂-S-, -S-CH₂-,-O-CH₂-, -CH₂-O-, -NR_{c}-CH₂-, -CH₂-NR_{c}-, -SO-CH₂-, -CH₂-SO-, -S(O)₂-CH₂-, -CH₂-S(O)₂-, -CH=CH-, NR_{c}-CO- or -CO-NR_{c}-. Preferably X₁ is -CH₂-O-, -CH₂-CH₂-, -CH₂-S- , -NR-CO- or -CO-NR_{c}-.
   Preferably X₁ is -CH₂-O-, -CH₂-CH₂- or -CH₂-S-.
n is an integer from one to four. Preferably, n is one, two or three. More preferably n is two. In alternative embodiments, other aliphatic or aromatic spacer groups (L) can be employed for (CH₂)ₙ.
M is >CR¹R². M is preferably >C(OH)R².
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴; or R¹ can be a covalent bond between the ring atom at M and an adjacent carbon atom in the ring which contains M. R¹ is preferably -H or -OH.
R² is -OH, a halogen, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group), -O-(substituted or unsubstituted aliphatic group) or -C(O)-(substituted or unsubstituted aromatic group) or -C(O)-(substituted or unsubstituted aliphatic group). R² is preferably an aromatic group or a substituted aromatic group.
R³, R⁴, R⁵ and R⁶ are independently -H, an acyl group_{;} a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group.
R¹ and R², R³ and R⁴, or R⁵ and R⁶ taken together with the atom to which they are bonded, can alternatively form a substituted or unsubstituted non-aromatic carbocyclic or heterocyclic ring.
R_{c} is hydrogen, an aliphatic group, a substituted aliphatic group, an aromatic group, a substituted aromatic group, a benzyl group or a substituted benzyl group.

In one example, R_{c} is -(CH₂)ₛ-COOR³⁰, -(CH₂)ₛ-C(O)-NR³¹R³² or -(CH₂)ₛ-NHC(O)-O-R³⁰, wherein s is an integer, such as an integer from one to three;

R³⁰, R³¹ and R³² are independently -H, an aliphatic group, a substituted aliphatic group, an aromatic group, a substituted aromatic group or a non-aromatic heterocyclic group. Alternatively, R³¹ and R³², taken together with the nitrogen atom to which they are bonded, form a non-aromatic heterocyclic ring.

In embodiments where M is >CR¹R² and R¹ is a covalent bond between the carbon atom at M and an adjacent carbon atom in the ring which contains M, the antagonist of chemokine function can be represented by Structural Formula (la). 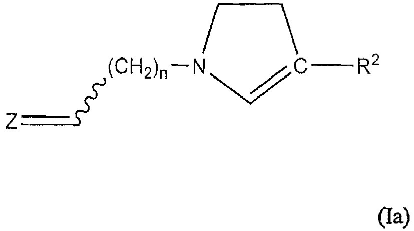
Z, n and R² are as described in Structural Formula (I).
R⁴⁰ is selected from the group consisting of:

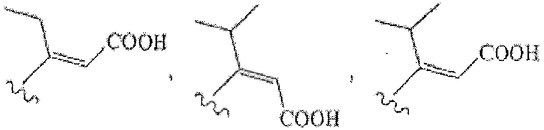

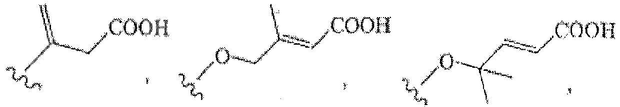

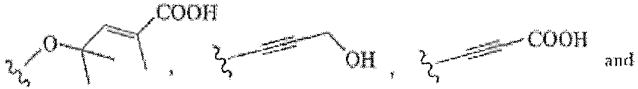

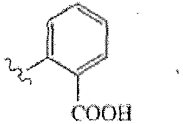

In a preferred embodiment, the chemokine receptor antagonist can be represented by Structural Formula I wherein n is three, M is C(OH)R², R² is a phenyl group or a halophenyl group (e.g., 4-chlorophenyl) and Z is represented by Structural Formula (VI) wherein X₁ is -CH₂-O-.

Thus, the antagonist of chemokine function can be represented by, Structural Formula (VIIa): 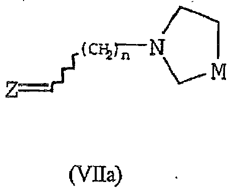 and physiologically acceptable salts thereof, wherein Z, n and M are as described in Structural Formula (VII), and the ring which contains M is substituted or unsubstituted. The ring containing M can have one or more suitable substituents which are the same or different. Suitable substituents for the ring which contains M and other nonaromatic heterocyclic rings are as described herein. For example, the ring containing M can be substituted with a methyl, ethyl, propyl, butyl or oxo group.

The compound is further represented by Structural Formula VIIi: 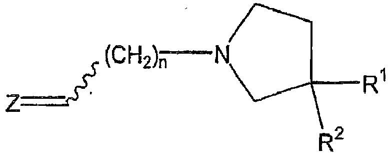 or a physiologically acceptable salt thereof, wherein n, R¹ and R² are as described in Structural Formula (I), and Z is as described in Structural Formula (VI).

In a certain embodiments, Z is represented by Structural Formula (VI) wherein X₁ is -CH₂-O-; n is two, R¹ is -H and R² is -NR⁵R⁶. Preferably, compounds of these embodiments have the structure: 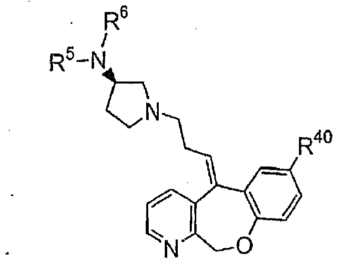 or a physiologically acceptable salt thereof, wherein R⁵ and R⁶ are as described in Structural Formula I, and preferred groups at R⁴⁰ are as described herein.

In particular embodiments, R⁵ is aliphatic group (e.g., C₁-C₆ alkyl) or substituted aliphatic group, and R⁶ is benzyl or substituted benzyl; or R⁵ and R⁶ taken together with the atom to which they are bonded, form a substituted or unsubstituted non-aromatic carbocyclic or heterocyclic ring. In more particular embodiments, R⁵ is C₁-C₆ alkyl and R⁶ is halo-substituted benzyl. In a preferred embodiment, R⁵ is ethyl and R⁶ is chloro-substituted benzyl (e.g., 4-chlorobenzyl).

The nitrogen atom in the ring containing M can be a tertiary nitrogen as depicted in Structural Formula (IV), or the nitrogen atom can be quaternized with a suitable substituent, such as a C₁ to about C₆ or a C₁ to about C₃ substituted or unsubstituted aliphatic group. Compounds which comprise a quaternary nitrogen atom can also contain a counteranion such as chloride, bromide, iodide, acetate, perchlorate and the like.

The antagonist of chemokine function can be represented by Structural Formula (VII) wherein the heterocyclic ring containing M is substituted with a suitable bivalent group which is bonded to two atoms that are in the ring, thereby forming a bicyclic moiety. Suitable bivalent groups include, for example, substituted or unsubstituted bivalent aliphatic groups, such as a C₁-C₆ alkylene group.

The chemokine receptor antagonist described herein can be prepared and administered as active compounds or as prodrugs. Generally, prodrugs are analogues of pharmaceutical agents which can undergo chemical conversion by metabolic processes to become fully active. For example, A prodrug of the invention can be prepared by selecting appropriate groups for R⁴⁰. In one embodiment, a prodrug can be represented by Structural Formula (XI): 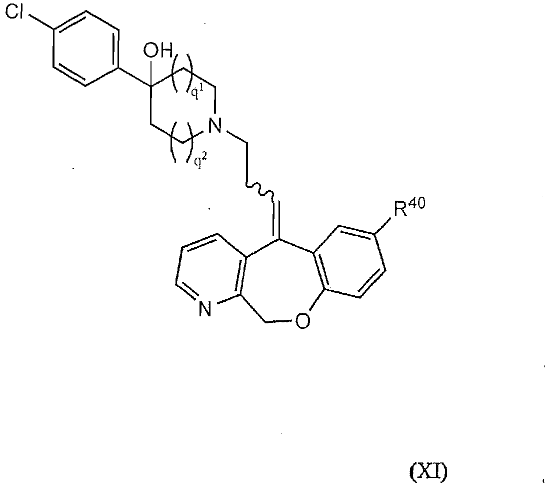 wherein, R⁴⁰ is Q-substituted aliphatic group, and the aliphatic group is substituted with -(O)ᵤ-(CH₂)ₜ-C(O)OR²⁰, wherein Q is -C(O)O-, u is one, t is zero and R²⁰ is a cyclic aliphatic group. For example, when the substituted aliphatic group is a substituted ethyl group, R⁴⁰ can be represented by: 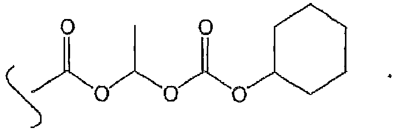 Such a prodrug can be converted to an active chemokine receptor antagonist represented by Structural Formula XI, wherein R⁴⁰ is -COOH.

Another embodiment of the present invention includes novel compounds employed in these methods.

The compounds disclosed herein can be obtained as E- and Z-configurational isomers. It is expressly pointed out that the invention includes compounds of the E-configuration and the Z-configuration around the double bond connecting Ring C of Z to the remainder of the molecule, and a method of treating a subject with compounds of the E-configuration, the Z-configuration, and mixtures thereof. Accordingly, in the structural formulas presented herein, the symbol: 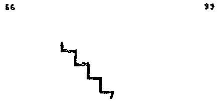 is used to represent both the E-configuration and the Z-configuration. Preferably Ring A and the alkylene chain bonded to Ring C are in the cis configuration. For example, the compounds can have the configuration of:

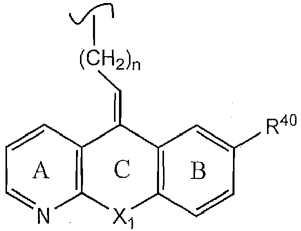

It is understood that one configuration can have greater activity than another. The desired configuration can be determined by screening for activity, employing the methods described herein.

Additionally, certain compounds of the invention may be obtained as different stereoisomers (e.g., diastereomers and enantiomers). The compounds of the invention can be prepared as racemates or as substantially pure stereoisomers. The stereoisomers of the invention (e.g., (*S*)*-* and (*R*)-enantiomers) can be prepared using any suitable method. For example, the enantiomers can be resolved from the racemate using chiral chromatography or recrystallization. Preferably, the stereoisomers (e.g., (*S*)- and/or (R)-enantiomers) are prepared by stereospecific synthesis as described herein.

The optical configuration of the stereoisomers of the invention are assigned using the (*R*),(*S*) method of Cahn-Ingold-Prelog. (See, J. March, "Advanced Organic Chemistry," 4th Edition, Wiley Interscience, New York, pp.109-111 (1992).)

The invention includes all isomeric forms and racemic mixtures of the disclosed compounds and a method of treating a subject with both pure isomers and mixtures thereof, including racemic mixtures. Sterioisomers can be separated and isolated using any suitable method, such as chromatography. Again, it is understood that one sterioisomer may be more active than another. The desired isomer determined by screening.

Also included in the present invention are physiologically acceptable salts of the compounds of the invention. Salts of compounds containing an amine or other basic group can be obtained, for example, by reacting with a suitable organic or inorganic acid, such as hydrogen chloride, hydrogen bromide, acetic acid, citric acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium, ammonium, calcium and the like. (See, for example, Berge S.M. et al., "Pharmaceutical Salts," J. Pharma. Sci., 66:1 (1977).)

As used herein, aliphatic groups include straight chained, branched or cyclic C₁-C₂₀ hydrocarbons which are completely saturated or which contain one or more units ofunsaturation. Preferred aliphatic groups are C₁ to about C₁₀ hydrocarbons. More preferred are C₁ to about C₆ or C₁ to about C₃ hydrocarbons. One or more carbon atoms in an aliphatic group can be replaced with a heteroatom, such as nitrogen, oxygen or sulfur. For example, suitable aliphatic groups include substituted or unsubstituted linear, branched or cyclic C₁-C₂₀ alkyl, alkenyl or alkynyl groups.

An aminoalkyl group is an alkyl group substituted with -NR²⁴R²⁵, R²⁴ and R²⁵ are as described herein. Preferably the alkyl moiety comprises one to about twelve, more preferably one to about six carbon atoms. The alkyl moiety of an aminoalkyl group can be unsubstituted or substituted as described herein for aliphatic groups. Examples of suitable aminoalkyl groups include aminomethyl, 2-aminoethyl, 3-aminopropyl, 4-aminobutyl, dimethylaminoethyl, diethylaminomethyl, methylaminohexyl, aminoethylenyl and the like.

Aromatic groups include carbocyclic aromatic groups such as phenyl, 1-naphthyl, 2-naphthyl, 1-anthracyl and 2-anthracyl, and heterocyclic aromatic or heteroaryl groups such as *N*-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl and 5-oxazolyl. Where these rings are fused, for example, to Ring C, the stated point of attachment can be either of the two fused bonds.

Aromatic groups also include fused polycyclic aromatic ring systems in which a carbocyclic aromatic ring or heteroaryl ring is fused to one or more other rings. Examples include tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 1-isoquinolinyl, 3-quinolinyl, 1-isoindolyl, 3-isoindolyl, acridinyl, 3-benzisoxazolyl, and the like. Also included within the scope of the term "aromatic group", as it is used herein, is a group in which one or more carbocyclic aromatic rings and/or heteroaryl rings are fused to a cycloalkyl or non-aromatic heterocyclic ring, for example, benzocyclopentane, benzocyclohexane,

Non-aromatic heterocyclic rings are non-aromatic carbocyclic rings which include one or more heteroatoms such as nitrogen, oxygen or sulfur in the ring. The ring can be five, six, seven or eight-membered and/or fused to another ring, such as a cycloalkyl on aromatic ring. Examples include 1,3-dioxolan-2-yl, 3-1H-benzimidazol-2-one, 3-1-alkyl-benzimidazol-2-one, 3-1-methyl-benzimidazol-2-one, 2-tetrahydrofuranyl, 3-tetrahydrofuranyl, 2-tetrahyrothiophenyl, 3-tetrahyrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidyl, 1-3-alkyl-phthalimidyl, benzoxane, benzopyrolidine, benzopiperidine, benzoxolane, benzothiolane, benzothiane, tetrahydrofuran-2-one-3-yl, 2,5-dihydro-5-oxo-4H-1,2,4-thiadiazol-3-yl, 2-oxo-3H-1,2,3,5-oxathiadiazol-4-yl,

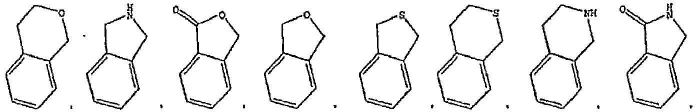

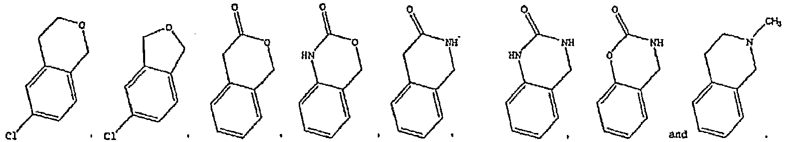

Suitable substituents on an aliphatic group, aromatic group (carbocyclic and heteroaryl), non-aromatic heterocyclic ring or benzyl group include, for example, an electron withdrawing group, a halogen (chloride, bromide, fluoride, iodide), azido, -CN, -COOH, -OH, -CONR²⁴R²⁵, -NR²⁴R²³, -OS(O)₂NR²⁴R²⁵, -S(O)₂NR²⁴R²⁵, -SO₃H, -S(O)₂NH₂, guanidino, ureido, oxalo, amidino, -C(=NR⁶⁰)NR²¹R²²,=NR⁶⁰. -(O)ᵤ-(CH₂)ₜ-C(O)OR²⁰, -(O)ᵤ-(CH₂)ₜ-OC(O)R²⁰, -(O)ᵤ-(CH₂)ₜ-(C(O)-NR²¹R²², -(O)ᵤ(CH₂)ₜ-NHC(O)O-R²⁰, -Q-H, -Q-(aliphatic group),-Q-(substituted aliphatic group), -Q-(aryl), -Q-(aromatic group), -Q-(substituted aromatic group), -Q-(CH₂)ₚ-(substituted or unsubstituted aromatic group) p is an integer from 1-5), -Q-(non-aromatic heterocyclic group) or -Q-(CH₂)ₚ-(non-aromatic heterocyclic group).

R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group) and wherein R²¹ and R²², taken together with the nitrogen atom to which they are bonded, can form a substituted or unsubstituted non-aromatic heterocyclic ring.

R⁶⁰ is a -H, -OH, -NH₂, an aromatic group or a substituted aromatic groupe

t is an integer from zero to about three, and the methylene group, -(CH₂)ₜ-, can be substituted, as described herein for aliphatic groups, or unsubstituted.

u is zero or one.

Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)N-H-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-_{.}

R²³ is -H, an aliphatic group, a benzyl group, an aryl group or non-aromatic heterocyclic group.

R²⁴ and R²⁵ are independently -H, -OH, an aliphatic group, a substituted aliphatic group, a benzyl group, an aryl group, non-aromatic heterocyclic group or R²⁴ and R²³ taken together with the nitrogen atom to which they are bonded can form a substituted or unsubstituted non-aromatic heterocyclic ring.

A substituted non-aromatic heterocyclic ring, benzyl group or aromatic group can also have an aromatic group, an aliphatic or substituted aliphatic group, as a substituent. When a non-aromatic ring (carbocyclic or heterocyclic) or an aromatic ring (carbocyclic aromatic or heteroaryl) is substituted with another ring, the two rings can be fused. A substituted aliphatic group can also have an oxo group, epoxy group, non-aromatic heterocyclic ring, benzyl group, substituted benzyl group, aromatic group or substituted aromatic group as a substituent. A substituted non-aromatic heterocyclic ring can also have =O, =S, =NH or =N(aliphatic, aromatic or substituted aromatic group) as a substituent. A substituted aliphatic, substituted aromatic, substituted non-aromatic heterocyclic ring or substituted benzyl group can have more than one substituent, which can be the same or different.

Acyl groups include substituted and unsubstituted aliphatic carbonyl, aromatic carbonyl, aliphatic sulfonyl and aromatic sulfonyl.

Suitable electron withdrawing groups include, for example, alkylimines, alkylsulfonyl, carboxamido, carboxylic alkyl esters, -CH=NH, -CN, -NO₂ and halogens. In the structural formulas depicted herein, the single or double bond by which a chemical group or moiety is connected to the remainder of the molecule or compound is indicated by the following symbol: 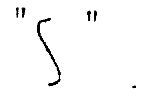 For example, the corresponding symbol in Structural Formulas (II), (III) and (IV) indicates the double bond by which the central ring of the tricyclic ring system is connected to the remainder of the molecule represented by Structural Formula (I).

A "subject" is preferably a bird or mammal, such as a human, but can also be an animal in need of veterinary treatment, e.g., domestic animals (e.g., dogs, cats, and the like), farm animals (e.g., cows, sheep, fowl, pigs, horses, and the like) and laboratory animals (e.g., rats, mice, guinea pigs, and the like).

An "effective amount" of a compound is an amount which results in the inhibition of one or more processes mediated by the binding of a chemokine to a receptor in a subject with a disease associated with aberrant leukocyte recruitment and/or activation. Examples of such processes include leukocyte migration, integrin activation, transient increases in the concentration of intracellular free calcium [Ca²⁺]ᵢ and granule release of proinflammatory mediators. Alternatively, an "effective amount" of a compound is a quantity sufficient to achieve a desired therapeutic and/or prophylactic effect, such as an amount which results in the prevention of or a decrease in the symptoms associated with a disease associated with aberrant leukocyte recruitment and/or activation.

The amount of compound administered to the individual will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compound can range from about 0.1 mg per day to about 100 mg per day for an adult. Preferably, the dosage ranges from about 1 mg per day to about 100 mg per day. An antagonist of chemokine receptor function can also be administered in combination with one or more additional therapeutic agents, e.g. theophylline, β-adrenergic bronchodilators, corticosteroids, antihistamines, antiallergic agents, immunosuppressive agents (e.g., cyclosporin A, FK-506, prednisone, methylprednisolone), hormones (e.g., adrenocorticotropic hormone (ACTH)), cytokines *(e.g.,* interferons (*e.g.,* IFNβ-1a, IFNβ-1b)) and the like.

The compound can be administered by any suitable route, including, for example, orally in capsules, suspensions or tablets or by parenteral administration. Parenteral administration can include, for example, systemic administration, such as by intramuscular, intravenous, subcutaneous, or intraperitoneal injection. The compound can also be administered orally (e.g., dietary), transdermally, topically, by inhalation (e.g., intrabronchial, intranasal, oral inhalation or intranasal drops), or rectally, depending on the disease or condition to be treated. Oral or parenteral administration are preferred modes of administration.

The compound can be administered to the individual in conjunction with an acceptable pharmaceutical or physiological carrier as part of a pharmaceutical composition for treatment of HIV infection, inflammatory disease, or the other diseases discussed above. Formulation of a compound to be administered will vary according to the route of administration selected (e.g., solution, emulsion, capsule). Suitable carriers may contain inert ingredients which do not interact with the compound. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% benzyl alcohol), phosphate-buffered saline, Hank's solution, Ringer's-lactate and the like. Methods for encapsulating compositions (such as in a coating of hard gelatin or cyclodextran) are known in the art (Baker, et al., "Controlled Release of Biological Active Agents", John Wiley and Sons, 1986).

The quantity of active ingredient (one or more compounds of the invention) in the composition can range from about 0.1 % to about 99.9% by weight. Preferably the quantity of active ingredient is about 10% to about 90%, or about 20% to about 80% by weight. A unit dose preparation can contain from 1 mg to about 1000 mg active ingredient, preferably about 10 mg to about 100 mg active ingredient. The composition can, if desired, also contain other compatible therapeutic agents, such as theophylline, β-adrenergic bronchodilators, corticosteroids, antihistamines, antiallergic agents, immunosuppressive agents (e.g., cyclosporin A, FK-506, prednisone, methylprednisolone), hormones (*e.g*., adrenocorticotropic hormone (ACTH)), cytokines (*e.g.,* interferons *(e.g.,* IFNβ-1a, IFNβ-1b) and the like.

In one embodiment, the pharmaceutical composition comprises the (*S*)-enantiomer of a compound of the invention (e.g., a compound of Structural Formula (XIII)) and a physiologically acceptable carrier or excipient. For example, in one embodiment, the composition comprises *(S)*-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol and a physiologically acceptable carrier or excipient. In certain embodiments, the pharmaceutical composition comprises the (*S*)-enantiomer of a compound of the invention (e.g., a compound of Structural Formula (XIII)) and is substantially free of the corresponding (*R*)-enantiomer (contains at least about 98% or at least about 99% enantiomeric excess of (*S*)-enantiomer). In another embodiments, the composition comprises the (*S*)-enantiomer of a compound of the invention (e.g., a compound of Structural Formula (XII)), the corresponding (R)-enantiomer and a physiologically acceptable carrier or excipient. In a more particular embodiment, the composition comprises a racemic compound of Structural Formula (XII), for example, racemic-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol In other embodiments, the ratio (*S*)-enantiomer:(*R*)-enantiomer (w/w) in the compositions is at least about 2:1 or about 5:1 or about 10:1 or about 20:1 or about 50:1.

The activity of compounds of the present invention can be assessed using suitable assays, such as receptor binding assays and chemotaxis assays. For example, as described in the Exemplification Section, small molecule antagonists of RANTES and MIP-1α binding have been identified utilizing THP-1 cells which bind RANTES and chemotax in response to RANTES and MIP-1α as a model for leukocyte chemotaxis. Specifically, a high through-put receptor binding assay, which monitors ¹²⁵I-RANTES and ¹²⁵-MIP-1α binding to THP-1 cell membranes, was used to identify small molecule antagonists which block binding of RANTES and MIP-1α Compounds of the present invention can also be identified by'virtue of their ability to inhibit the activation steps triggered by binding of a chemokine to its receptor, such as chemotaxis, integrin activation and granule mediator release. They can also be identified by virtue of their ability to block RANTES and MIP-1α mediated HL-60, T-cell, peripheral blood mononuclear cell, and eosinophil chemotactic response.

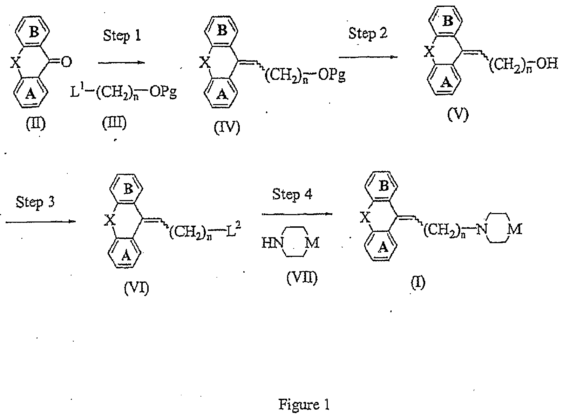

The compounds disclosed herein can be prepared accordingly to the schemes shown in Figures 1- 5 and 7. The schemes are described in greater detail below.

Figure 1 shows the preparation of compounds represented by Structural Formula (I). L¹ is PPh₃Cl, PPh₃Br, PPh₃I or (EtO)₂P(O), L² is a suitable leaving group such as halogen, p-toluene sulfonate, mesylate, alkoxy, and phenoxy; Pg is a suitable protecting group such as tetrahydropyranyl; and the other symbols are as defined above.

In Step 1 of Figure 1, a Wittig reaction is carried out in a solvent such as ether, or tetrahydrofuran (THF) in the presence of a base such as sodium hydride, n-butyl lithium or lithium diisopropylamide (LDA) at 0°C up to the reflux temperature for the solvent used for 5 minutes to 72 h. Compounds represented by Formula II in Figure 1 can be prepared by methods disclosed in JP 61/152673, U.S. Patent 5089496, WO 89/10369, WO 92/20681 and WO 93/02081.

In Step 2 of Figure 1, deprotection is carried out with an acid in a solvent such as methanol at room temperature up to the reflux temperature for the solvent used for 5 minutes to 72 h. Alternatively, a compound of represented by Formula V in Figure 1 can be prepared directly from step 1 without isolating an intermediate. The reaction mixture obtained after the work up of the reaction described in step 1 can be dissolved in the solvent and reacted with the acid.

In Step 3 of Figure 1, the hydroxy group can be converted to a leaving group by known methods. Compounds represented by Formula VI in Figure 1 can be prepared by methods disclosed in J. Med. Chem., 1992 (35) 2074-2084 and JP 61/152673.

In Step 4 of Figure 1, an alkylation reaction is carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

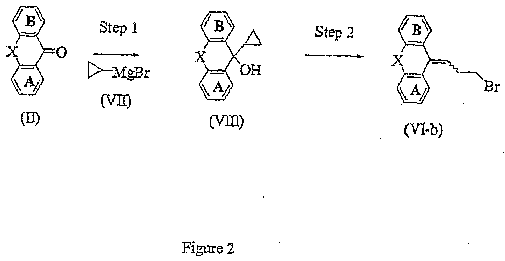

Figure 2 shows the preparation of compounds represented by Compound (VI-b). In Step 1 of Figure 2, a Grignard reaction may be carried out in a solvent such as ether, or tetrahydrofuran (THF) at 0°C up to the reflux temperature for the solvent used for 5 minuets to 72 h. Compound VII is available commercially.

In Step 2 of Figure 2, bromination may be carried out with brominate agents such as hydrobromic acid, bromotrimethylsilane or boron tribromide-methyl sulfide complex in a solvent such as acetic acid, dichloromethane or dichloroethane at room temperature up to the reflux temperature for the solvent used for 5 minutes to 72 h.

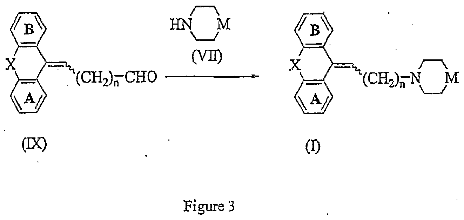

Figure 3 shows the preparation of compounds represented by Structural Formula (I). In Figure 3, a reductive amination may be carried out with reducing regents such as sodium cyanoborohydride, sodium acetoxyborohydride or sodium borohydride in a solvent such as methanol, ethanol, tetrahydrofuran (THF), dichloromethane or dichloroethane at room temperature up to the reflux temperature for the solvent used for 5 minutes to 72 h.

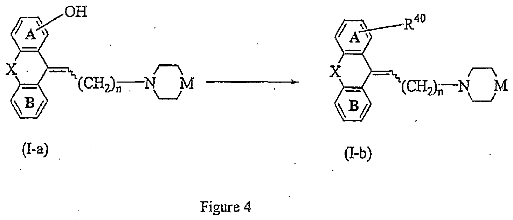

Figure 4 shows the preparation of compounds represented by Structural Formula (I), wherein Ring
A and/or Ring B in Z is substituted with R⁴⁰. In Figure 4, the alkylation reaction can be carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

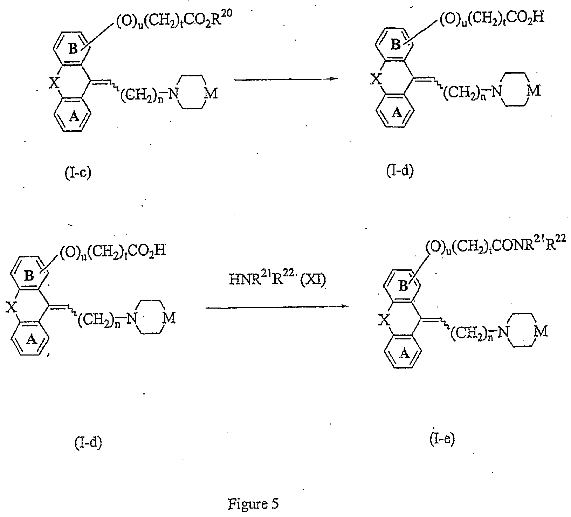

Figure 5 is a schematic showing the preparation of the compounds represented by Structural Formula (I), wherein
Ring A and/or Ring B in Z is substituted with -(O)ᵤ-(CH₂)ₜ-COOR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰. In Figure 5, the hydrolysis reaction may be carried out in a mixture of aqueous alkali metal hydroxide solution and a solvent such as methanol, ethanol, tetrahydrofuran (THF) or dioxane at room temperature up to the reflux temperature for the solvent used for 5 minutes to 72 h. The acylation reaction can be carried out using dicyclohexylcarbodiimide (DCC) or (1-ethyl-3-(3-dimethylaminopropyl)carbodiimide (DEC) in a solvent such as tetrahydrofuran (THF), dimethylformamide (DMF) or methylene chloride in the presence of a base such as pyridine or triethylamine (when necessary) at temperatures of 0 to 100°C for 5 minutes to 72 h.

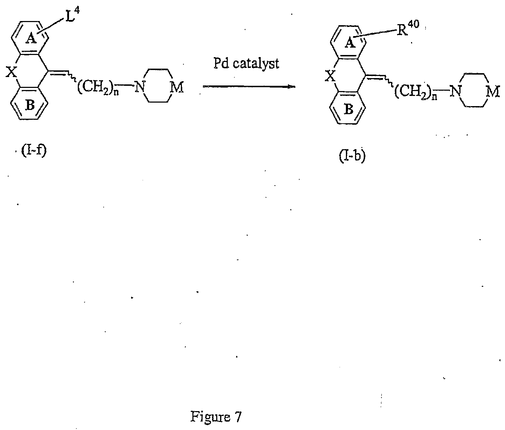

Figure 7 shows the preparation of compounds represented by Structural Formula (I), wherein Ring
A or Ring B in Z is substituted with **R⁴⁰**. L⁴ is a suitable leaving group such as halogen or trifluoromethylsulfonate. In Figure 7, a palladium coupling reaction such as Stille coupling, Suzuki coupling, Heck reaction, or carboxylation using carbon monoxide may be carried out using a palladium catalyst such as tetrakis(triphenylphosphine)palladium, bis(triphenylphosphine)palladium chloride, and palladium acetate in a solvent such as tetrahydrofuran (THF), 1,4-dioxane, toluene, dimethylformamide (DMF), or dimethylsufoxide (DMSO) in the presence of additive (when necessary) such as triphenylphosphine, 1,1'-bis(diphenylphosphino)ferrocene, triethylamine, sodium bicarbonate, tetraethylammonium chloride, or lithium chloride at room temperature up to the reflux temperature for the solvent used for 5 minutes to 72 h.

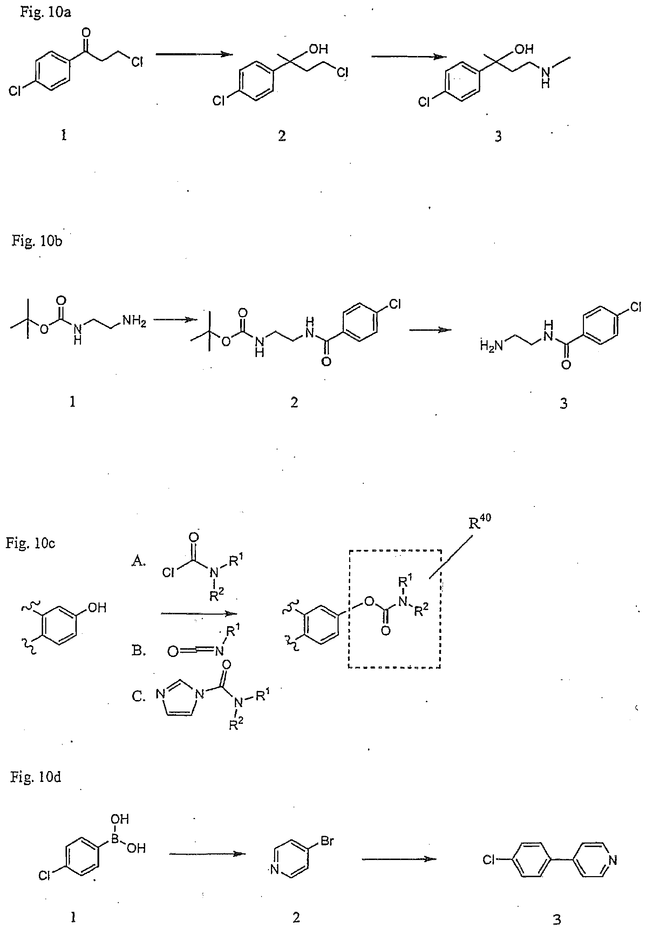

Figure 10C shows three procedures for the preparation of compounds wherein ring A or Ring B in Z is
substituted with R⁴⁰, In Figure 10C, R⁴⁰ is represented by -(O)ᵤ-(CH₂)-C(O)-NR²¹R²², u is one, t is zero. In Figure 10C a compound containing a phenol can be reacted with a carbonate equivalent, such as a carbamoyl chloride (method A), an isocyanate (method B) or an acylimidazole (method C), in the presence of a base such as sodium hydroxide, potassium carbonate or sodium carbonate in a solvent such as dimethylformamide or tetrahydrofuran, at a temperature from 0°C to reflux temperature for a period of about 5 minutes to about 72 hours.

Compounds represented by Structural Formula (I), wherein X is -CO-NR_{c}- and R_{c} is -(CH₂)ₛ-COOR³⁰,
-(CH₂)ₛ-C(O)-NR³¹R³² or -(CH₂)₅NHC(O)-O-R³⁰, can be prepared by suitable modification of the scheme shown in Figure 1-5 and 7. One modification utilizes the starting material shown in Figure 1, wherein X is -CO-NH-. The amide is then alkylated with L³-(CH₂)ₛ-COOR³⁰, wherein L³ is a suitable leaving group, using the alkylation procedures described above. The remainder of the synthesis is as described in Figures 1 - 5 and 7.

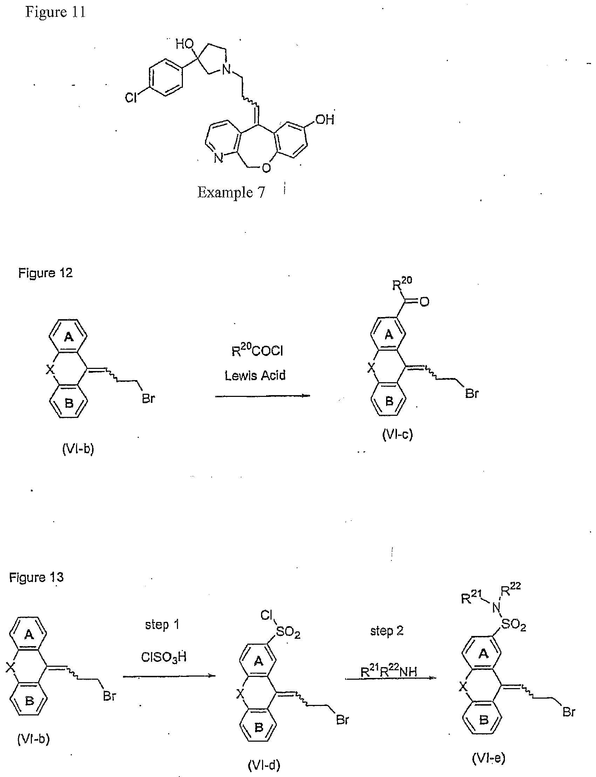

Figure 12 shows the preparation of compounds of formula (VI-c). The Friedel-Crafts acylation can be carried out using an acid chloride in the presence of a Lewis acid, such as aluminum trichloride or titanium tetrachloride, in a solvent such as dichloromethane, dichloroethane, nitrobenzene or carbon disulfide. The acylation reaction can be run at a temperature of about room temperature up to the reflux temperature of the chosen solvent, and for a period of about 5 minutes to about 72 hours.

Figure 13 shows the preparation of compounds of formula (VI-e). In Step 1 of Figure 13, a chlorosulfonylation can be carried out using chlorosulfonic acid in a solvent, such as dichloromethane, or in the absence of a solvent at a temperature of about 0°C to about 60°C for a period of about 5 minutes to about 72 hours. In Step 2 of Figure 12, a coupling reaction can be carried out using an amine in the presence of a base, such as triethylamine, in a solvent such as dichloromethane, acetone, ethanol, THF or DMF. The reaction can be carried out at a temperature of about room temperature up to the reflux temperature of the selected solvent, and for a period of about 5 minutes to about 72 hours.

Although Figures 1 - 5, 7, 12 and 13 show the preparation of compounds in which Rings A and B are phenyl rings, analogous compounds with heteroaryl groups for Rings A and B can be prepared by using starting materials with heteroaryl groups in the corresponding positions. These starting materials can be prepared according to methods disclosed in JP 61/152673, U.S. Patent 5089496, WO 89/10369, WO 92/20681 and WO 93/02081.

The invention is illustrated by the following examples which are not intended to be limiting in any way.

### REFERENCE EXAMPLES

Membrane Preparations for Chemokine Binding and Binding Assays Membranes were prepared from THP-1 cells (ATCC #TIB202). Cells were harvested by centrifugation, washed twice with PBS (phosphate-buffered saline), and the cell pellets were frozen at -70 to -85°C. The frozen pellet was thawed in ice-cold lysis buffer consisting of 5 mM HEPES (N-2-hydroxyethylpiperazine-N'-2-ethane-sulfonic acid) pH 7.5, 2 mM EDTA. (ethylenediaminetetraacetic acid), 5 µg/ml each aprotinin, leupeptin, and chymostatin (protease inhibitors), and 100 µg/ml PMSF (phenyl methane sulfonyl fluoride - also a protease inhibitor), at a concentration of 1 to 5 x 10⁷ cells/ml. This procedure results in cell lysis. The suspension was mixed well to resuspend all of the frozen cell pellet. Nuclei and cell debris were removed by centrifugation of 400 x g for 10 minutes at 4°C. The supernatant was transferred to a fresh tube and the membrane fragments were collected by centrifugation at 25,000 x g for 30 minutes at 4°C. The supernatant was aspirated and the pellet was resuspended in freezing buffer consisting of 10 mM HEPES pH 7.5, 300 mM sucrose, 1µg/ml each aprotinin, leupeptin, and chymostatin, and 10 µg/ml PMSF (approximately 0.1 ml per each 10⁸ cells). All clumps were resolved using a minihomogenizer, and the total protein concentration was determined using a protein assay kit (Bio-Rad, Hercules, CA, cat #500-0002). The membrane solution was then aliquoted and frozen at -70 to -85°C until needed.

Binding Assays utilized the membranes described above. Membrane protein (2 to 20 µg total membrane protein) was incubated with 0.1 to 0.2 nM ¹²⁵I-labeled RANTES or MIP-1α with or without unlabeled competitor (RANTES or MIP-1α) or various concentrations of compounds. The binding reactions were performed in 60 to 100 µl of a binding buffer consisting of 10 mM HEPES pH 7.2, 1 mM CaCl₂, 5 mM MgCl₂, and 0.5% BSA (bovine serum albumin), for 60 min at room temperature. The binding reactions were terminated by harvesting the membranes by rapid filtration through glass fiber filters (GF/B or GF/C, Packard) which were presoaked in 0.3% polyethyleneimine. The filters were rinsed with approximately 600 µl of binding buffer containing 0.5 M NaCl, dried, and the amount of bound radioactivity was determined by scintillation counting in a Topcount beta-plate counter.

The activities of test compounds are reported in the Table below as IC₅₀ values or the inhibitor concentration required for 50% inhibition of specific binding in receptor binding assays using ¹²⁵I-RANTES or ¹²⁵I-MIP-1α as ligand and THP-1 cell membranes. Specific binding is defined as the total binding minus the non-specific binding; non-specific binding is the amount of cpm still detected in the presence of excess unlabeled Rantes or MIP-1α.

**Table BIOLOGICAL DATA**

| Example | IC50 (µM) | Example | IC50 (µM) | Example | IC50 (µm) | Example | IC50 (µM) |
|---|---|---|---|---|---|---|---|
| 1 | <1 | 24 | <20 | 33 | <1 | 44 | <10 |
| 2 | <1 | 25 | <1 | 34 | <1 | 45 | <10 |
| 3 | <1 | 26 | <2 | 35 | <1 | 46 | <20 |
| 18 | <1 | 27 | <10 | 36 | <1 | 47 | <1 |
| 19 | <20 | 28 | <1 | 37 | <1 | 48 | <1 |
| 20 | <1 | 29 | <10 | 40 | <10 | 49 | <1 |
| 21 | <1 | 30 | <1 | 41 | <10 | 50 | <10 |
| 22 | <10 | 31 | <1 | 42 | <10 | 51 | <10 |
| 23 | <20 | 32 | <10 | 43 | <10 | | |

### Example 1: 4-(4-Chlorophenyl)-1-[3-(5,11-dihydro-7-hydroxy[1]benzoxepino[2,3-b]pyridine-5-ylidene)propyl]piperidin-4-ol

### Step 1

To a solution of the product of example 2, step 1 (4.3g) in dichloroethane (100ml) was added boron tribromide-methyl sulfide complex (19.3g) and the mixture was heated to reflux for 3 hour. Water and ethyl acetate were added to the reaction mixture and neutralized with dilute NaOH solution. The organic layer was separated and washed with saturated aqueous sodium chloride, and dried over magnesium sulfate. The solvent was distilled off under reduced pressure. The residue was purified by silica gel chromatography eluting with ethyl acetate-hexane (1:2) to give 5-(3-bromopropylidene)-5,11-dihydro-7-hydroxy [1]benzoxepino[2,3-b]pyridine (3.2g).
¹H-NMR (CDCl₃) δ: 2.72(2H,q), 3.45(2H,t), 5.28(2H,brs), 6.03(1H,t), 6.66-6.80(3H,m), 7.26(1H,dd), 7.58(1H,dd), 8.51(1H,dd).

### Step 2

The titled compound was prepared by following the procedure of example 2, step 3, but replacing 5-(3-bromopropylidene)-5,11-dihydro-7-methoxy [1]benzoxepino[2,3-b]pyridine with the product of step 1. ¹H-NMR (DMSO-d₆) δ:1.46-1.51(2Hm), 1.74-1-85(2H,m), 2.29-2.51(8H,m), 5.15(2H,brs), 6.07(1H,t), 6.61-6.70(3H,m), 7.33-7.48(5H,m), 7.73(1H,dd), 8.47(1H,dd), 9.06(1H,s).
MS m/z: 463(M+1)

### Example 2: 4-(4-Chlorophenyl)-1-[3-(5,11-dihydro-7-methoxy[1]benzoxepino[2,3-b]pyridin-5-ylidene)propyl]piperidin-4-ol

### Step 1

To a solution of 5,11-dihydro-7-methoxy [1]benzoxepino[2,3-b]pyridin-5-one (5.0g) in THF (50ml) was added 1.1M cyclopropylmagnesium bromide THF solution (25ml) at 0°C. The reaction mixture was warmed to room temperature, and stirred for 30 minutes. Aqueous ammonium chloride and ethyl acetate were added to the reaction mixture, the organic layer was separated and washed with saturated aqueous sodium chloride, and dried with magnesium sulfate. The solvent was distilled off under reduced pressure. The residue was filtered and washed with ethyl acetate-hexane (1:2) to give 5-cyclopropyl-5,11-dihydro-7-methoxy[1]benzoxepino[2,3-b]pyridin-5-ol (5.0g).

### Step 2

To a solution of the product of step 1 (4.3g) in acetic acid (30ml) was added 48% aqueous HBr (25ml) at 10°C. The reaction mixture was warmed to room temperature, and stirred for 12 hours. Water and ethyl acetate were added to the reaction mixture and neutralized with dilute NaOH solution. The organic layer was separated and washed with saturated aqueous sodium chloride, and dried over magnesium sulfate. The solvent was distilled off under reduced pressure. The residue was purified by silica gel chromatography eluting with ethyl acetate-hexane (1:4) to give 5-(3-bromopropylidene)-5,11-dihydro-7-methoxy [1]benzoxepino[2,3-b]pyridine (5.6g).
¹H-NMR (CDCl₃) δ: 2.74(2H,q), 3.46(2H,t), 3.78(3H,s), 5.25(2H,brs), 6.07(1H,t), 6.72-6.82(3H,m), 7.21-7.42(5H,m), 7.56(1H,dd), 8.45(1H,dd).

### Step 3

To a solution the product of step 2 (1.1g) in DMF (15ml) were added 4-(4-chlorophenyl)-4-hydroxypiperidine (0.81g) and potassium carbonate (0.53g) and the mixture was stirred at room temperature for 3 hours. Water and ethyl acetate were added to the reaction mixture, the organic layer was separated and washed with saturated aqueous sodium chloride, and dried with magnesium sulfate. The solvent was distilled off under reduced pressure. The residue was purified by silica gel chromatography eluting with methylene chloride-methanol (10:1) to give the titled compound as major regioisomer (0.86g) and minor one (0.05g).
Major isomer
¹H-NMR (CDCl₃) δ: 1.64-1.69(2H,m), 1.91-2.08(3H,m), 2.34-2.69(BH,m), 3.77(3H,s), 5.25(2H,brs), 6.07(1H,t), 6.72-6.82(3H,m), 7.21-7.42(5H,m),
7.56(1H,dd), 8.45(1H,dd).
MS m/z: 477(M+1)
Minor isomer
1H-NMR (CDCl₃) d: 1.65-1.79(3H,m), 2.01-2.13(2H,m), 2.35-2.76(8H,m), 3.76(3H,s), 5.22(2H,brs), 5.95(1H,t), 6.72-6.80(2H,m), 7.06(1H,d), 7.16(1H,dd), 7.28(2H,d), 7.42(2H,d), 7.66(1H,dd), 8.39(1H,dd).
MS m/z: 477(M+1)

### Example 3: 4-(4-Chlorophenyl)-1-[3-(5,11-dihydro[1]benzoxepino[2,3-b]pyridin-5-ylidene)propyl]piperidin-4-ol

### Step 1

5-(3-Bromopropylidene)-5,11-dihydro[1]benzoxepino[2,3-b]pyridine was prepared by following the procedure of example 2, step 1 and 2, but replacing 5,11-dihydro-7-methoxy[1]benzoxepino[2,3-b]pyridin-5-one with 5,11-dihydro[1]benzoxepino[2,3-b]pyridin-5-one.
¹H-NMR (CDCl₃) δ: 2.71(2H,q), 3.46(2H,t), 5.33(2H,brs), 6.04(1H,t), 7.01-7.17(3H,m), 7.29(1H,dd), 7.56(1H,dd), 8.53(1H,dd).

### Step 2

The titled compound was prepared by following the procedure of example 2, step 3, but replacing 5-(3-bromopropylidene)-5,11-dihydro-7-methoxy [1]benzoxepino[2,3-b]pyridine with the product of step 1.
¹H-NMR (CDCl₃) δ: 1.66-1.71(2H,m), 2.00-2.20(3H,m), 2.36-2.69(8H,m); 5.34(2H,brs), 6.10(1H,t), 6.83-6.96(3H,m),
7.17-7.44(6H,m), 7.60(1H,dd), 8.46(1H,dd).
MS m/z: 447(M+1)

### Example 5:

### 1-[3-(7-Acetyl-5,11-dihydro[1]benzoxepino[2,3-b]pyridin-5-ylidene)propyl]-4-(4-chlorophenyl)piperidin-4-ol

### Step 1

To a solution of example 3, step 1 (7.2g) in dichloromethane (70 ml) was added aluminum chloride (9.1 g) and acetyl chloride (3.2 ml), and the mixture stirred at 0°C for 10 minutes. The reaction mixture was poured into ice. The aqueous layer was extracted with ethyl acetate, and the organic layer was washed with saturated aqueous sodium chloride, and dried with magnesium sulfate. The solvent was distilled off under reduced pressure. The Residue was purified by silica gel chromatography, eluting with ethyl acetate-hexane (1:2) to give 7-acetyl-5-(3-bromopropylidene)-5,11-dihydro[1]benzoxepino[2,3-b]pyridine (7.9 g).
¹H-NMR (CDCl₃) δ:2.57(3H,s), 2.77(2H,m), 3.49(2H,t), 5.40(2H, brs), 6.16(1H,t),6.88(1H,d), 8.33(1H,dd), 7.58(1H,dd), 7.77(1H,dd), 7.96(1H,d), 8.56(1H,dd).

### Step 2

The titled compound was prepared by following the procedure of example 1, step 2, but replacing the product of example 1, step 1 with the product of step 1. ¹H-NMR (CDCl₃) δ:1.52-1.79(2H,m), 1.93-2.11(2H,m), 2.27-2.49(4H,m), 2.49-2.60(5H,m), 2.60-2.73(2H,m), 5.40(2H,brs), 6.22(1H,t),6.87(1H,d), 7.29-7.34(3H,m), 7.42(2H,d), 7.59(1H,dd), 7.75(1H,dd), 7.96(1H,d), 8.53(1H,dd).
MS m/z: 489(M+1)

### Example 7:

### 3-(4-chlorophenyl)-1-[3-(5,11-dihydro-7-(methoxy[1]benzoxepino[2,3-b]pyridin-5-ylidene)propyl]pyrrolidzne

### Step 1

A mixture of 1-benzyl-3-pyrrolidinone (10.0 g, 57 mmol), di-*tert*-butyl dicarbonate (13.7 g, 63 mmol) and palladium on active carbon (2.5 g, w/w 20%) in MeOH was shaken in a Parr hydrogenation vessel (50 psi H₂) for 48 hours. The reaction mixture was filtered through celite and concentrated in vacuo. Chromatographic purification on silica gel (Hexane/EtOAc =1/1) provided 6.21 g 1-*t*-butoxycarbonyl-3-pyrrolidinone (59%). ¹H NMR (250 MHz, CDC13) δ: 1.46 (9H, s), 2.57 (2H, t, *J* = 7.8 Hz), 3.71-3.75 (4H, m)

### Step 2

To a stirred solution of 1-*t*-butoxycarbonyl-3-pyrrolidinone (0.57 g, 3.23 mmol) in THF (10 mL) was added 4-chlorophenyl magnesium bromide (1.0 M, 5.2 mL) under the protection of argon at 0°C. The reaction was stirred at room temperature for 1 hour then quenched by the addition of saturated aqueous NH₄OH (8 mL). The aqueous layer was extracted with EtOAc (50 mL x 2), dried over MgSO₄ and concentrated in vacuo. Chromatographic purification on silica gel (Hexane/EtOAc = 3/1) provided 0.57 g 1-*t*-butoxycarbonyl-3-(4-chlorophenyl)-3-hydroxypyrrolidine (60%). m/z 298 (m+1)

### Step 3

To a stirred solution of 1-*t*-butoxycarbonyl-3-(4-chlorophenyl)-3-hydroxypyrrolidine (0.335 g, 1.28 mmol) in CH₂Cl₂ (8 mL) was added trifluoroacetic acid (2 mL) at 0°C slowly. The reaction was stirred at room temperature for 30 minutes and concentrated in vacuo. This provided 0.355 g 3-(4-chlorophenyl)-3-hydroxypyrrolidine (100%) the desired product. m/z 198 (m+1)

### Step 4

The titled compound was prepared by following the procedure for example 1 but replacing 4-(4-chlorophenyl)-4-hydroxypiperidine with 3-(4-chlorophenyl)-3-hydroxypyrrolidine. m/z (m+1).

### Example 18:

### (R)-2-[5-(3-{3-[(4-Chloro-benzyl)-ethyl-amino]-pyrrolidin-1-yl}-propylidene)-5,11-dihydro-10-oxa-1-aza-dibenzo[a,d]cyclohepten-7-yl]-propan-2-ol

(R)-Pyrrolidin-3-yl-carbamic acid tert-butyl ester (7.85 g, 42.1 mmol) was dissolved in methylene chloride at 0°C and triethylamine (11.72 mL, 84.3 mmol) and ethyl chloroformate (8.06 mL, 84.3 mmol) were added. The solution was warmed to room temperature and stirred for 30 min, the reaction was washed with NaHCO₃. The organics were combined and dried over MgSO₄, filtered and evaporated *in vacuo.* The residue was purified by Isco flash system (70% hexane/30% ethyl acetate) to yield the ester (7.73 g, 71%).

### Part 2:

(R)-3-*tert*-Butoxycarbonylamino-pyrrolidine-1-carboxylic acid ethyl ester (7.73 g, 29.9 mmol) was dissolved in 4M HCl/dioxane. The solution was stirred at rt for 1h. The solvent was removed *in vacuo* and the mixture was carried on to the next step without further purification as the hydrochloride salt.

### Part 3:

(R)-3-Amino-pyrrolidine-1-carboxylic acid ethyl ester and acetaldehyde (1.76 mL, 31.43 mmol) were mixed with sodium triacetoxyborohydride (9.54g, 45 mmol) in dichloroethane (200 mL) containing acetic acid (1%) and the resulting mixture was stirred at room temperature overnight. The reaction mixture was diluted with CH₂Cl₂ and washed with 1 N NaOH solution and brine and dried over magnesium sulfate. The reaction was concentrated *in vacuo.* The residue was used directly in the next reaction (4.0 g, 72%)

### Part 4:

(R)-3-Ethylamino-pyrrolidine-1-carboxylic acid ethyl ester (1.00g, 5.4 mmol) was dissolved in acetonitrile (100mL) and potassium carbonate (3.7 g, 27 mmol), KI (0.050 mg) and 1-bromomethyl-4-chloro-benzene (1.1 g, 5.4 mmol) were added. The solution was heated at 60 °C for 20 h. The reaction mixture was filtered and concentrated *in vacuo.* The residue was purified by Isco flash system (75% hexane/25% ethyl acetate) to yield coupled product (0.410 g, 25 %).

### Part 5:

(R)-3-[(4-Chloro-benzyl)-ethyl-amino]-pyrrolidine-1-carboxylic acid ethyl ester (4.00g, 1.29 mmol) was dissolved in 15 mL of ethanol and potassium hydroxide (1.5 g, 26.7 mmol) in water (8 mL) was added. The solution was heated to reflux for 14 h and the ethanol was removed *in vacuo.* The residue was partitioned between water and methylene chloride. The organics were removed and washed with sat'd solution of NaHCO₃ and brine, then dried over magnesium sulfate. The reaction was concentrated *in vacuo* (0.253 g, 82%).

### Part 6:

To a solution of the (R)-(4-chloro-benzyl)-ethyl-pyrrolidin-3-yl-amine (0.253 g, 1.06 mmol) in isopropanol was added 2,6-lutidine (0.225 g, 2.1 mmol) and catalytic potassium iodide. This mixture was heated to 80°C, and treated with 2-[5-(3-Bromo-propylidene)-5,11-dihydro-10-oxa-1-aza-dibenzo[*a,d*]cyclohepten-7-yl]-propan-2-ol (0.224 g, 0.6 mmol), added in portions over 1 h. The solution was then stirred at 80 °C for an additional 14 h. The reaction was concentrated *in vacuo*, then purified by Isco flash chromatography (15% methanol/85% methylene chloride) to yield the title compound (0.100 g, 31%). ¹H-NMR (MeOD): δ 0.94 (t, 3H), 1.40 (s, 6H), 1.88-2.01 (m, 1H), 2.02-2.18 (m, 1H), 2.42-2.67 (m, 4H), 3.03-3.36 (m, 7H), , 3.56 (d, 1H), 3.72. (d, 1H), 5.16 (bs, 2H), 6.03 (t, 1H), 6.77 (d, 1H), 7.22-7.37 (m, 4H), 7.38-7.47 (m, 2H), 7.69 (d, 1H), 8.30 (s, 1H), 8.37 (dd, 1H). ESI-MS m/z: 534 (M + 1), retention time 1.22.

### Example 19

### (R)-2-[5-(3-{3-[(4-Chloro-phenyl)-ethyl-amino]-pyrrolidin-1-yl}-propylidene)-5,11-dihydro-10-oxa-1-aza-dibenzo[a,d]cyclohepten-7-yl]-propan-2-ol

### Part 1:

(R)-3-Ethylamino-pyrrolidine-1-carboxylic acid ethyl ester (2.0 g, 10.8 mmol) and 1-bromo-4-chloro-benzene (2.06 g, 10.8 mmol) was dissolved in toluene along with sodium t-butoxide (1.45 g, 15.12 mmol), Pd₂(dba)₃ (0.195 g, 0.21 mmol) and BINAP (0.13 g, 0.21 mmol). The solution was heated to 100 °C for 2 days, then filtered, and the reaction was concentrated *in vacuo,* and then purified by Isco flash chromatography (50% ethyl acetate/ 50 % hexane) to yield the product (0.536 g, 17%).

### Part 2:

(R)-3-[(4-Chloro-phenyl)-ethyl-amino]-pyrrolidine-1-carboxylic acid ethyl ester (0.536g, 1.8 mmol) was dissolved in 15 mL of ethanol and potassium hydroxide (2.05 g, 36.2 mmol) in water (8 mL) was added. The solution was heated to reflux for 14 h and the ethanol was removed *in vacuo.* The residue was partitioned between water and methylene chloride. The organics were removed and washed with sat'd solution of NaHCO₃ and brine, then dried over magnesium sulfate. The reaction was concentrated *in vacuo* (0.273 g, 67%).

### Part 3:

To a solution of the (R)-(4-Chloro-phenyl)-ethyl-pyrrolidin-3-yl-amine (0.270 g, 1.2 mmol) in isopropanol was added 2,6-lutidine (0.20 g, 1.7 mmol) and catalytic potassium iodide. This mixture was heated to 80°C, and treated with 2-[5-(3-Bromo-propylidene)-5,11-dihydro-10-oxa-1-aza-dibenzo[*a*,*d*]cyclohepten-7-yl]-propan-2-ol (0.180 g, 0.48 mmol), added in portions over 1 h. The solution was then stirred at 80 °C for an additional 14 h. The reaction was concentrated *in vacuo*, then purified by Isco flash chromatography (15% methanol/85% methylene chloride) to yield the title compound (0.180 g, 72%). ¹H-NMR (CDCl₃): δ 1.08 (t, 3H), 1.54 (s, 6H), 1.68-1.92 (m, 2H), 2.08-2.25 (m, 1H), 2.36-2.92 (m, 7H), 3.26 (q, 2H), 42.8 (t, 1H), 5.29 (bs, 2H), 6.12 (t, 1H), 6.74 (d, 2H), 6.82 (d, 1H), 7.08 (d, 2H), 71.3-7.31 (m, 2H), 7.44 (s, 1H), 7.52 (d, 1H), 8.48 (dd, 1H). ESI-MS m/z: 520 (M + 1), retention time 2.01.

### Example 20

### (R)-2-{5-[3-(3-{[(4-Chloro-benzyl)-ethyl-amino]-methyl}-pyrrolidin-1-yl)-propylidene] 5,11-dihydro-10-oxa-1-aza-dibenzo[a,d]cyclohepten-7-yl}-propan-2-ol

### Part 1:

(R)-3-Aminomethyl-pyrrolidine-1-carboxylic acid tert-butyl ester (0.385 g, 1.92 mmol) and 1-bromomethyl-4-chloro-benzene (0.395 g, 1.92 mmol) were dissolved in acetonitrile at 0 °C (10 mL) and triethyl amine (0.793 mL, 5.76 mmol) was added. The solution was allowed to warm to room temperature and stir overnight. The reaction was concentrated down and partitioned between IN NaOH and extracted with CH₂Cl₂ (3x). The organics were collected together and dried over MgSO₄, filtered and evaporated *in vacuo.* The residue was purified by Isco flash system (20% hexane/80% ethyl acetate) to yield the product (0.35 g, 57 %).

### Part 2:

(R)- 3-[(4-Chloro-benzylamino)-methyl]-pyrrolidine-1-carboxylic acid tert-butyl ester (0.35 g, 0.1 mmol) and acetaldehyde (0.15 mL, 0.15 mmol) were mixed with sodium triacetoxyborohydride (0.35 g, 0.15 mmol) in dichloroethane (15 mL) containing acetic acid (1%) and the resulting mixture was stirred at room temperature overnight. The reaction mixture was diluted with CH₂Cl₂ and washed with 1 N NaOH solution and brine and dried over magnesium sulfate. The reaction was concentrated *in vacuo.* The residue was used directly in the next reaction (0.34 g, 100%)

### Part 3:

3-{[(4-Chloro-benzyl)-ethyl-amino]-methyl}-pyrrolidine-1-carboxylic acid tert-butyl ester (0.34 g, 0.98 mmol) was dissolved in 4M HCl/dioxane. The solution was stirred at rt for 1h. The solvent was removed in *vacuo* and the mixture. was carried on to the next step without further purification as the hydrochloride salt.

### Part 4:

To a solution of (R)- (4-Chloro-benzyl)-ethyl-pyrrolidin-3-ylmethyl-amine in acetonitrile/water (8:2) (10 mL) was added K₂CO₃ (0.54g, 3.9 mmol) and 2-[5-(3-bromo-propylidene)-5,11-dihydro-10-oxa-1-aza-dibenzo[a,d]cyclohepten-7-yl]-propan-2-ol (0.368 g, 0.98 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo,* then purified by Isco flash chromatography (5% methanol/95% methylene chloride/ ammonium hydroxide) to yield the title compound (0.110 g, 33 %). ¹H-NMR (CDCl₃): δ0.94 (t, 3H), 1.37 (m, ¹H), 1.48 (s, 6H), 1.76-2.15 (m, 4H), 2.22-2.68 (m, 10H), 3.41 (d, 1H), 3.52 (d, 1H), 5.28 (bs, 2H), 6.11 (t, 1H), 6.82 (d, 1H), 7.14-7.32 (m, 6H), 7.48 (s, 1H), 7.54 (d, 1H), 8.51 (dd, 1H). ESI-MS m/z: 548 (M + 1), retention time 1.02.

### Example 21

### (R)-5-(3-{3-[(4-Chloro-benzyl)-ethyl-amino]-pyirolidin-1-yl}-propylidene)-5,11-dihydro-10-oxa-1-aza-dibenzo[a,d]cycloheptene-7-carboxylic acid

### Part 1:

To a solution of (R)- (4-Chloro-benzyl)-ethyl-pyrrolidin-3-yl-amine (0.43 g, 1.8 mmol) in acetonitrile/water (8:2) was added K₂CO₃ (0.99 g, 7.2 mmol) and 1-[5-(3-Bromo-propylidene)-5,11-dihydro-10-oxa-1-aza-dibenzo[*a*,*d*]cyclohopten-7-yl]-ethanone (0.65 g, 1.8 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo,* then purified by Isco flash chromatography (5% methanol/ 95% methylene chloride/ ammonium hydroxide) to yield the ketone tricycle (0.683 g, 73 %). ¹H-NMR (MeOD): δ 0.96 (t. 3H), 1.39 (dd, 1H), 1.48 (dd, 1H), 1.84 (s, 1H), 1.81 (m, 1H), 1.94 (m, 1H), 2.33-2.47 (m, 2H), 2.53 (s, 3H), 2.58 -2.66 (m, 2H), 2.68-2.86 (m, 4H), 5.42 (bs, 2H), 6.24 (t, 1H), 6.81 (d, 1H), 7.17-7.32 (m, 4H), 7.46 (dd, 1H), 7.77 (t, 2H), 7.96 (s, 1H), 8.48 (d, 1H). ESI-MS m/z: 517 (M+ 1), retention time 2.61.

### Part 2:

Bromine (0.13 mL, 2.5 mmol) was added dropwise to a solution of NaOH (0.332g, 8.3 mmol) in water (3 mL) at 10 °C. The solution was cooled to 0 °C and (R)-1-[5-(3-{3-[(4-Chloro-benzyl)-ethyl-amino]-pyrrolidin-1-yl}-propylidene)-5,11-dihydro-10-oxa-1-aza-dibenzo[*a*,*d*]cyclohepten-7-yl]-ethanone (0.43 g, 0.83 mmol) dissolved in dioxane (8 mL) was added to the solution dropwise. The reaction was allowed to warm to room temperature and stirred for 3 h. The reaction was evaporated *in vacuo* and purified by HPLC. ¹H-NMR (MeOD): δ 0.96 (t. 3H), 1.37 (m, 1H), 1.48 (m, 1H), 1.95 (s, 1H), 1.94 (m, 1H), 2.10 (m, 1H), 2.42-2.63 (m, 3H), 2.90 (t, 1H), 2.97 -3.23 (m, 3H), 5.82 (bs, 2H), 6.26 (t, 1H), 6.65 (d, 1H), 7.13-7.31 (m, 4H), 7.39 (t, 1H), 7.61 (d, 1H), 7.67 (d, 1H), 7.86 (s, 1H), 8.37 (d, 1H). ESI-MS m/z: 519 (M + 1), retention time 1.91.

### Example 22

### 2-{5-[3-(3-{[2-(4-Chloro-phenyl)-ethyl]-ethyl-amino}-pyrrolidin-1-yl)-propylidene]-5,11-dihydro-10-oxa-1-aza-dibenzo[a,d]cyclohepten-7-yl}-propan-2-ol

### Part 1:

To a solution of 3-amino-pyrrolidine-1-carboxylic acid tert-butyl ester (0.300g, 1.37 mmol) dissolved in dimethylformamide was added K₂CO₃ (0.567g, 4.11 mmol) and 1-(2-bromo-ethyl)-4-chloro-benzene (0.30 g, 1.37 mmol). The solution was heated to 60°C for 2 d and then concentrated down and partitioned between EtOAc/H₂O, extracted with EtOAc (3x). The organics were collected together dried over Mg₂SO₄, filtered and evaporated *in vacuo,* then purified by Isco flash chromatography (60% ethyl acetate/40% hexane) to yield the intermediate compound (0.263 g, 59 %).

### Part 2:

(R)- 3-[2-(4-Chloro-phenyl)-ethylamino]-pyriolidine-1-carboxylic acid tert-butyl ester (0.263 g, 0.81 mmol) and acetaldehyde (0.068 mL, 1.2 mmol) were mixed with sodium triacetoxyborohydride (0.25 g, 1.2 mmol) in dichloroethane containing acetic acid (1%) and the resulting mixture was stirred at room temperature overnight, The reaction mixture was diluted with CH₂Cl₂ and washed with 1 N NaOH solution and brine and dried over magnesium sulfate. The reaction was concentrated *in vacuo.* The residue was used directly in the next reaction (0.24 g, 85 %)

### Part 3:

(R)-3-{[2-(4-Chloro-phenyl)-ethyl]-ethyl-amino}-pyrrolidine-1-carboxylic acid tert-butyl ester (0.242 g) was dissolved in 4M HCl/dioxane. The solution was stirred at rt for 1h. The solvent was removed *in vacuo* and the mixture was carried on to the next step without further purification as the hydrochloride salt.

### Part 4:

To a solution of (R)-[2-(4-Chloro-phenyl)-ethyl]-ethyl-pyrrolidin-3-yl-amine (0.100 g, 0.39 mmol) in acetonitrile/water (8:2) (10 mL) was added K₂CO₃( 0.837 g, 6.06 mmol) and 2-[5-(3-Bromo-propylidene)-5,11-dihydro-10-oxa-1-aza-dibenzo[*a,d*]cyclohepten-7-yl]-propan-2-ol (0.100 g, 0.26 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated in *vacuo,* then purified by Isco flash chromatography (5% methanol/ 95% methylene chloride/ ammonium hydroxide) to yield the title compound (0.048 g, 33 %). ¹H-NMR (CDCl₃): δ 1.06 (t, 3H), 1.52 (s, 6H), 1.58 (m, 1H), 2.10 (m, 1H), 2.20-2.78 (m, 13H), 3.30(m, 1H), 5.28 (bs, 2H), 6.12 (t, 1H), 6.80 (d, 1H), 7.06 (d, 2H), 7.14-7.30 (m, 4H), 7.46 (s, 1H), 7.52 (d, 1H), 8.52 (dd, 1H). ESI-MS m/z: 548 (M+1), retention time 1.35

### Example 23

### (S)-2-(5-{3-[3-(4-Chloro-benzyloxy)-pyrrolidin-1-yl]-propylidene}-5,11-dihydro-10-oxa-1-aza-dibenzo[a,d]cyclohepten-7-yl)-propan-2-ol

To a solution of (S) -3-hydroxy-pyrrolidine-1-carboxylic acid tert-butyl ester (0.5 g, 2.67 mmol) in THF (10 mL) was added NaH (0.128g, 3.2 mmol) (60 % dispersion in oil). The resulting solution was stirred for 5 min. and 1-bromomethyl-4-chloro-benzene (0.658g, 3.2 mmol) dissolved in THF (5 mL) was added. The reaction mixture was stirred overnight. The reaction was quenched with water and extracted with ethyl acetate (3x). The organics were collected together dried over Mg₂S₄, filtered and evaporated *in vacuo,* then purified by Isco flash chromatography (15% ethyl acetate/ 85% hexane) to yield the intermediate compound (0.48 g, 58 %).

### Part 2:

(S)-3-(4-Chloro-benzyloxy)-pyrrolidine-1-carboxylic acid tert-butyl ester (0.48 g) was dissolved in 4M HCl/dioxane. The solution was stirred at rt for 1h. The solvent was removed *in vacuo* and the mixture was carried on to the next step without further purification as the hydrochloride salt.

### Part 3:

To a solution of (S)- 3-(4-chloro-benzyloxy)-pyrrolidine (0.38 g, 1.54 mmol) in acetonitrile/water (8:2) (10 mL) was added K₂CO₃ (0.85 g, 6.15 mmol) and 2-[5-(3-bromo-propylidene)-5,11-dihydro-10-oxa-1-aza-dibenzo[*a*,*d*]cyclohepten-7-yl]-propan-2-ol (0.575 g, 1.54 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo*, then purified by Isco flash chromatography (5% methanol/ 95% methylene chloride/ ammonium hydroxide) to yield the title compound (0.112 g, 14 %). ¹H-NMR (CDCl₃): δ 1.54 (s, 6H), 1.81 (m, 1H), 1.94-2.12 (m, 2H), 2.31 (t, 3H), 2.48-2.72 (m, 4H), 4.10 (m, 1H), 4.38 (q, 2H), 5.28 (bs, 2H), 6.12 (t, 1H), 6.84 (d, 1H), 7.16-7.30 (m, 6H), 7.48 (s, 1H), 7.56 (d, 1H), 8.52 (dd, 1H). ESI-MS m/z: 507 (M + 1), retention time 1.56.

### Example 24

### 1-{3-[7-(1-Hydroxy-1-methyl-ethyl-11H-10-oxa-1-aza-dibenzo[a,d]cyclohepten-5-ylidene]-propyl}-pyrrolidine-3-carboxylic acid 4-chloro-benzylamide

### 3-(4-Chloro-benzylcarbamoyl)-pyrrolidine-1-carboxylic acid tert-butyl ester

To a solution of di-tert-butyl-dicarbonate (1.86 g, 8.5 mmol) in dioxane (100 mL) was added 3-pyrrolidine carboxylic acid (1.0 g, 8.5 mmol) and 1 N NaOH (5 mL). The solution was stirred at rt for 12h. The reaction was concentrated down and partitioned between EtOAc/ 1N HCl. The reaction was quenched with 1N HCl and extracted with EtOAc (3X), and the organic layers were collected together, dried over MgSO₄ and evaporated *in vacuo.* The residue was was used directly in the next reaction.

### Step 2:

Pyrrolidine-1,3-dicarboxylic acid (1.59g, 7.3 mmol) was dissolved in CH₂Cl₂ and EDCI (2.53g, 13.2 mmol), HOBt (1.48g, 10.9 mmol) and 4-chlorobenzylamine (0.97 mL, 8.05 mmol) was added. The solution was allowed to stir at room temperature for 10h and then washed 1N NaOH (1x), IN HCl (1x) and brine (1x). The organic layers was dried over MgSO₄ and evaporated *in vacuo*, then purified by Biotage flash chromatography (50% ethyl acetate/ 50% hexane) to yield the title compound (1.50 g, 62 %).¹H-NMR (CDCl₃): δ 1.43 (s, 9H), 2.07 (m, 2H), 2.85 (pentet, 1H), 3.30 (q, 1H), 3.49 (q, 1H), 3.55 (q, 2H), 4.38 (d, 2H), 6.16 (bs, 1H), 7.17 (d, 2H), 7.27 (d, 2H).

3-(4-Chloro-benzylcarbamoyl)-pyrrolidine-1-carboxylic acid *tert-*butyl ester (0.707g, 2.07 mmol) was dissolved in 4M HCl/Dioxane (5 mL). The solution was stirred at rt for 1h. The solvent was removed *in vacuo* and the mixture was carried on to the next step without further purification as the hydrochloride salt.

To a solution of pyrrolidine-3-carboxylic acid 4-chloro-benzylamide hydrochloride (0.2 g, 0.83 mmol) in acetonitrile/water (8:2) (8 mL) was added K₂CO₃ (0.476g, 3.4 mmol) and 2-[5-(3-bromo-propylidene)-5,11-dihydro-10-oxa-1-aza-dibenzo[*a,d*]cyclohepten-7-yl]-propan-2-ol (0.282 g, 0.83 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo,* then purified by Biotage flash chromatography (5% methanol/ 95% methylene chloride to 10% methanol/ 90% methylene chloride) to yield the title compound (0.240 g, 60 %).¹H-NMR (CDCl₃): 1.54 (s, 6H), 1.93 (m, 1H), 2.12 (m, 2H), 2.29- 2.82 (m, 10H), 4.26 (bs, 2H), 5.26(BS, 1h), 6.05 (t, 1H), 6.79 (d, 1H), 7.03 (d, 2H), 7.19-7.26 (m, 4H), 7.42 (s, 1H), 7.51 (d, 1H), 8.46 (d, 1H). ESI-MS m/z: 532.05 (M + 1), retention time 1.68.

### Example 25

### 1-{3-[7-(1-Hydroxy-1-methyl-ethyl)-11H-10-oxa-1-aza-dibenzo[a,d]cyclohepten-5-ylidene]-propyl}-pyrrolidine-3-carboxylic acid (4-chloro-phenyl)-ethyl-amide

### 3-[(4-Chloro-phenyl)-ethyl-carbamoyl)]-pyrrolidine-1-carboxylic acid tert-butyl ester

To a solution of di-tert-butyl-dicarbonate (1.86 g, 8.5 mmol) in dioxane (100 mL) was added 3-pyrrolidine carboxylic acid (1.0 g, 8.5 mmol) and 1 N NaOH (5 mL). The solution was stirred at rt for 12h. The reaction was concentrated down and partitioned between EtOAc/ IN HCl. The reaction was quenched with 1N HCl and extracted with EtOAc (3X), and the organic layers were collected together, dried over MgSO₄ and evaporated *in vacuo.* The residue was used directly in the next reaction.

### Step 2:

Pyrrolidine-1,3-dicarboxylic acid (0.300g, 1.4 mmol) was dissolved in CH₂Cl₂ and EDCI (0.477g, 2.4 mmol), HOBt (0.280g, 2.1 mmol) and N-ethyl-4-chloro aniline (0.236 g, 1.5 mmol) was added. The solution was allowed to stir at room temperature for 24h and then washed IN NaOH (Ix), IN HCl (1x) and brine (1x). The organic layers was dried over MgSO₄ and evaporated *in vacuo,* then purified by Biotage flash chromatography (30% ethyl acetate/ 70% hexane to 40% ethyl acetate/ 60% hexane) to yield the title compound 0.040 g, 8 %).¹H-NMR (CDCl₃): δ 1.08 (t, 3H), 1.41 (s, 9H), 1.78 (m, 1H), 2.13 (m, 1H), 2.76 (pentet, 1H), 3.10 (q, 1H), 3.36 (pentet, 2H), 3.50 (t, 1H), 3.73 (m, 2H), 7.09 (d, 2H), 7.42 (d, 2H).

3-[(4-Chloro-phenyl)-ethyl-carbamoyl)]-pyrrolidine-1-carboxylic acid *tert-*butyl ester (0.040 g, 0.1 mmol) was dissolved in 4M HCl/Dioxane (2 mL). The solution was stirred at rt for 1h. The solvent was removed *in vacuo* and the mixture was carried on to the next step without further purification as the hydrochloride salt.

To a solution of pyrrolidine-3-carboxylic acid (4-chloro-phenyl)-ethylamide hydrochloride (0.033 g, 0.11 mmol) in acetonitrile/water (8:2) (2 mL) was added K₂CO₃ (0.125g, 0.89 mmol) and 2-[5-(3-bromo-propylidene)-5,11-dihydro-10-oxa-1-aza-dibenzo[a,d]cyclohepten-7-yl]-propan-2-ol (0.042 g, 0.11 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated in *vacuo,* then purified by Biotage flash chromatography (2 1/2% methanol/ 971/2% methylene chloride to 5% methanol/ 95% methylene chloride) to yield the title compound (0.028 g, 46 %).¹H-NMR (CDCl₃): δ 1.07 (t, 3H), 1.55 (6H, s), 1.62 (m, 1H), 1.99 (m, 3H), 2.30 (pentet, 3H), 2.47-2.77 (m, 5H), 3.68 (m, 2H), 5.26 (bs, 2H), 6.09 (t, 1H), 6.79 (d, 1H), 7.03 (d, 2H), 7.24 (m, 2H), 7.37 (d, 2H), 7.42 (s, 1H), 7.52 (d, 1H), 8.47 (d, 1H). ESI-MS m/z: 546.04 (M + 1), retention time 1.44.

### Example 26

### (R)-2-(5-{3-[3-(5-Chloro-1,3-dihydro-isoiadol-2-yl)-pyrrolin-1-y;]-propylidene-5,11-dihydro-10-oxa-1-aza-dibenzo[a,d]cyclohepten-7-yl-propan-2-ol

### (R)-3-(5-Chloro-1,3-dioxo-1,3-dihydro-isoindol-2-yl)-pyrrolidine-1-carboxylic acid tert-butyl ester

4-Chlorophthalic acid monosodium (2.00g, 8.9 mmol) and PCl₅ (5.61g, 26.9 mmol) were added neat to a flask and heated to 180 °C (caution the initial mixture of the acid and PCl₅ was very exothermic). The mixture was heated for 3h and then the suspension was diluted with toluene and filtered. The reaction was concentrated down and used without further purification.

### Part 2:

(R)-4-Chloro-phthaloyl dichloride (0.556g, 2.3 mmol) and (R)-3-amino-1-*N-*Boc-pyrrolidine (0.400g, 2.1 mmol) were dissolved in 21 mL of pyridine and heated to 80 °C for 2 1/2 h. The solution was diluted with water and extracted with EtOAc. The organic were collected together and washed with 1N HCl, IN NaOH, brine and dried over Mg₂SO₄, filtered and evaporated *in vacuo,* then purified by Biotage flash chromatography (5 % methanol/ 95 % methylene chloride to 10% ethyl acetate/ 90% hexane to 20% ethyl acetate/80% hexane) to yield the title compound (0.200 g, 26 %). ¹H-NMR (CDCl₃): δ 1.46 (s, 9H), 2.12 (m, 1H), 2.59 (pentet, 1H), 3.38 (quartet, 1H), 3.71 (m, 3H), 4.83 (pentet, 1H), 7.69 (d, 1H), 7.77 (d, 1H), 7.80 (s, 1H). ESI-MS m/z: 351 (M + 1), retention time 2.73.

### (R)-3-(5-Chloro-1,3-dihydro-isoindol-2-yl)-pyrrolidine-1-carboxylic acid tert-butyl ester

(R)-3-(5-Chloro-1,3-dioxo-1,3-dihydro-isoindol-2-yl)-pyrrolidine-1-carboxylic acid *tert-*butyl ester (0.200g, , 0.57 mmol) was dissolved in ether and cooled to 0 °C and LiAlH₄ (2.85 mL, 2.8 mmol) was added dropwise, followed by AlCl₃ (0.380g, 2.8 mmol). The solution was allowed to stir at 0 °C for 1 ½ h and then slowly quenched with water and extracted with ethyl acetate (3x). The organic were collected together and dried over Mg₂SO₄, filtered and evaporated *in vacuo,* to yield the title compound (0.076 g, 41 %) without further purification. ESI-MS m/z: 323 (M + 1), retention time 1.27.

(R)-3-(5-Chloro-1,3-dihydro-isoindol-2-yl)-pyrrolidine-1-carboxylic acid *tert-*butyl ester (0.076 g, 0.23 mmol) was dissolved in 4M HCl/Dioxane (2 mL). The solution was stirred at rt for 1h. The solvent was removed *in vacuo* and the mixture was carried on to the next step without further purification as the hydrochloride salt.

### Step 2:

To a solution of (R)-5-chloro-2-pyrrolin-3-yl-2,3-dihydro-1*H-*isoindole hydrochloride (0.052 g, 0.23 mmol) in acetonitrile/water (8:2) (2 mL) was added K₂CO₃(0.215g, 1.5 mmol) and 2-[5-(3-bromo-propylidene)-5,11-dihydro-10-oxa-1-aza-dibenzo[a,d]cyclohepten-7-yl]-propan-2-ol (0.079 g, 0.21 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated in *vacuo,* then purified by Biotage flash chromatography (5% methanol/ 95% methylene chloride to 10% methanol/ 90% methylene chloride to 15% methanol/85% methylene chloride) to yield the title compound (0.055 g, 55 %).¹H-NMR (CDCl₃): δ 1.55(s, 9H), 1.77 (m, 1H), 2.02 (m, 2H), 2.37(pentet, 3H), 2.42-2.64 (m, 4H), 2.81 (t, 1H), 3.17 (pentet, 1H), 3.43 (s, 3H), 3.84 (s, 4H), 5.26 (bs, 2H), 6.11 (t, 1H), 6.79 (d, 1H), 7.08-7.28 (m, 5H), 7.43 (s, 1H), 7.56 (d, 1H), 8.46 (d, 1H). ESI-MS m/z: 516.02 (M + 1), retention time 1.26.

### Example 27

### (R)-1-(4-Chloro-benzyl)-1-(1-{3-[7-(1-hydroxy-1-methyl-ethyl)-11H-10-oxa-1-aza-dibenzo[a,d]cyclohepten-5-ylidene]-propyl}-pyrrolidin-3-yl)urea (R)-1-(4-Chloro-benzyl)-1-pyrrolidin-3-yl-urea

(R)-3-(4-Chloro-benzylamino)-pyrrolidine-1-carboxylic acid *tert*-butylester (0.690g, 2.2 mmol) was dissolved in CH₂Cl₂ with a few drops of isopropanol and trimethylsilylisocyanate (0.365 mL, 2.6 mmol) were added. The solution was allowed to stir at room temperature for 12h, the reaction was concentrated down and used directly in the next reaction. ESI-MS m/z: 354 (M + 1), retention time 2.15.

### Part 2:

(R)-3-[1(4-Chloro-benzyl)-ureido]-pyrrolidine-1-carboxylic acid *tert*-butyl ester (0.300 g, 0.84 mmol) was dissolved in 4M HCl/Dioxane (2 mL). The solution was stirred at rt for 1h. The solvent was removed *in vacuo* and the mixture was carried on to the next step without further purification as the hydrochloride salt.

To a solution of (R)-1-(4-Chloro-benzyl)-1-pyrrolidin-3-yl-urea (0.200g, 0.78 mmol) in acetonitrile/water (8:2) (8 mL) was added K₂CO₃(0.449 g, 3.2 mmol) and 2-[5-(3-bromo-propylidene)-5,11-dihydro-10-oxa-1-aza-dibenzo[a,d]cyclohepten-7-yl]-propan-2-ol (0.267 g, 0.71 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo,* then purified by Biotage flash chromatography (5% methanol/ 95% methylene chloride to 10% methanol/ 90% methylene chloride) to yield the title compound (0.231 g, 60 %).¹H-NMR (CDCl₃): δ 1.55 (s, 6H), 1.88 (pentet, 1H), 2.01-2.28 (m, 5H), 2.53 (pentet, 6H), 3.12 (t, 1H), 3.14 (t, 1H), 3.83 (q, 1H), 4.37 (s, 1H), 4.53 (s, 2H), 5.28 (bs, 2H), 6.18 (t, 1H), 6.78 (d, 1H), 7.11 (dd, 1H), 7.16 (s, 1H), 7.18-7.27 (m, 4H), 7.49 (d, 1H), 7.51 (s, 1H), 8.47 (dd, 1H). ESI-MS m/z: 547.06 (M + 1), retention time 1.65.

### Example 28

### (R)-8-Chloro-4-(1-{3[7-(-hydroxy-1-methyl-ethyl)-11H-10-oxa-1-aza-dibenzo[a,d] cyclohepten-5-ylidene]-propyl}-pyrrolidine-3-yl)-1,3,4,5-tetrahydro-benzo[e][1,4] diazepin-2-one

### Step 1:3-(4-Chloro-2-nitro-benzylamino)-pyrrolidine-1-carboxylic acid tert-butyl ester

To a solution of 3-Amino-pyrrolidine-1-carboxylic acid tert-butyl ester (2 g, 10.7 mmol) in methanol (20 mL) was added 4-Chloro-2-nitro-benzaldehyde (1.98 g, 10.7 mmol). The resultant solution was heated at 60 °C overnight. The reaction mixture was cooled to 0 °C and to it was added NaBH4 (629 mg, 10 mmol). After 1 h, the reaction solution was quenched with saturated NaHC03 solution and extracted with ethyl acetate. The organic extract was dried over MgS04, filtered and concentrated to provide the crude product as an oil. The crude product was used in the subsequent reaction without further purification. LCMS (retention time = 1.28, ES+; 356)

### Step 2: 3-[(4-Ghloro-benzyl)-methoxycarbonylmethyl-amino]-pyrrolidine-1-carboxylic acid tert-butyl ester

To a solution of 3-(4-Chloro-2-nitro-benzylamino)-pyrrolidine-1-carboxylic acid tert-butyl ester (3.8 g, 10.7 mmol) in DMF (40 mL) was added Bromo-acetic acid methyl ester (1.01 mL, 10.7 mmol) and K₂CO₃ (1.48 g, 10.7 mmol). The resultant suspension was stirred at 70 °C overnight. The reaction mixture was diluted with water and ethyl ether. The organic extract was dried over MgSO₄, filtered and concentrated to provide the desired product as an oil. The crude product was purified on SiO₂ and concentrated to provide the desired product as an oil (2 g, 44%). LCMS (retention time = 3.03, ES+ 428)

### Step 3: 3-(8-Chloro-2-oxo-1,2,3,5-tetrahydro-benzo[e][1,4]diazepin-4-yl)-pyrrolidine-1-carboxylic acid tert-butyl ester

To a solution of 3-[(4-Chloro-2-nitro-benzyl)-methoxycarbonylmethyl-amino]-pyrrolidine-1-carboxylic acid tert-butyl ester (1.04 g, 2.4 mmol) in ethyl acetate (20 mL) was added 10% Pd/C. Hydrogenolysis of the reaction solution at rt under atmospheric pressure provided the desired product after 1.5 h. The reaction mixture was filtered through a pad of celite and concentrated. The crude product (662 mg, 1.7 mmol) was dissolved in DMF (5 mL) and to it was added 60% NaH (100 mg, 2.5 mmol). The resultant solution was stirred at it overnight. The reaction was quenched with water and extracted with ethyl ether. The organic extract was dried over MgSO₄, filtered and concentrated to get the crude product. Purification on SiO₂ provided the desired product. LCMS (retention time = 1.89, ES+; 310)

(R)-3-(8-Chloro-2-oxo-1,2,3,5-tetrahydro-benzo[*e*][1,4]diazepin-4-yl)pyrrolidine-1-carboxylic acid *tert*-butyl ester (0.210 g, 0.57 mmol) was dissolved in 4M HCl/Dioxane (5.7 mL). The solution was stirred at rt for 1h. The solvent was removed *in vacuo* and the mixture was carried on to the next step without further purification as the hydrochloride salt.

### Step 2:

To a solution of (R)-8-chloro-4-pyrrolidin-3-yl-1,3,4,5-tetrahydro-benzo[*e*][1,4]diazepin-2-one (0.198 g, 0.58 mmol) in acetonitrile/water (8:2) (5.8 mL) was added K₂CO₃ (0.655g, 4.6 mmol) and 2-[5-(3-bromo-propylidene)-5,11-dihydro-10-oxa-1-aza-dibenzo[a,d]cyclohepten-7-yl]-propan-2-ol (0.183 g, 0.48 mmol). The solution was allowed to stir at room temperature for 48 h. The reaction was concentrated down and partitioned between EtOAc/H₂O, extracted with EtOAc (3x). The organics were collected together dried over Mg₂SO₄, filtered and evaporated *in vacuo,* then purified by Biotage flash chromatography (5% methanol/ 95% methylene chloride to 10% methanol/ 90% methylene chloride to 15% methanol/85% methylene chloride) to yield the title compound (0.077 g, 28 %).¹H-NMR (CDCl₃): δ1.55 (s, 6H), 1.74 (septet, 1H), 2.01 (sextet, 1H), 2.32-2.60 (m, 6H), 2.76 (t, 1H), 3.26 (pentet, 1H), 3.43 (d, 1H), 3.46 (s, 2H), 3.74 (s, 2H), 5.27 (bs, 2H), 6.12 (t, 1H), 6.78 (d, 1H), 6.96 (s, 1H), 7.06 (dd, 1H), 7.13 (d, 1H), 7.19 - 7.27 (m, 2H), 7.44 (d, 1H), 7.55 (dd, 1H), 8.47 (dd, 1H), 8.49 (s, 1H). ESI-MS m/z: 559 (M + 1), retention time 1.24.

### Example 29

### (R)-8-Chloro-1-ethyl-4-(1-{3[7-(-hydroxy-1-methyl-ethyl)-11H-10-oxa-1-aza-dibenzo[a,d] cyclohepten-5-ylidene]-propyl}-pyrolidine-3-yl)-1,3,4,5-tetrahydro-benzo[e][1,4]diazepin-2-one

### (R)-3-(8-Chloro-1-ethyl-2-oxo-1,2,3,5-tetrahydro-benzo[e][1,4]diazepin-4-yl)pyrrolidine-1-carboxylic acid tert-butyl ester

(R)-3-(8-Chloro-2-oxo-1,2,3,5-tetrahydro-benzo[*e*][1,4]diazepin-4-yl)pyrrolidine-1-carboxylic acid *tert*-butyl ester (0.258g, 0.70 mmol) was dissolved in THF (7mL) and cooled to 0°C. To the solution was added NaH (0.042g, 1.0 mmol) and stirred for 15 min, followed by ethyl iodide (0.085 mL, 1.0 mmol). The solution was allowed to warm to room temperature slowly and stir at room temperature for 14h. The reaction was quenched with water and extracted with EtOAc (3x). The organics were collected together dried over Mg₂SO₄, filtered and evaporated *in vacuo,* then purified by Biotage flash chromatography (75% ethyl acetate/ 25% ethyl acetate to 100% ethyl acetate) to yield the title compound (0.130 g, 47 %).¹H-NMR (CDCl₃): δ 1.16 (t, 3H), 1.43 (s, 9H), 1.75 (sextet, 1H), 2.18 (pentet, 1H), 3.05 -3.9 (m, 1H), 7.20 (m, 3H). ESI-MS m/z: 394 (M + 1), retention time 2.25.

(R)-3-(8-Chloro-1-ethyl-2-oxo-1,2,3,5-tetrahydro-benzo[*e*][1,4]diazepin-4-yl)pyrrolidine-1-carboxylic acid *tert*-butyl ester (0.130 g, 0.32 mmol) was dissolved in 4M HCl/Dioxane (3.2 mL). The solution was stirred at rt for 1h. The solvent was removed *in vacuo* and the mixture was carried on to the next step without further purification as the hydrochloride salt.

### Step 2:

To a solution of (R)-8-chloro-1-ethyl-4-pyrrolidin-3-yl-1,3,4,5-tetrahydro-benzo[*e*][1,4]diazepin-2-one (0.121 g, 0.32 mmol) in acetonitrile/water (8:2) (3.2 mL) was added K₂CO₃ (0.368g, 2.6 mmol) and 2-[5-(3-bromo-propylidene)-5,11-dihydro-10-oxa-1-aza-dibenzo[a,d]cyclohepten-7-yl]-propan-2-ol (0.102 g, 0.27 mmol). The solution was allowed to stir at room temperature for 48 h. The reaction was concentrated down and partitioned between EtOAc/H₂O, extracted with EtOAc (3x). The organics were collected together dried over MgₐSO₄, filtered and evaporated *in vacuo,* then purified by Biotage flash chromatography (5% methanol/ 95% methylene chloride to 10% methanol/90% methylene chloride to 15% methanol/85% methylene chloride) to yield the title compound (0.033 g, 21 %).¹H-NMR (CDCl₃): δ1.17 (t, 3H), 1.55 (s, 6H), 1.73 (septet, 1H), 2.08, (m, 1H), 2.34(pentet, 2H), 2.43-2.61 (m, 4H), 2.85 (t, 1H), 2.92 (d, 1H), 3.10 (q, 1H), 3.22 (m, 1H), 3.52 (q, 2H), 3.91 (septet, 2H), 5.29 (bs, 2H), 6.12 (t, 1H), 6.79 (dd, 1H), 7.15-7.28 (m, 5H), 7.44 (s, 1H), 7.56 (d, 1H), 8.47 (d, 1H). ESI-MS m/z: 587 (M + 1), retention time 1.37.

### Example 30

### [(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-acetic acid methyl ester

### Step 1: 3-(4-Chloro-benzylamino)-pyrrolidine-1-carboxylic acid tert-butyl ester

To a solution of 3-Amino-pyrrolidine-1-carboxylic acid tert-butyl ester (2 g, 10.7 mmol) in methanol (20 mL) was added 4-Chloro-benzaldehyde (1.5 g, 10.7 mmol). The resultant solution was heated at 60 °C overnight. The reaction mixture was cooled to 0 °C and to it was added NaBH4 (629 mg, 10 mmol). After 1 h, the reaction solution was quenched with saturated NaHC03 solution and extracted with ethyl acetate. The organic extract was dried over MgSO4, filtered and concentrated to provide the crude product as an oil. The crude product was used in the subsequent reaction without further purification. LCMS (retention time = 1.27, ES+ 311)

### Step 2: 3-[(4-Chloro-benzyl)-methoxycarbonylmethyl-amino]-pyrrolidine-1-carboxylic acid tert-butyl ester

To a solution of 3-(4-Chloro-benzylamino)-pyrrolidine-1-carboxylic acid tert-butyl ester (3.3 g, 10.7 mmol) in DMF (40 mL) was added Bromo-acetic acid methyl ester (1.01 mL, 10.7 mmol) and K₂CO₃ (1.48 g, 10.7 mmol). The resultant suspension was stirred at 70 °C overnight. The reaction mixture was diluted with water and ethyl ether. The organic extract was dried over MgSO₄, filtered and concentrated to provide the desired product as an oil. The crude product was purified on SiO₂ and concentrated to provide the desired product as an oil (3.5 g, 87%). LCMS (retention time = 2.96, ES+ 383)

### Step 3: [(4-Chloro-benzyl)-pyrrolidin-3-yl-amino]-acetic acid methyl ester

3-[(4-Chloro-benzyl)-methoxycarbonylmethyl-amino]-pyrrolidine-1-carboxylic acid tert-butyl ester (3.5 g, 9.1 mmol) was dissolved in 4N HCl in dioxane (60 mL). The resultant solution was stirred for 2 h at rt then concentrated to provide the desired product as a white solid. LCMS (retention time =1.26, ES+ 283)

### Step 4: [(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-acetic acid methyl ester

To a solution of [(4-Chloro-benzyl)-pyrrolidin-3-yl-amino]-acetic acid methyl ester (188 mg, 0.67 mmol) in isopropanol (5 mL) was added 2,6-lutidine (186 uL, 1.6 mmol), and a catalytic amount of potassium iodide. The resultant solution was heated at 80 °C and to it was added 2-[5-(3-Bromo-propylidene)-5,11-dihydro-10-oxa-1-aza-dibenzo[a,d]cyclohepten-7-yl]-propan-2-ol (149 mg, 0.4 mmol) portionwise over 0.5 h. The reaction solution was heated overnight then partitioned between water and ethyl acetate. The product was purified on SiO₂ (ethyl acetate: Methanol: triethylamine 95:4.5:0.4) and isolated as a film. LCMS (retention time = 1.84, ES+; 576); ¹H NMR (CD₃OD): δ 8.46 (1H,d), 7.76 (1H, d), 7.45 (2H, m), 7.23 (5H, m), 6.74 (1H, d), 6.15 (1H, m), 3.72 (2H, m), 3.60 (4H, s), 3.30 (6H, m), 2.78 (4H, m), 2.42 (2H, m), 2.05 (1H, m), 1.82 (1H, m), 1.50 (6H, s).

### Example 31

### [(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-acetic acid

### Step 1: [(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-acetic acid

To a solution of [(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-acetic acid methyl ester (116 mg, 0.2 mmol) in THF:H₂O (4:1, 10 mL total volume) was added lithium hydroxide monohydrate (17 mg, 0.4 mmol). The resultant solution was stirred at rt for 3 h. The reaction solution was quenched with IN HCl and extracted with ethyl ether, dried over MgSO₄, filtered and concentrated. The final product was filtered through filter paper and re-concentrated. This product was submitted without further purification. LCMS (retention time = 1.72, ES+ 562); ¹H NMR (CD₃OD): δ 8.48 (1H, d), 7.78 (1H, d), 7.48 (2H, m), 7.31 (5H, m), 6.76 (1H, d), 6.15 (1H, t), 3.85 (2H, dd), 3.60 (2H, m), 3.30 (7H, m), 3.24 (1H, s), 3.18 (1H, m), 3.06 (1H, m), 2.58 (1H, m), 2.48 (1H, t), 2.06 (2H, m), 1.50 (6H, s).

### Example 32

### 3-(4-Chloro-phenyl)-1-ethyl-1-(1-{3-[7-(1-hydroxy-1-methyl-ethyl)-11H-10-oxa-1-aza-dibenzo [a,d]cyclohepten-5-ylidene]-propyl}-pyrrolidin-3-yl)-urea

### Step 1: 3-Ethylamino-pyrrolidine-1-carboxylic acid tert-butyl ester

To a solution of (R)- 3-amino-1-N-Boc pyrrolidine (1 eq, 5.37 mmol) in methanol (20 mL) was added acetaldehyde (0.95 eq) and the resulting mixture was heated to 80 °C and stirred for 2 h., then stirred at room temperature overnight. Sodium borohydride (0.95 eq, 5.1 mmol) was added and reaction mixture was stirred for an additional 2h. The reaction mixture was concentrated, dissolved in ethyl acetate and washed with saturated aqueous sodium bicarbonate. The combined organics were washed with brine and dried over sodium sulfate. The crude product was purified on silica using hexane/ethyl acetate (10%) to hexane /ethyl acetate (50%). ¹H-NMR (CDCl₃) δ: 1.05 (3H, t), 1.45 (9H, s), 1.60-1.75 (1H, m), 2.00-2.10 (1H, m), 2.68 (4H, q), 2.90-3.15 (1H, m), 3.25-3.65 (4H, m). ESI-MS m/z : 215 (M + 1), UV retention time: 0.75 min.

### Step 2: 3-[3-(4-Chloro-phenyl)-1-ethyl-ureido]-pyrrolidine-1-carboxylic acid tert-butyl ester

To a solution of 3-ethylamino-pyrrolidine-1-carboxylic acid tert-butyl ester (0.33 g, 1.5 mmol) in DMF (5.0 mL) was added drop wise a solution of 4-chlorophenyl isocyanate (0.23 g, 1.51 mmol) in DMF (2.5 mL) at room temperature and the resulting mixture was stirred for 3 h. The reaction mixture was concentrated *in vacuo* and the crude product was purified on silica using hexane/ethyl acetate (50%) followed by ethyl acetate (100%) to give the desired product as white foamy solid.
ESI-MS m/z : 368 (M + 1), UV retention time: 2.61 min.

### Step 3: 3-(4-Chloro-phenyl)-1-ethyl-1-pyrrolidin-3-yl-urea

A solution of 3-[3-(4-chloro-phenyl)-1-ethylureido]-pyrrolidine-1-carboxylic acid tert-butyl ester (0.4 g, 1.08 mmol) in dichloromethane (5.0 ml) cooled at 0 °C was treated with trifluoroacetic acid (20%) and warmed to room temperature and stirred for 2 h. The reaction was concentrated *in vacuo,* diluted with ethyl acetate and free based with 10% aqueous sodium bicarbonate. The combined organics were dried over sodium sulfate. The product was used without any further purification. ESI-MS m/z : 268 (M +1), UV retention time: 1.02 min.

### Step 4 : 3-(4-Chloro-phenyl)-1-ethyl-1-(1-{3-[7-(1-hydroxy-1-methyl-ethyl)-11H-10-oxa-1-aza-dihenzo [a,d]cyclohepten-5-ylidene]-propyl}-pyrrolidin-3-yl)-urea

To a solution of 3-(4-chloro-phenyl)-1-ethyl-1-pyrrolidin-3-yl-urea (0.21 g, 0.8 mmol) in acetonitrile:water (4:1) was added potassium carbonate (0.16 g, 0.8 mmol) and (E)- 2-[5-(3-bromo-propylidene)-5,11-dihydro-10-oxa-1-aza-dibenzo[a,d]cyclohepten-7-yl]-propan-2-ol (0.15 g, 0.4 mmol) and the resulting mixture was stirred at 50 °C for 24 h. The reaction mixture was concentrated in *vacuo*, diluted with ethyl acetate, and dried over sodium sulfate. The crude product was purified by flash chromatography on silica using gradient elution from ethyl acetate/methanol (5%) to ethyl acetate/methanol (10%) to give the desired product as yellow solid. ¹H-NMR (CDCl₃) δ: 1.05 (3H, t), 1.40 (6H, s), 1.80 (1H, m), 2.20-2.40 (3H, m), 2.50-3.00 (8H, m), 3.40 (4H, q and m), 4.20 (1H, m), 5.20 (2H, br, s), 6.20 (1H, t), 6.65(1H, d), 7.00 (2H, dd), 7.20-7.40 (5H, m), 7.70 (1H, dd), 8.50 (1H, dd) 9.80 (1H, br s). ESI-MS m/z : 561 (M + 1), UV retention time: 1.45 min.

### Example 33

### 2-(5-{3-[3-(4-Chloro-benzyloxy)-pyrrolidin-1-yl]-propylidene}-5,11-dihydro-10-oxa-1-aza-dibenzo[a,d]cyclohepten-7-yl)-propan-2-ol

### Step 1: (R) -3-(4-Chloro-benzyloxy)-pyrrolidine-1-carboxylic acid tert-butyl ester

A solution of (R)-3-hydroxy-pyrrolidine-1-carboxylic acid tert-butyl ester (1.14 mmol), 1.0 eq) in THF (2.5 mL) was added to a suspension of sodium hydride (1.15 eq) in THF (2.5 mL) cooled at 0 °C and the resulting mixture was stirred for 45 min. A solution of 4-chlorobenzyl bromide (1.31 mmol, 1.15 eq) in THF (2.0 mL) was then added drop wise and reaction mixture was slowly warmed to room temperature and stirred for 18 h. The reaction mixture was quenched with water (1.0 ml) and diluted with ethyl acetate. The combined organics were dried over sodium sulfate. The crude product was purified on silica using gradient elution from hexane/ethyl acetate (10%) to hexane/ethyl acetate (30%). ¹H-NMR (CDCL₃) δ: 1.45 (9H, s), 1.80-2.05 (2H, m), 3.30-3.55 (4H, m), 4.05 (1H, m), 4.45 (2H, s), 7.15-7.30 (4H, m). ESI-MS m/z:311 (M + 1), UV retention time: 2.94 min.

### Step 2: (R)-3-(4-Chloro-benzyloxy)-pyrrolidine

A solution of (R)-3-(4-chlorobenzyloxy)-pyrrolidine-1-carboxylic acid tert-butyl ester (0.88 mmol) in dichloromethane cooled at 0°C was treated with trifluoroacetic acid (20%) and warmed to room temperature and stirred for 2 h. The reaction mixture was concentrated *in vacuo,* diluted with ethyl acetate and free based with 10% aqueous sodium bicarbonate. The combined organics were dried over sodium sulfate. The product was used without any further purification. ESI-MS m/z : 211(M + 1), UV retention time: 0.97 min.

### Step 3: 2-(5-{3-[3-(4-Chloro-benzyloxy)-pyrrolidin-1-yl]-propylidene}-5,11-dihydro-10-oxa-1-aza-dibenzo[a,d]cyclohepten-7-yl)-propan-2-ol

To a solution of (R)-3-(4-chloro-benzyloxy)-pyrrolidine (0.88 mmol, 2.0 eq) in acetonitrile:water (4:1) was added potassium carbonate (0.88 mmol, 2.0 eqv) and (E)-2-[5-(3-bromo-propylidene)-5,11-dihydro-10-oxa-1-aza-dibenzo[a,d]cyclohepten-7-yl]-propan-2-ol and the resulting mixture was stirred at room temperature for 24 h. The reaction mixture was concentrated *in vacuo,* diluted with ethyl acetate, and dried over sodium sulfate. The crude product was purified by Reverse Phase HPLC. ¹H-NMR (CDCl₃) δ: 1.40 (6H, s), 1.15-1.35 (1H, m), 1.85-2.05 (1H, m), 2.20-2.40 (1H, m), 2.35-2.65 (8H, m), 3.90-4.10 (1H, m), 4.30- 4.50 (2H, d), 5.10-5.30 (2H, br, s), 6.05 (1H, t), 6.50 (1H, dd), 7.10-7.50 (4H, m), 7.70 (1H, dd), 8.50 (1H, dd). ESI-MS m/z : 505 (M + 1), UV retention time: 1.52 min.

### Example 34

### 2-[5-(3-{3-[(4-Chloro-benzyl)-isobutyl-amino]-pyrrolidin-1-yl}-propylidene)-5,11-dihydro-10-oxa-1-aza-dibenzo[a,d]cyclohepten-7-yl]-propan-2-ol

### Step 1: 3-(4-Chloro-benzylamino)-pyrrolidine-1-carboxylic acid tert-butyl ester

(R)- 3-amino-1-N-Boc pyrrolidine (1.0 eq, 2.68 mmol), 4-chlorobenzyl bromide (1.0 eq, 2.68 mmol), and potassium carbonate (1.75 eq) were mixed in ethanol (10 mL) and the resulting mixture was stirred at room temperature overnight. The reaction mixture was concentrated *in vacuo,* taken up in ethyl acetate, filtered and concentrated. The crude product was purified by flash chromatography on silica using ethyl acetate. ESI-MS m/z : 311 (M + 1), UV retention time: 1.18 min.

### Step 2: 3-[(4-Chloro-benzyl)-isobutyl-amino]-pyrrolidine-1-carboxylic acid tert-butyl ester

To a solution of 3-(4-chloro-benzylamino)-pyrrolidine-1-carboxylic acid tert-butyl ester (0.4 g, 1.28 mmol) and isobutyraldehyde (0.14 g, 1.92 mmol) in dichloroethane was added sodium triacetoxy borohydride (0.81 g, 3.85 mmol) and catalytic acetic acid. The resulting reaction mixture was stirred at room temperature overnight. The reaction mixture was concentrated, dissolved in dichloromethane and washed with saturated aqueous sodium bicarbonate. The combined organics were washed with brine and dried over sodium sulfate. The crude product was purified on silica using gradient elution from hexane/ethyl acetate (10%) to hexane/ethyl acetate (50%). ¹H-NMR (CDCl₃) δ: 0.80 (6H, 2 x d), 1.45 (9H, s), 1.60-1.90 (2H, m), 2.20 (2H, d), 3.00-3.70 (7H, m), 7.20 (4H,s). ESI-MS m/z : 367 (M+1), UV retention time: 1.89 min.

### Step 3: (4-Chloro-benzyl)-isobutyl-pyrrolidin-3-yl-amine

To a solution of 3-[(4-chloro-benzyl)-isobutyl-amino]-pyrrolidine-1-carboxylic acid tert-butyl ester in dichloromethane cooled at 0 °C was added trifluoroacetic acid (20%) and warmed to room temperature and stirred for 2 h. The reaction mixture was concentrated *in vacuo,* diluted with ethyl acetate and free based with 10% aqueous sodium bicarbonate. The combined organics were dried over sodium sulfate and concentrated. The product was used without any further purification.
ESI-MS m/z : 267(M + 1), UV retention time: 0.93 min.

### Step 4: 2-[5-(3-{3-[(4-Chloro-benzyl)-isobutyl-amino]-pyrrolidin-1-yl}-propylidene)-5,11-dihydro-10-oxa-1-aza-dibenzo[a,d]cyclohepten-7-yl]-propan-2-ol

To a solution of (4-chloro-benzyl)-isobutyl-pyrrolidin-3-yl-amine (0.16 g, 0.6 mmol) in acetonitrile:water (4:1) was added potassium carbonate (0.8 mmol) and (E)-2-[5-(3-bromo-propylidene)-5,11-dihydro-10-oxa-1-aza-dibenzo[a,d]cyclohepten-7-yl]-propan-2-ol (0.15 g, 0.4 mmol) and the resulting mixture was stirred at 50 °C for 24 h. The reaction was concentrated, diluted with ethyl acetate, and dried over sodium sulfate. The crude product was purified by flash chromatography using ethyl acetate/methanol (5%). ¹H-NMR (CDCl₃) δ: 0.80 (6H, d), 1.55(6H, s), 1.60-1.90 (4H, m), 2.15 (1H, d), 2.20-2.60 (6H, m), 3.25-3.60 (2H, m), 5.30 (2H, br, s), 6.10 (1H, t), 6.80 (1H, dd), 7.15-7.30 (6H, m), 7.40 (1H, d), 7.55 (1H, dd), 8.45 (1H, dd). ESI-MS m/z : 560 (M + 1), UV retention time: 1.66 min.

### Example 35

### 2-[5-(3-{3-[(4-Chloro-benzyl)-isopropyl-amino]-pyrrolidin-1-yl}-propylidene)-5,11-dihydro-10-oxa-1-aza-dibenzo[a,d]cyclohepten-7-yl]-propan-2-ol

### Step 1: 3-Isopropylamino-pyrrolidine-1-carboxylic acid tert-butyl ester

To a solution of (R)-3-amino-1-N-Boc pyrrolidine (1.0 eq, 2.68 mmol) in methanol (15 mL) was added acetone (1.07 eq), sodium cyano borohydride (2.0 eq) and few drops of acetic acid and the resulting mixture was stirred at room temperature overnight. The reaction mixture was concentrated dissolved in dichloromethane and washed with saturated aqueous sodium bicarbonate. The combined organics were washed with brine and dried over sodium sulfate. The crude product was purified on silica using gradient elution from hexane/ethyl acetate (5%) to hexane/ethyl acetate (30%). ¹H-NMR (CDCl₃) δ: 1.05 (6H, d), 1.45 (9H, s), 1.60 (1H, m), 2.80-3.00 (2H, m), 3.20-3.70 (4H, m). ESI-MS m/z : 229 (M + 1), UV retention time: 0.77 min.

### Step 2: 3-[(4-Chloro-benzyl)-isopropyl-amino]-pyrrolidine-1-carboxylic acid tert-butyl ester

To a solution of isopropyl-pyrrolidin-3-yl-amine (0.3 g, 1.31 mmol) in dichloroethane (5.0 ml) was added 4-chlorobenzaldehyde (0.22 g, 1.57 mmol), sodium triacetoxy borohydride (0.83 g, 3.93 mmol) and catalytic acetic acid and the resulting reaction mixture was stirred at room temperature overnight. The reaction mixture was concentrated, dissolved in dichloromethane and washed with 10% aqueous sodium bicarbonate. The combined organics were washed with brine and dried over sodium sulfate. The crude product was purified on silica using hexane/ethyl acetate (50%).¹H-NMR (CDCl₃) δ: 1.05 (6H, d), 1.45 (9H, s), 1.60 (1H, m), 2.80-3.00 (2H, m), 3.20-3.70 (4H, m), 7.20 (4H, m). ESI-MS m/z : 353(M + 1), UV retention time: 1.49 min.

### Step 3: (4-Chloro-benzyl)-isopropyl-pyrrolidin-3-yl-amine

A solution of 3-[(4-chloro-benzyl)-isopropyl-amino]-pyrrolidine-1-carboxylic acid tert-butyl ester in dichloromethane cooled at 0 °C was treated with trifluoroacetic acid (20%) and warmed to room temperature and stirred for 2 hours. The reaction mixture was concentrated *in vacuo,* diluted with ethyl acetate and free based with 10% aqueous sodium bicarbonate. The combined organics were dried over sodium sulfate. The product was used without any further purification. ESI-MS m/z : 253 (M + 1, UV retention time: 0.59 min.

### Step 4: 2-[5-(3-{3-[(4-Chloro-benzyl)-isopropyl-amino]-pyrrolidin-1-yl}-propylidene)-5,11-dihydro-10-oxa-1-aza-dibenzo[a,d]cyclohepten-7-yl]-propan-2-ol

To a solution of (4-chloro-benzyl)-isopropyl-pyrrolidin-3-yl-amine (0.68 mmol, 1.7 eq) in acetonitrile:water (4:1) was added potassium carbonate (0.8 mmol, 2 eq) and (E)-2-[5-(3-bromo-propylidene)-5,11-dihydro-10-oxa-1-aza-dibenzo[a,d]cyclohepten-7-yl]-propan-2-ol (0.4 mmol, 1.0 eq) and the resulting mixture was stirred at 50 °C for 24 h. The reaction mixture was concentrated *in vacuo,* diluted with ethyl acetate, and dried over sodium sulfate. The crude product was purified by flash chromatography using ethyl acetate/methanol (5%). ¹H-NMR (CDCl₃) δ: 0.85 (6H, d), 1.50-1.90 (10H, m), 2.10-2.60 (8H, m), 3.50 (3H, m), 5.30 (2H, br, s), 6.10 (1H, t), 6.80 (1H, dd), 7.10-7.40 (8H, m), 7.50 (1H, d), 8.50 (1H, dd). ESI-MS m/z : 546 (M + 1), UV retention time: 1.40 min.

### Example 36

### 2-[(4-Chloro-benzyl)-(1-{3-[7-(1-hydroxy-1-methyl-ethyl)-11H-10-oxa-1-aza dibenzo[a,d]cyclohepten-5-ylidene]-propyl}-pyrrolidin-3-yl)-amino]-acetamide

### Step 1: 3-(Carbamoylmethyl-amino)-pyrrolidine-1-carboxylic acid tert-butyl ester

To a solution of (R)-3-amino-1-N-Boc pyrrolidine (0.5 g, 2.68 mmol) and 2-bromo acetamide (0.44 g, 3.21 mmol) in DMF (6.0 mL) was added potassium carbonate (1.11 g, 8.04 mmol) and the reaction mixture was stirred at room temperature overnight. The reaction mixture was concentrated and re-dissolved in dichloromethane. Potassium carbonate was filtered off and filtrate was concentrated and purified using ethyl acetate/methanol (5%). ¹H-NMR (CDCl₃) δ: 1.50 (9H, s), 1.60-1.80 (2H, m), 2.10 (1H, m), 3.10-3.60 (6H, m), 5.65 (1H, br, s), 6.90 (1H, br, s). ESI-MS m/z : 367(M + 1), UV retention time: 0.61 min.

### Step 2: 3-[Carbamoylmethyl-(4-chloro-benzyl)-amino]-pyrrolidine-1-carboxylic acid tert-butyl ester

To a solution of 3-(carbamoylmethyl-amino)-pyrrolidine-1-carboxylic acid tert-butyl ester (0.49 g, 2.03 mmol) in dichloroethane (5.0 mL) was added 4-chlorobenzaldehyde 0.34 g, 2.44 mmol), sodium triacetoxy borohydride (0.86 g, 4.06 mmol) and catalytic acetic acid and the resulting mixture was stirred at room temperature overnight. The reaction mixture was concentrated, dissolved in dichloromethane and washed with 10% aqueous sodium bicarbonate. The combined organics were washed with brine and dried over sodium sulfate. The crude product was purified on silica using ethyl acetate. ¹H-NMR (CDCl₃) δ: 1.50 (9H, s), 1.60-1.80 (2H, m), 2.10 (1H, m), 3.10-3.60 (8H, m), 5.65 (1H, br, s), 6.90 (1H, br, s) 7.20-7.40 (4H, m). ESI-MS m/z : 367(M + 1), UV retention time: 1.94 min.

### Step 3: 2-[(4-Chloro-benzyl)-pyrrolidin-3-yl-amino]-acetamide

A solution of 3-[carbamoylmethyl-(4-chloro-benzyl)-amino]-pyrrolidine-1-carboxylic acid tert-butyl ester (0.49 g, 1.32 mmol) in dichloromethane cooled at 0 °C was treated with trifluoroacetic acid (20%). The resulting mixture was warmed to room temperature and stirred for 2 h. The reaction mixture was concentrated in *vacuo,* diluted with ethyl acetate and free based with 10% aqueous sodium bicarbonate. The combined organics were dried over sodium sulfate. The product was used without any further purification ESI-MS m/z : 267 (M + 1), UV retention time: 0.82 min.

### Step 4: 2-[(4-Chloro-benzyl)-(1-{3-[7-(1-hydroxy-1-methyl-ethyl)-11H-10-oxa-1-aza dibenzo[a,d] cyclohepten-5-ylidene]-propyl}-pyrrolidin-3-yl)-amino]-acetamide

To a solution of 2-[(4-chloro-benzyl)-pyrrolidin-3-yl-amino]-acetamide (0.21 g, 0.8 mmol) in acetonitrile:water (4:1) was added potassium carbonate (0.6 mmol) and (E)-2-[5-(3-bromo-propylidene)-5,11-dihydro-10-oxa-1-aza-dibenzo[a,d]cyclohepten-7-yl]-propan-2-ol (0.15 g, 0.4 mmol) and the resulting mixture was stirred at 50 °C for 24 h. The reaction mixture was concentrated in *vacuo,* diluted with ethyl acetate, and dried over sodium sulfate. The crude product was purified by HPLC. ¹H-NMR (CDCl₃) δ: 1.35 (6H, s), 1.55-1.75 (1H, m), 1.80-2.00 (1H, m), 2.20-2.85 (8H, m), 2.90-3.10 (2H, d), 3.30-3.50 (1H, m), 3.60 (1H, d), 4.80-5.30 (2H, br, s), 6.10 (1H, t), 6.70 (1H, dd), 7.00-7.45 (7H, m), 7.70 (1H, dd), 8.50 (1H, dd). ESI-MS m/z : 562 (M + 1), UV retention time: 1.31 min.

### Example 37

### 2-[(4-Chloro-benzyl)-(1-{3-[7-(1-hydroxy-1-methyl-ethyl)-11H-10-oxa-1-aza-dibenzo[a,d] cyclohepten-5-ylidene]-propyl}-pyrrolidin-3-yl)-amino]-N-ethyl-acetamide

### Step 1: 3-(Methoxycarbonylmethyl-amino)-pyrrolidine-1-carboxylic acid tert-butyl ester

To a solution of (R)-3-amino-1-N-Boc pyrrolidine (0.5 g, 2.68 mmol) and methyl bromo acetate (0.49 g, 2.95 mmol) in DMF (6.0 mL) was added potassium carbonate (1.11 g, 8.04 mmol) and the reaction mixture was stirred at room temperature overnight. The reaction mixture was concentrated and re-dissolved in dichloromethane. Potassium carbonate was filtered off and filtrate was concentrated and purified using gradient elution from hexane/ethyl acetate (10%) to hexane/ethyl acetate (50%).
H-NMR (CDCl₃) δ: 1.40 (9H, s), 1.70 (2H, m), 2.00 (1H, m), 3.20-3.60 (5H, m), 3.79 (3H, br s). ESI-MS m/z : 258(M + 1), UV retention time: 0.74 min.

### Step 2: 3-[(4-Chloro-benzyl)-methoxycarbonylmethyl-amino]-pyrrolidine-1-carboxylic acid tert-butyl ester

To a solution of 3-(methoxycarbonylmethyl-amino)-pyrrolidine-1-carboxylic acid tert-butyl ester (0.5 g, 2.03 mmol) in dichloroethane (5.0 mL) was added 4-chlorobenzaldehyde (0.34 g, 2.44 mmol), sodium triacetoxy borohydride (0.86 g, 4.06 mmol) and catalytic acetic acid and the resulting mixture was stirred at room temperature overnight. The reaction mixture was concentrated, dissolved in dichloromethane and washed with 10% aqueous sodium bicarbonate. The combined organics were washed with brine and dried over sodium sulfate. The crude product was purified on silica using ethyl acetate. ¹H-NMR (CDCl₃) δ: 1.40 (9H, s), 1.80 (1H, m), 2.10 (1H, m), 3.00-3.80 (7H, m), 4.70(1H, br,s), 7.20 (4H,m). ESI-MS m/z: 383(M + 1), UV retention time: 2.91min.

### Step 3: 3-[Carboxymethyl-(4-chloro-benzyl)-amino]-pyrrolidine-1-carboxylic acid tert-butyl ester

A solution of 3-[(4-chloro-benzyl)-methoxycarbonylmethyl-amino]-pyrrolidine-1-carboxylic acid tert-butyl ester (0.5 g, 1.31 mmol) in methanol (4.00 mL) and 1.0 N NaOH (aqueous) (4.00 mL) was heated to reflux at 80 °C for 2.5 h. The reaction mixture was concentrated, acidified to pH 5.0 using 1.0 N HCl and extracted with dichloromethane(3X). The product was used for the next step without further purification. ESI-MS m/z: 369(M + 1), UV retention time: 1.85 min.

### Step 4: 3-[(4-Chloro-benzyl)-ethylcarbamoylmethyl-amino]-pyrrolidine-1-carboxylic acid tert-butyl ester

To a solution of 3-[carboxymethyl-(4-chloro-benzyl)-amino]-pyrrolidine-1-carboxylic acid tert-butyl ester (0.48 g, 1.30 mmol) in tetrahydrofuran (10 mL) was added EDCI (0.39 g, 1.95 mmol), HOBT (0.26 g, 1.95 mmol), and stirred for 30 min., followed by the addition of N-methyl morpholine (0.43 mL, 3.90 mmol) and N-ethyl amine (0.1 mL, 1.95 mmol). The reaction mixture was stirred at room temperature for 18 h. Upon concentration, the residue was dissolved in ethyl acetate and washed with saturated aqueous sodium bicarbonate. The combined organics were washed with brine and dried over sodium sulfate. The crude product was purified on silica using gradient elution from hexane/ethyl acetate (50%) to ethyl acetate (100%).
ESI-MS m/z: 396 (M + 1), UV retention time: 2.18 min.

### Step 5: 2-[(4-Chloro-benzyl)-pyrrolidin-3-yl-amino]-N-ethyl-acetamide

A solution of 3-[(4-chloro-benzyl)-ethylcarbamoylmethyl-amino]-pyrrolidine-1-carboxylic acid tert-butyl ester (0.12 g, 0.31 mmol) in dichloromethane cooled at 0 °C was treated with trifluoroacetic acid (20%) The resulting mixture was warmed to room temperature and stirred for 2 hours. The reaction mixture was concentrated *in vacuo,* diluted with ethyl acetate and free based with 10% aqueous sodium bicarbonate. The combined organics were dried over sodium sulfate. The product was used without any further purification. ESI-MS m/z : 296 (M + 1), UV retention time: 1.02 min.

### Step 6: 2-[(4-Chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amino]-N-ethyl-acetamide

To a solution of 2-[(4-chloro-benzyl)-pyrrolidin-3-yl-amino]-N-ethyl-acetamide (0.09 g, 0.31 mmol) in acetonitrile:water (4:1) was added potassium carbonate (0.07 g, 0.51 mmol) and (E)-2-[5-(3-bromo-propylidene)-5,11-dihydro-10-oxa-1-aza-dibenzo[a,d]cyclohepten-7-yl]-propan-2-ol (0.095 g, 0.25 mmol) and the resulting mixture was stirred at 50 °C for 24 h. The reaction mixture was concentrated *in vacuo,* diluted with ethyl acetate, and dried over sodium sulfate. The crude product was purified on silica using ethyl acetate. ¹H-NMR (CDCl₃) δ: 0.90 (3H, t), 1.40(6H, s), 1.60 (1H, m), 1.85 (1H, m), 2.10-2.55 (8H, m), 2.90-3.10 (4H, m),3.25-3.40 (4H, m), 3.55 (2H, d), 4.95 (2H, s), 5.10 (2H, br, s), 6.10 (1H, t), 6.70 (1H, dd), 7.15-7.25 (1H, dd), 7.25-7.45 (4H, m), 7.55-7.75 (2H, m), 8.50 (1H, dd). ESI-MS m/z: 589 (M + 1), UV retention time: 1.42 min.

### Example 38

### 2-[5-(3-{3-[(4-Chloro-benzyl)-(2-hydroxy-ethyl)-amino]-pyrrolidin-1-yl}-propylidene)-5,11-dihydro-10-oxa-1-aza-dibenzo[a,d]cyclohepten-7-yl]-propan-2-ol

### Step 1: 3-[(4-Chloro-benzyl)-(2-hydroxy-ethyl)-amino]-pyrrolidine-1-carboxylic acid tert-butyl ester

To a solution of 3-[{4-chloro-benzyl)-methoxycarbonylmethyl-amino]-pyrrolidine-1-carboxylic acid tert-butyl ester (0.44 g, 1.15 mmol) in methanol cooled to 0 °C was added sodium borohydride (0.13 g, 3.45 mmol) and the resulting mixture was heated to 65 °C for 16h. Additional sodium borohydride (0.26 g, 6.90 mmol) was added to the reaction mixture and stirred further at 65 °C for 18h. The reaction mixture was concentrated, dissolved in ethyl acetate and washed with saturated aqueous sodium bicarbonate. The aqueous layer was extracted with ethyl acetate. The combined organics were dried over sodium sulfate. The crude product was purified on silica using gradient elution from hexane/ethyl acetate (50%) to ethyl acetate (100%). ESI-MS m/z :355 (M + 1), UV retention time: 1.27 min.

### Step 2: 2-[(4-Chloro-benzyl)-pyrrolidin-3-yl-amino]-ethanol

A solution of 3-[(4-chloro-benzyl)-(2-hydroxy-ethyl)-amino]-pyrrolidine-1-carboxylic acid tert-butyl ester (0.22 g, 0.6 mmol) in dichloromethane cooled at 0 °C was treated with trifluoroacetic acid (20%). The resulting mixture was warmed to room temperature and stirred for 2 h. The reaction mixture was concentrated in *vacuo,* diluted with ethyl acetate and free based with 10% aqueous sodium bicarbonate. The combined organics were dried over sodium sulfate. The product was used without any further purification. ESI-MS m/z :255 (M + 1), UV retention time: 0.34 min.

### Step 3: 2-[5-(3-{3-[(4-Chloro-benzyl)-(2-hydroxy-ethyl)-amino]-pyrrolidin-1-yl}-propylidene)-5,11-dihydro-10-oxa-1-aza-dibenzo[a,d]cyclohepten-7-yl]-propan-2-ol

To a solution of 2-[(4-chloro-benzyl)-pyrrolidin-3-yl-amino]-ethanol (0.15 g, 0.6 mmol) in acetonitrile:water (4:1) was added potassium carbonate (0.11 g, 0.8 mmol) and (E)-2-[5-(3-bromo-propylidene)-5,11-dihydro-10-oxa-1-aza-dibenzo[a,d]cyclohepten-7-yl]-propan-2-ol (0.15 g, 0.4 mmol) and the resulting mixture was stirred at 50 °C for 24 h. The reaction mixture was concentrated *in vacuo,* diluted with ethyl acetate and dried over sodium sulfate. The crude product was purified on silica. ¹H-NMR (CDCl₃) δ: 1.40 (6H, s), 1.60 (1H, m), 1.80 (1H, m), 2.10-2.60 (8H, m), 3.20-3.40 (6H, m), 3.50 (2H, m), 5.20 (2H, br, s), 6.15 (1H, t), 6.70 (1H, t), 7.18-7.42 (7H, m), 7.70 (1H, dd), 8.50 (1H, dd). ESI-MS m/z : 548 (M + 1), UV retention time: 1.26 min.

### Example 39

### 3-[{4-Chloro-benzyl)-(1-{3-[7-(1-hydroxy-1-methyl-ethyl)-11H-10-oxa-1-aza-dibenzo[a,d]cyclo hepten-5-ylidene]-propyl}-pyrrolidin-3-yl)-amino]-propionic acid methyl ester

### Step 1: 3-(2-Methoxycarbonyl-ethylamino)-pyrrolidine-1-carboxylic acid tert-butyl ester

To a solution of (R)-3-amino-1-N-Boc pyrrolidone (0.5 g, 2.68 mmol) and 3-bromo propionate (0.49 g, 2.95 mmol) in DMF (6.0 mL) was added potassium carbonate (1.11 g, 8.04 mmol) and the resulting mixture was stirred at room temperature overnight. The reaction mixture was concentrated and re-dissolved in dichloromethane. Potassium carbonate was filtered off and filtrate was concentrated and crude product was used further in the next step. ESI-MS m/z : 272 (M + 1), UV retention time: 1.28 min.

### Step 2: 3-[(4-Chloro-benzyl)-(2-methoxycarbonyl-ethyl)-amino]-pyrrolidine-1-carboxylic acid tert-butyl ester

To a solution of 3-(2-methoxycarbonyl-ethylamino)-pyrrolidine-1-carboxylic acid tert-butyl ester (0.73 g, 2.68 mmol) in dichloroethane (10.0 mL) was added 4-chlorobenzaldehyde (0.42 g, 2.95 mmol), sodium triacetoxy borohydride (1.71g, 8.04 mmol) and catalytic acetic acid and the resulting mixture was stirred at room temperature overnight. The reaction mixture was concentrated, dissolved in dichloromethane and washed with saturated aqueous sodium bicarbonate. The combined organics were washed with brine and dried over sodium sulfate. The crude product was purified on silica using gradient elution from hexane /ethyl acetate (15%) to hexane/ethyl acetate (50%). ¹H-NMR (CDCl₃) δ: 1.35 (9H, s), 1.80 (1H, m), 1.90 (1H, m), 2.40 (2H, t), 2.85 (2H, t), 3.00-3.80 (7H, m), 4.70(1H, d), 7.20 (4H, m). ESI-MS m/z: 397 (M + 1), UV retention time: 1.99 min.

### Step 3: 3-[(4-Chloro-benzyl)-pyrrolidin-3-yl-amino]-propionic acid methyl ester

A solution of 3-[(4-chloro-benzyl)-(2-methoxycarbonyl-ethyl)-amino]-pyrrolidine-1-carboxylic acid tert-butyl ester (0.08 g, 0.2 mmol) in dichloromethane cooled at 0 °C was treated with trifluoroacetic acid (20%). The resulting mixture was warmed to room temperature and stirred for 2 h. The reaction mixture was concentrated *in vacuo,* diluted with ethyl acetate and free based with 10% aqueous sodium bicarbonate. The combined organics were dried over sodium sulfate. The product was used without any further purification. ESI-MS m/z : 297 (M + 1), UV retention time: 1.05 min.

### Step 4: 3-[(4-Chloro-benzyl)-(1-{3-[7-(1-hydroxy-1-methyl-ethyl)-11H-10-oxa-1-aza-dibenzo[a,d] cyclohepten-5-ylidene]-propyl}-pyrrolidin-3-yl)-amino]-propionic acid methyl ester

To a solution of 3-[(4-chloro-benzyl)-pyrrolidin-3-yl-amino]-propionic acid methyl ester (0.06 g, 0.2 mmol) in acetonitrile:water (3:1) was added potassium carbonate (0.056 g, 0.4 mmol) and (E)-2-[5-(3-bromo-propylidene)-5,11-dihydro-10-oxa-1-aza-dibenzo[a,d]cyclohepten-7-yl]-propan-2-ol (0.065 g, 0.17 mmol) and reaction stirred at 50 °C for 24 h. The reaction mixture was concentrated *in vacuo,* diluted with ethyl acetate and dried over sodium sulfate. The crude product was purified by flash chromatography on silica using ethyl acetate/methanol (5%). ¹H-NMR (CDCl₃) δ: 1.40 (6H, s), 1.60 (1H, m), 1.80 (1H, m), 2.10-2.60 (8H, m), 3.20-3.40 (6H, m), 3.50 (2H, m; 2H, s), 5.20 (2H, br s), 6.15 (1H, t), 6.70 (1H, t), 7.18-7.42 (7H, m), 7.70 (1H, dd), 8.50 (1H, dd). ESI-MS m/z: 590 (M + 1), UV retention time: 1.51min.

### Example 40-51

### General Procedures:

The N-BOC protected amine (0.0565 - 0.6911 mmol) was subjected to IN NaOH, extracted with dichloromethane, washed with brine, dried over magnesium sulfate and filtered. The solution was evaporated via steady N₂ airflow. To the residue was added the corresponding bromide (0.8 eq), potassium carbonate (1.0 eq), 11 mLs of acetonitrile and 2.75 mLs water. The resulting solution was agitated via and orbital shaker for 48 hours. The vials were transferred to a heating plate and stirred at 50°C for 48 hours. The solutions were quenched with a 1:1 brine/water mixture, washed with 1NNaOH, dried over magnesium sulfate, filtered and concentrated via steady N₂ airflow. The residue was dissolved in dichloromethane and subjected to column chromatography (SiO₂, Biotage 12M column, 95% dichloromethane/5%methanol with 0.1% triethyl amine) to afford the desired product.

### Example 40

### N-(4-Chloro-benzyl)-N-(1-{3-[7-(1-hydroxy-1-methyl-ethyl)-11H-10-oxa-1-aza-dibenzo[a,d]cyclohepten-5-ylidene]-propyl}-pyrrolidin-3-yl)-2-methyl-butyramide

To a heated (77°C) stirring solution of 1-(4-chloro-phenyl)-2-imidazolidin-1-yl-4-methyl-hexan-3-one(110mg, 0.374mmol), 2,6-lutidiene(130 µL, 11.229 mmol) and 5 mL isopropanol was added 2-[5-(3-bromo-propylidene)-5,11-dihydro-10-oxa-1-aza-dibenzo[a,d]cyclohepten-7-yl]-propan-2-ol (70 mg, 0.187 mmol) portion-wise over a 30 minute period. The resulting solution was monitored using thin layer chromatography and allowed to stir at 77°C for 16 hours. The solution was concentrated *in vacuo* at 35°C. The residue was dissolved in ethyl acetate and subjected to column chromatography (SiO₂, Biotage 12M column, gradient elution 100% ethyl acetate → 92% ethyl acetate/8% methanol with 1 % triethyl amine) to afford 54 mg of 1-(4-chloro-phenyl)-2-(3-{3-[7-(1-hydroxy-1-methyl-ethyl)-11H-10-oxa-1-aza-dibenzo[a,d]cyclohepten-5-ylidene]-propyl}-imidazolidin-1-yl)-4-methyl-hexan-3-one as a tan foam. LC/MS: *t*_{UV} = 1.68 min, M/Z = 588 amu.

### Example 41

### N-(4-Chloro-benzyl)-N-(1-{3-[7-(1-hydroxy-1-methyl-ethyl)-11H-10-oxa-1-aza-dibenzo[a,d]cyclohepten-5-ylidene]-propyl}-pyrrolidin-3-yl)-acetamide.

LC/MS: *t*_{UV} = 1.66 min, M/Z = 546 amu. ¹H NMR ((CD₃)₂SO): δ 8.54 (1H,d), 7.70 (1H,m), 7.44 (3H,m), 7.28 (3H,m), 7.13 (1H,m), 6.76 (1H,d), 6.12 (1H,d), 5.21 (3H,m), 5.00 (1H,s), 4.63 (2H,m), 4.50 (2H,m), 3.12 (2H,d), 2.39 (4H,m), 2.27 (2H,m), 2.18 (3H,s), 1.90 (5H,m), 1.60 (2H,m),1.43 (6H,s), 1.21 (2H,t), 0.92 (1H,m).

### Example 42

### N-(4-Chloro-benzyl)-N-(1-{3-[7-(1-hydroxy-1-methyl-ethyl)-11H-10-oxa-1-aza-dibenzo[a,d]cyclohepten-5-ylidene]-propyl}-pyrrolidin-3-yl)-isobutyramide.

LC/MS: *t*_{UV} = 1.56 min, M/Z = 574 amu.. ¹H NMR ((CD₃)₂SO): δ 8.51 (1H,d), 7.66 (1H,d), 7.42 (3H,m), 7.25 (2H,t), 7.12 (1H,m), 7.04 (1H,d), 6.74 (1H,d), 6.11 (1H,m), 5.18 (2H,m), 4.98 (2H,s), 4.91 (2H,m), 4.48 (1H,s), 3.04 (1H,m), 2.40 (5H,m), 2.14 (4H,m), 1.40 (6H,s), 1.04 (3H,d), 0.90 (3H,m).

### Example 43

### N-(4-Chloro-benzyl)-N-(1-{3-[7-(1-hydroxy-1-methyl-ethyl)-11H-10-oxa-1-aza-dibenzo[a,d]cyclohepten-5-ylidene]-propyl}-pyrrolidin-3-yl)-propionamide.

LC/MS: *t*_{UV} = 1.49 min, M/Z = 560 amu. ¹H NMR ((CD₃)₂SO): δ 8.48 (1H,d), 7.64 (1H,d), 7.40 (3H,m), 7.27 (2H,t), 7.07 (2H,m), 6.72 (1H,d), 6.10 (1H,m), 5.17 (3H,m), 4.96 (2H,s), 4.52 (4H,m), 2.35 (3H,m), 2.12 (5H,m), 1.52 (1H,m), 1.34 (6H,s), 1.00 (2H,t), 0.89 (1H,m).

### Example 44

### Cyclopentanecarboxylic acid (4-chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amide.

LC/MS: *t*_{UV} = 1.73 min, M/Z = 600 amu. ¹H NMR ((CD₃)₂SO): δ 8.50 (1H,d), 7.65 (1H,d), 7.40 (3H,m), 7.24 (2H,t), 7.11 (1H,m), 7.02 (1H,d), 6.72 (1H,d), 6.11 (1H,m), 5.14 (3H,m), 4.96 (1H,s), 4.82 (1H,m), 4.64 (2H,m), 4.46 (1H,s), 3.13 (1H,m), 2.34 (3H,t), 2.19 (2H,m), 2.06 (2H,m), 1.71 (2H,m), 1.57 (7H,m), 1.39 (6H,s).

### Example 45

### Cyclohexanecarboxylic acid (4-chloro-benzyl)-(1-{3-[7-(1-hydroxy-1-methyl-ethyl)-11H-10-oxa-1-aza-dibenzo[a,d]cyclohepten-5-ylidene]-propyl}-pyrrolidin-3-yl)-amide.

LC/MS: *t*_{UV} = 1.79 min, M/Z = 614 amu. ¹H NMR ((CD₃)₂SO): δ 8.48 (1H,d), 7.64 (1H,d), 7.39 (3H,m), 7.23 (2H,t), 7.10 (1H,m), 7.00 (1H,d), 6.69 (1H,d), 6.08 (1H,m), 5.13 (2H,m), 4.95 (1H,s), 4.58 (2H,m), 4.43 (1H,s), 2.73 (1H,m), 2.34 (3H,m), 2.12 (4H,m), 1.67 (7H,m), 1.38 (6H,s), 1.29 (3H,m), 1.03 (3H,m),.

### Example 46

### 2-Ethyl-hexanoicacid(4-chloro-benzyl)-(1-{3-[7-(1-hydroxy-1-methyl-ethyl)-11H-10-oxa-1-aza-dibenzo[a,d]cyclohepten-5-ylidene]-pxopyl}-pyrrolidin-3-yl)-amide.

LC/MS: *t*_{UV} = 1.92 min, M/Z = 630 amu. ¹H NMR ((CD₃)₂SO): δ 8.44 (1H,m), 7.61 (1H,m), 7.35 (3H,m), 7.20 (2H,m), 7.11 (1H,m), 7.02 (1H,d), 6.68 (1H,d), 6.05 (1H,m), 5.10 (3H,m), 4.91 (1H,s), 4.61 (2H,m), 4.44 (1H,s), 2.66 (3H,m), 2.32 (3H,m), 2.07 (4H,m), 1.46 (3H,m), 1.34 (6H,s), 1.17 (4H,m), 0.94 (2H,m), 0.75 (4H,m), 0.60 (1H,m).

### Example 47

1-(4-Chloro-benzyl)-1-(1-{3-[7-(1-hydroxy-1-methyl-ethyl)-1H-10-oxa-1-aza-dibenzo[a,d]cyclohepten-5-ylidene]-propyl}-pyrrolidin-3-yl)-3-isopropyl-urea. LC/MS: *t*_{UV} = 1.53 min, M/Z = 589 amu. ¹H NMR ((CD₃)₂SO): δ 8.50 (1H,m), 7.69 (1H,d), 7.40 (2H,m), 7.32 (2H,d), 7.19 (3H,m), 6.71 (1H,d), 6.09 (1H,m), 5.17 (2H,m), 4.95 (1H,s), 4.41 (2H,s), 4.12 (1H,m), 3.69 (1H,m), 3.05 (2H,m), 2.78 (2H,m), 2.58 (1H,m), 2.26 (2H,m), 1.91 (2H,m), 1.61 (1H,m), 1.38 (6H,s), 1.16 (1H,m), 0.92 (6H,m).

### Example 48

### 1-(4-Chloro-benzyl)-3-cyclohexyl-1-(1-{3-[7-(1-hydroxy-1-methyl-ethyl)-11H-10-oxa-1-aza-dibenzo[a,d]cyclohepten-5-ylidene]-propyl}-pyrrolidin-3-yl)-urea.

LC/MS: *t*_{UV} = 1.70 min, M/Z = 629 amu. ¹H NMR ((CD₃)₂SO): δ 8.50 (1H,d), 7.69 (1H,d), 7.40 (2H,m), 7.32 (4H,m), 7.18 (3H,m), 6.70 (1H,d), 6.10 (1H,m), 5.16 (2H,m), 4.95 (1H,s), 4.43 (2H,m), 4.02 (1H,m), 3.14 (1H,m), 2.71 (3H,m), 2.22 (3H,m), 1.87 (2H,m), 1.62 (5H,m), 1.38 (6H,s), 0.98 (8H,m).

### Example 49

### 1-(4-Chloro-benzyl)-3-ethyl-1-(1-{3-[7-(1-hydroxy-1-methyl-ethyl)-11H-10-oxa-1-aza-dib enzo [a,d] cyclohepten-5-ylidene]-propyl}-pyrrolidin-3-yl)-urea.

LC/MS: *t*_{UV} = 1.46 min, M/Z = 5.75 amu. ¹H NMR ((CD₃)₂SO): δ 8.52 (1H,d), 7.70 (1H,d), 7.43 (2H,m), 7.33 (2H,d), 7.23 (1H,dd), 7.17 (2H,d), 6.73 (1H,d), 6.13 (1H,t), 5.19 (2H,m), 4.98 (1H,s), 4.43 (2H,s), 4.20 (1H,m), 2.96 (4H,m), 2.71 (2H,m), 2.26 (3H,m), 1.95 (2H,m), 1.61 (1H,m), 1.41 (6H,s), 1.19 (1H,m), 1.01 (1H,m), 0.85 (2H,m).

### Example 50

### 1-(4-Chloro-benzyl)-1-(1-{3-[7-(1-hydroxy-1-methyl-ethyl)-11H-10-oxa-1-aza-dibenzo[a,d]cyclohepten-5-ylidene]-propyl}-pyrrolidin-3-yl)-3-(1,1,3,3-tetramethyl-butyl)-urea. LC/MS: t_{UV} = 2.05 min, M/Z = 659 amu.

### Example 51

### N-(4-Chloro-benzyl)-N-(1-{3-[7-(1-hydroxy-1-methyl-ethyl)-11H-10-oxa-1-aza-dibenzo[a,d]cyclohepten-5-ylidene]-propyl}-pyrrolidin-3-yl)-methanesulfonamide. LC/MS: t_{UV} = 1.49 min, M/Z = 582 amu.

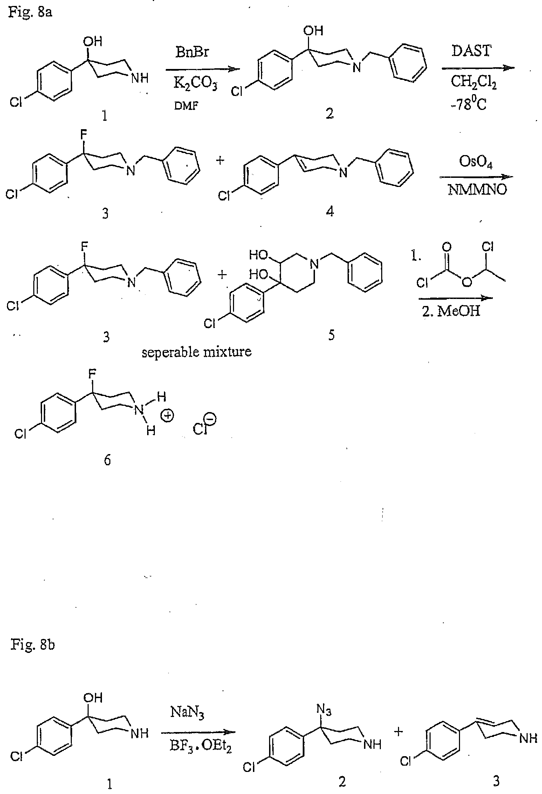

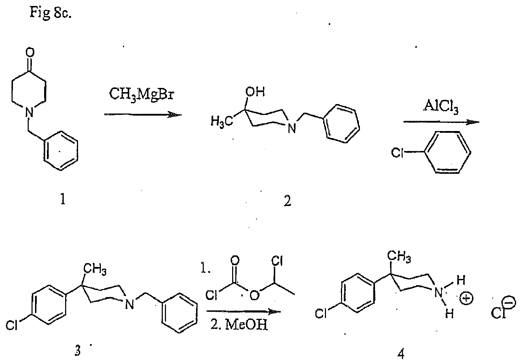

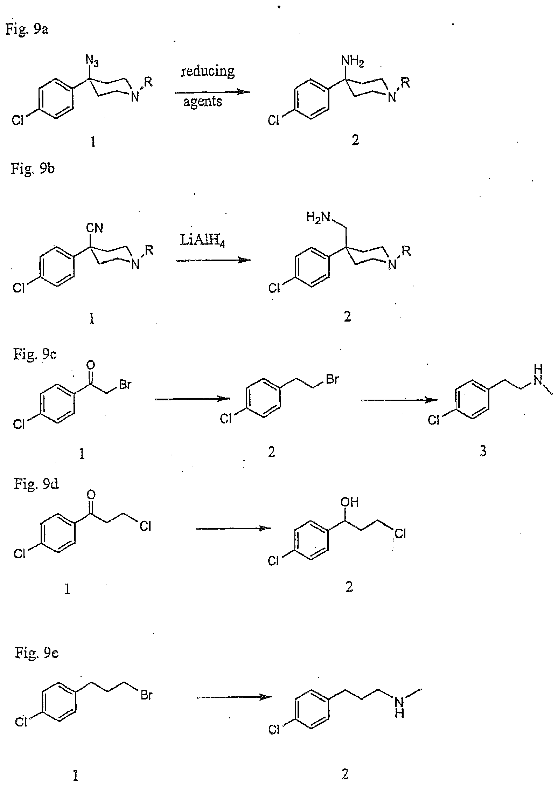

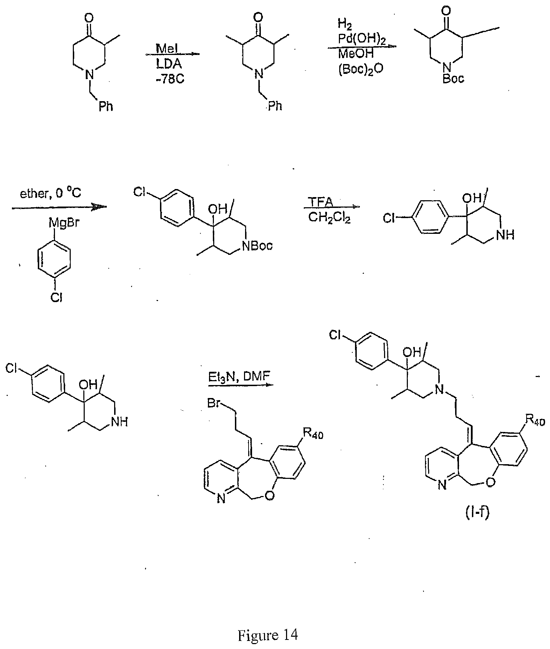

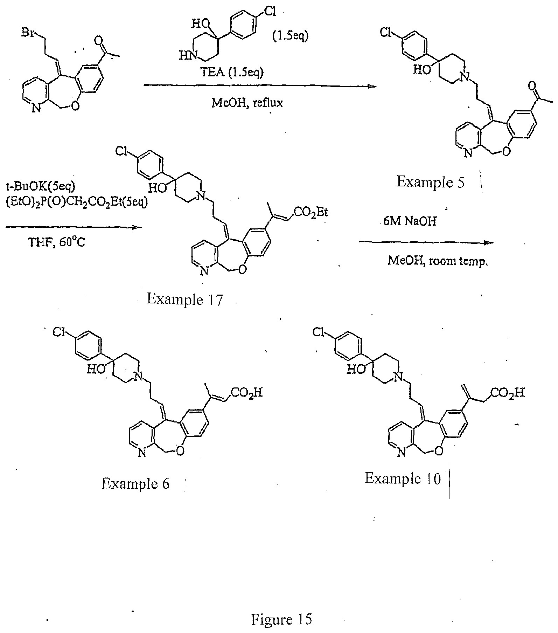

Additional compounds of the invention can be prepared by the schemes set forth in Figures 1-5, 7, 8A-8C, 9A-9E, 10a-10d and 12-15 and by the procedures described herein.

## Claims

1. A compound having the formula: 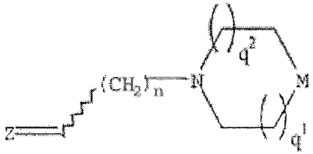 or physiologically acceptable salt thereof, wherein:
n is an integer from one to four;
M is >CR¹R²;
q¹ is zero;
q² is one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, a halogen, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group,
-O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 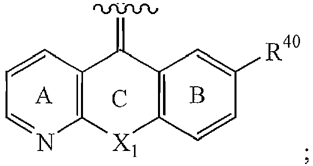 X₁ is -S-, -CH₂-, -CH₂-CH₂-, -CH₂-S-, -S-CH₂-, -O-CH₂-, -CH₂-O-, -NR_{c}-CH₂-, -CH₂-NR_{c}-, -SO-CH₂-, -CH₂-SO-, -S(O)₂-CH₂-, -CH₂-S(O)₂-, -CH=CH-, -NR_{c}-CO-, a bond, -O-, or -CO-NR_{c}-;
R_{c} is -H, an aliphatic group, a substituted aliphatic group, an aromatic group, a substituted aromatic group, a benzyl group or a substituted benzyl group;
said acyl group is an aliphatic carbonyl, aromatic carbonyl, aliphatic sulfonyl or aromatic sulfonyl
said aliphatic group is a C₁-C₆ alkyl, alkenyl or alkynyl;
said aromatic group is selected from the group consisting of phenyl, 1-naphthyl, 2-naphthyl, 1-anthracyl, 2-anthracyl, *N*-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl, 5-oxazolyl, tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 1-isoquinolinyl, 3-quinolinyl, 1-isoindolyl, 3-isoindolyl, acridinyl, 3-benzisoxazolyl, benzocyclopentyl, and benzocyclohexyl;
said non-aromatic heterocyclic group is a five to eight-membered non-aromatic ring which contains one or more heteroatoms independently selected from the group consisting of nitrogen, oxygen and sulfur;
said substituted aliphatic group is substituted with one or more substituents selected from the group consisting of oxo group, epoxy group, non-aromatic heterocyclic ring, benzyl group, substituted benzyl group, aromatic group or substituted aromatic group, electron withdrawing group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said substituted non-aromatic heterocyclic ring is substituted with one or more substituents selected from the group consisting of =O, =S, electron withdrawing group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CB₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said substituted aromatic group and substituted benzyl group are substituted with one or more substituents selected from the group consisting of electron withdrawing group, halo, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group) and -Q-(CH₂)ₚ-(non-aromatic heterocyclic group);
said electron withdrawing group is alkylimine, alkylsulfonyl, carboxamido, carboxylic alkyl esters, -CH=NH, or -NO₂;
Q is -O-, -S-, -S(O)-, -S(O)₂-, -OS(O)₂-, -C(O)-, -OC(O)-, -C(O)O-, -C(O)C(O)-O-, -O-C(O)C(O)-, -NHC(O)-, -OC(O)NH-, -NH-C(O)-NH-, -S(O)₂NH-, -NHS(O)₂-, -C(NR²³)NHNH-, -NHNHC(NR²³)-, -NR²⁴C(O)- or -NR²⁴S(O)₂-;
R²⁰, R²¹ and R²² are independently -H, an aliphatic group, an aromatic group, a non-aromatic heterocyclic group, -NHC(O)-O-(aliphatic group), -NHC(O)-O-(aromatic group) or -NHC(O)-O-(non-aromatic heterocyclic group) or R²¹ and R²², taken together with the nitrogen atom to which they are bonded, form a substituted or unsubstituted non-aromatic heterocyclic ring;
R²³ is -H, an aliphatic group, a benzyl group, an aryl group or non-aromatic heterocyclic group;
R²⁴ and R²⁵ are independently -H, -OH, an aliphatic group, a substituted aliphatic group, a benzyl group, an aryl group, non-aromatic heterocyclic group or R²⁴ and R²⁵ taken together with the nitrogen atom to which they are bonded can form a substituted or unsubstituted non-aromatic heterocyclic ring;
R⁶⁰ is a -H, -OH, -NH₂, an aromatic group or a substituted aromatic group;
t is zero to three;
u is zero to three;
p is one to five; and
R⁴⁰ is selected from the group consisting of 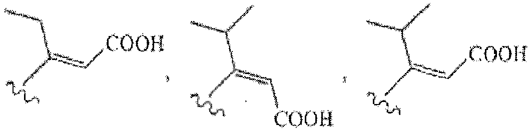 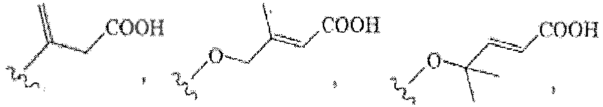 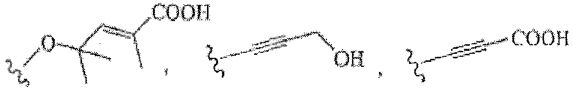 and

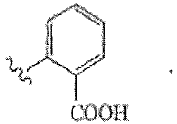

2. The compound of Claim 1 wherein:
R¹ is -H or -OH; and
R² is a substituted aromatic group.

3. The compound of Claim 2 wherein R² is phenyl substituted with a halogen.

4. The compound of Claim 3 wherein R² is 4-chlorophenyl.

5. The compound of Claim 4 wherein n is 2, X₁ is -CH₂-O-, and R¹ is -OH.

6. A pharmaceutical composition comprising a compound of any one of claims 1 to 5 and a physiologically acceptable carrier.

7. A compound according to any one of claims 1 to 5 for use in treating a disease selected from the group consisting of arthritis, atherosclerosis, arteriosclerosis, restenosis, ischemia/reperfusion injury, diabetes mellitus, psoriasis, multiple sclerosis, inflammatory bowel diseases, rejection of a transplanted orgari or tissue, graft versus host disease, allergy and asthma.

## Patentansprüche

1. Verbindung der Formel: 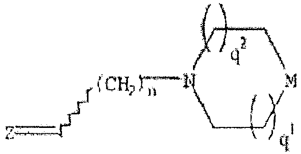 oder ein physiologisch annehmbares Salz davon, worin:
n eine ganze Zahl von eins bis vier ist;
M >CR¹R² ist;
q¹ null ist;
q² eins ist;
R¹ -H, -OH, -N₃, ein Halogen, eine aliphatische Gruppe, eine substituierte aliphatische Gruppe, eine Aminoalkylgruppe, -0-(aliphatische Gruppe), -0-(substituierte aliphatische Gruppe), -SH, -S-(aliphatische Gruppe), -S-(substituierte aliphatische Gruppe), -OC(0)-(aliphatische Gruppe), -O-C(O)-(substituierte aliphatische Gruppe), -C(0)0-(aliphatische Gruppe), -C(0)0-(substituierte aliphatische Gruppe), -COOH, -CN, -CO-NR³R⁴, -NR³R⁴ ist oder R¹ eine kovalente Bindung zwischen dem Ringatom bei M und einem benachbarten Kohlenstoffatom in dem Ring, welcher M enthält, ist;
R² -OH, ein Halogen, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nichtaromatische heterocyclische Gruppe, eine substituierte nichtaromatische heterocyclische Gruppe, -0-(substituierte oder unsubstituierte aromatische Gruppe) oder -0-(substituierte oder unsubstituierte aliphatische Gruppe) ist;
R³, R⁴, R⁵ und R⁶ unabhängig -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nichtaromatische heterocyclische Gruppe oder eine substituierte nichtaromatische heterocyclische Gruppe sind; oder
R¹ und R², R³ und R⁴ oder R⁵ und R⁶ zusammengenommen mit dem Atom, an das sie gebunden sind, einen substituierten oder unsubstituierten nichtaromatischen carbocyclischen oder heterocyclischen Ring bilden;
Z 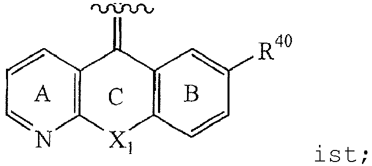 X, -S-, -CH₂-, -CH₂-CH₂-, -CH₂-S-, -S-CH₂-, -O-CH₂-, -CH₂-O-, -NR_{c}-CH₂-, -CH₂-NR_{c}-, -SO-CH₂-, -CH₂-SO-, -S(O)₂-CH₂-, -CH₂-S(O)₂-, -CH=CH-, -NR_{c}-CO-, eine Bindung, -0- oder -CO-NR_{c}- ist;
R_{c} -H, eine aliphatische Gruppe, eine substituierte aliphatische Gruppe, eine aromatische Gruppe, eine substituierte aromatische Gruppe, eine Benzylgruppe oder eine substituierte Benzylgruppe ist;
die Acylgruppe eine aliphatische Carbonyl-, aromatische Carbonyl-, aliphatische Sulfonyl- oder aromatische Sulfonylgruppe ist;
die aliphatische Gruppe eine C₁-C₆-Alkyl-, -Alkenyl- oder -Alkinylgruppe ist;
die aromatische Gruppe ausgewählt ist aus der Gruppe, bestehend aus Phenyl, 1-Naphthyl, 2-Naphthyl, 1-Anthracyl, 2-Anthracyl, N-Imidazolyl, 2-Imidazolyl, 4-Imidazolyl, 5-Imidazolyl, 2-Thienyl, 3-Thienyl, 2-Furanyl, 3-Furanyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, 3-Pyrazolyl, 4-Pyrazolyl, 5-Pyrazolyl, 2-Pyrazinyl, 2-Thiazolyl, 4-Thiazolyl, 5-Thiazolyl, 5-Tetrazolyl, 2-Oxazolyl, 4-Oxazolyl, 5-Oxazolyl, Tetrahydronaphthyl, 2-Benzothienyl, 3-Benzothienyl, 2-Benzofuranyl, 3-Benzofuran-yl, 2-Indolyl, 3-Indolyl, 2-Chinolinyl, 3-Chinolinyl, 2-Benzothiazolyl, 2-Benzooxazolyl, 2-Benzimidazolyl, 1-Isochinolinyl, 3-Chinolinyl, 1-Isoindolyl, 3-Isoindolyl, Acridinyl, 3-Benzisoxazolyl, Benzocyclopentyl und Benzocyclohexyl;
die nichtaromatische heterocyclische Gruppe ein fünf- bis achtgliedriger nichtaromatischer Ring ist, der ein oder mehr Heteroatom(e) enthält, unabhängig ausgewählt aus der Gruppe, bestehend aus Stickstoff, Sauerstoff und Schwefel;
die substituierte aliphatische Gruppe substituiert ist mit einem oder mehreren Substituenten, ausgewählt aus der Gruppe, bestehend aus Oxogruppe, Epoxygruppe, nichtaromatischem heterocyclischem Ring, Benzylgruppe, substituierter Benzylgruppe, aromatischer Gruppe oder substituierter aromatischer Gruppe, elektronenziehender Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nichtaromatische heterocyclische Gruppe) und -Q-(CH₂)ₚ-(nichtaromatische heterocyclische Gruppe);
der substituierte nichtaromatische heterocyclische Ring substituiert ist mit einem oder mehreren Substituenten, ausgewählt aus der Gruppe, bestehend aus =0, =S, elektronenziehender Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nichtaromatische heterocyclische Gruppe) und -Q-(CH₂)ₚ-(nichtaromatische heterocyclische Gruppe);
die substituierte aromatische Gruppe und substituierte Benzylgruppe substituiert sind mit einem oder mehreren Substituenten, ausgewählt aus der Gruppe, bestehend aus elektronenziehender Gruppe, Halogen, Azido, -CN, -CONR²⁴R²⁵, -NR²⁴R²⁵, -OS(O)₂NR²⁴R²⁵, -S(O)₂NR²⁴R²⁵, -SO₃H, Guanidino, Oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nichtaromatische heterocyclische Gruppe) und -Q-(CH₂)ₚ-(nichtaromatische heterocyclische Gruppe);
die elektronenziehende Gruppe Alkylimin, Alkylsulfonyl, Carboxamido, Carbonsäurealkylester, -CH=NH oder -NO₂ ist; Q -O-, -S-, -S(O)-, -S(O)₂-, -OS(O)₂-, -C(O)-, -OC(O)-, -C(O)O-, -C(O)C(O)-O-, -O-C(O)C(O)-, -NHC(O)-, -OC(O)NH-, -NH-C(O)-NH-, -S(O)₂NH-, -NHS(O)₂-, -C(NR²³)NHNH-, -NHNHC(NR²³)-, -NR²⁴C(O)- oder -NR²⁴S(O)₂- ist;
R²⁰, R²¹ und R²² unabhängig -H, eine aliphatische Gruppe, eine aromatische Gruppe, eine nichtaromatische heterocyclische Gruppe, -NHC(0)-0-(aliphatische Gruppe), -NHC(O)-O-(aromatische Gruppe) oder -NHC(O)-O-(nichtaromatische heterocyclische Gruppe) sind oder R²¹ und R²², zusammengenommen mit dem Stickstoffatom, an das sie gebunden sind, einen substituierten oder unsubstituierten nichtaromatischen heterocyclischen Ring bilden;
R²³ -H, eine aliphatische Gruppe, eine Benzylgruppe, eine Arylgruppe oder nichtaromatische heterocyclische Gruppe ist;
R²⁴ und R²⁵ unabhängig -H, -OH, eine aliphatische Gruppe, eine substituierte aliphatische Gruppe, eine Benzylgruppe, eine Arylgruppe, nichtaromatische heterocyclische Gruppe sind oder R²⁴ und R²⁵ zusammengenommen mit dem Stickstoffatom, an das sie gebunden sind, einen substituierten oder unsubstituierten nichtaromatischen heterocyclischen Ring bilden können;
R⁶⁰ ein -H, -OH, -NH₂, eine aromatische Gruppe oder eine substituierte aromatische Gruppe ist;
t null bis drei ist;
u null bis drei ist;
p eins bis fünf ist und
R⁴⁰ ausgewählt ist aus der Gruppe, bestehend aus 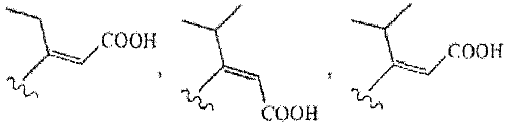 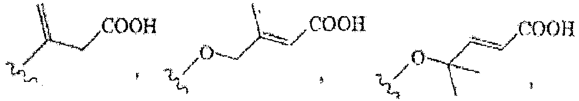 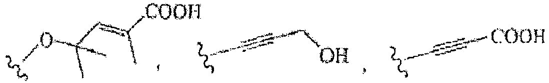 und

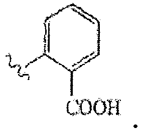

2. Verbindung nach Anspruch 1, worin:
R¹ -H oder -OH ist und
R² eine substituierte aromatische Gruppe ist.

3. Verbindung nach Anspruch 2, worin R² Phenyl ist, substituiert mit einem Halogen.

4. Verbindung nach Anspruch 3, worin R² 4-Chlorphenyl ist.

5. Verbindung nach Anspruch 4, worin n 2 ist, X₁ -CH₂-O- ist und R¹ -OH ist.

6. Pharmazeutische Zusammensetzung, umfassend eine Verbindung nach einem der Ansprüche 1 bis 5 und einen physiologisch annehmbaren Trägerstoff.

7. Verbindung nach einem der Ansprüche 1 bis 5 zur Verwendung bei der Behandlung einer Erkrankung, ausgewählt aus der Gruppe, bestehend aus Arthritis, Atherosklerose, Arteriosklerose, Restenose, Ischämie/Reperfusionsschaden, Diabetes mellitus, Psoriasis, multipler Sklerose, entzündlichen Darmerkrankungen, Abstoßung eines transplantierten Organs oder Gewebes, Graft-versus-Host-Disease, Allergie und Asthma.

## Revendications

1. Composé de formule : 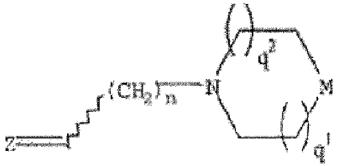 ou sel physiologiquement acceptable d'un tel composé, formule dans laquelle :
n est un nombre entier valant de un à quatre ;
M est >CR¹R² ;
q¹ est égal à zéro ;
q² est égal à un ;
R¹ est -H, -OH, -N₃, un atome d'halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), -C(O)O-(groupe aliphatique), -C(O)O-(groupe aliphatique substitué), -COOH, -CN, -CO-NR³R⁴, -NR³R⁴ ou R¹ est une liaison covalente entre l'atome formant le cycle en M et un atome de carbone adjacent dans le cycle qui contient M ;
R² est -OH, un atome d'halogène, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué, -O-(groupe aromatique substitué ou non substitué) ou -O-(groupe aliphatique substitué ou non substitué) ;
R³, R⁴, R⁵ et R⁶ représentent indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ;
ou
R¹ et R², R³ et R⁴, ou R⁵ et R⁶, pris ensemble avec l'atome auquel ils sont liés, forment un cycle carbocyclique ou hétérocyclique non aromatique substitué ou non substitué ;
Z est : 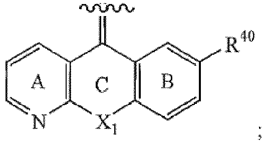 X₁ est -S-, -CH₂-, -CH₂-CH₂-, -CH₂-S-, -S-CH₂-, -O-CH₂-, -CH₂-O-, -NRC-CH₂-, -CH₂-NR_{c}-, -SO-CH₂-, -CH₂-SO-, -S(O)₂-CH₂-, -CH₂-S(O)₂-, -CH=CH-, -NR_{c}-CO-, une liaison, -O-, ou -CO-NR_{c}- ;
R_{c} est -H, un groupe aliphatique, un groupe aliphatique substitué, un groupe aromatique, un groupe aromatique substitué, un groupe benzyle ou un groupe benzyle substitué ;
ledit groupe acyle est un groupe carbonyle aliphatique, carbonyle aromatique, sulfonyle aliphatique ou sulfonyle aromatique ;
ledit groupe aliphatique est un groupe alkyle, alcényle ou alcynyle en C₁-C₆ ;
ledit groupe aromatique est choisi dans l'ensemble constitué par les groupes phényle, 1-naphtyle, 2-naphtyle, 1-anthracyle, 2-anthracyle, *N-*imidazolyle, 2-imidazolyle, 4-imidazolyle, 5-imidazolyle, 2-thiényle, 3-thiényle, 2-furanyle, 3-furanyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 4-pyridazinyle, 3-pyrazolyle, 4-pyrazolyle, 5-pyrazolyle, 2-pyrazinyle, 2-thiazolyle, 4-thiazolyle, 5-thiazolyle, 5-tétrazolyle, 2-oxazolyle, 4-oxazolyle, 5-oxazolyle, tétrahydronaphtyle, 2-benzothiényle, 3-benzothiényle, 2-benzofuranyle, 3-benzofuranyle, 2-indolyle, 3-indolyle, 2-quinolinyle, 3-quinolinyle, 2-benzothiazolyle, 2-benzo-oxazolyle, 2-benzimidazolyle, 1-isoquinolinyle, 3-quinolinyle, 1-iso-indolyle, 3-iso-indolyle, acridinyle, 3-benzisoxazolyle, benzocyclopentyle et benzocyclohexyle ;
ledit groupe hétérocyclique non aromatique est un cycle non aromatique à cinq à huit chaînons qui contient un ou plusieurs hétéroatomes choisis indépendamment dans l'ensemble constitué par les atomes d'azote, d'oxygène et de soufre ;
ledit groupe aliphatique substitué est substitué par un ou plusieurs substituants choisis dans l'ensemble constitué par le groupe oxo, le groupe époxy, un cycle hétérocyclique non aromatique, le groupe benzyle, un groupe benzyle substitué, un groupe aromatique ou un groupe aromatique substitué, un groupe attirant les électrons, halogéno, azido, -CN, -CONR²⁴R²⁵, -NR²⁴R²⁵, -OS(O)₂NR²⁴_{R}²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique) et -Q-(CH₂)ₚ-(groupe hétérocyclique non aromatique) ;
ledit cycle hétérocyclique non aromatique substitué est substitué par un ou plusieurs substituants choisis dans l'ensemble constitué par =O, =S, un groupe attirant les électrons, halogéno, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)N^{R21}R²², =NR⁶⁰, -(O)u-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)t-NHC(O)O-R²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique) et -Q-(CH₂)ₚ-(groupe hétérocyclique non aromatique) ;
ledit groupe aromatique substitué et ledit groupe benzyle substitué sont substitués par un ou plusieurs substituants choisis dans l'ensemble constitué par un groupe attirant les électrons, halogéno, azido, -CN, -CONR²⁴R²⁵, -NR²⁴R²⁵, -OS(O)₂NR²⁴R²⁵, -S(O)₂NR²⁴R²⁵, -SO₃H, guanidino, oxalo, -C(=NR⁶⁰)NR²¹R²², =NR⁶⁰, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique) et -Q-(CH₂)ₚ-(groupe hétérocyclique non aromatique) ;
ledit groupe attirant les électrons est un groupe alkylimine, alkylsulfonyle, carboxamido, un groupe choisi parmi des esters alkyliques d'acides carboxyliques, -CH=NH ou -NO₂ ;
Q est -O-, -S-, -S(O)-, -S(O)₂-, -OS(O)₂-, -C(O)-, -OC(O)-, -C(O)O-, -C(O)C(O)-O-, -O-C(O)C(O)-, -NHC(O)-, -OC(O)NH-, -NH-C(O)-NH-, -S(O)₂NH-, -NHS(O)₂-, -C(NR²³)NHNH-, -NHNHC(NR²³)-, -NR²⁴C(O)- ou -NR²⁴S(O)₂- ;
R²⁰, R²¹ et R²² représentent indépendamment -H, un groupe aliphatique, un groupe aromatique, un groupe hétérocyclique non aromatique, -NHC(O)-O-(groupe aliphatique), -NHC(O)-O-(groupe aromatique) ou -NHC(O)-O-(groupe hétérocyclique non aromatique) ou R²¹ et R²², pris ensemble avec l'atome d'azote auquel ils sont liés, forment un cycle hétérocyclique non aromatique substitué ou non substitué ;
R²³ est -H, un groupe aliphatique, un groupe benzyle, un groupe aryle ou un groupe hétérocyclique non aromatique ;
R²⁴ et R²⁵ représentent indépendamment -H, -OH, un groupe aliphatique, un groupe aliphatique substitué, un groupe benzyle, un groupe aryle, un groupe hétérocyclique non aromatique ou R²⁴ et R²⁵, pris ensemble avec l'atome d'azote auquel ils sont liés, forment un cycle hétérocyclique non aromatique substitué ou non substitué ;
R⁶⁰ est -H, -OH, -NH₂, un groupe aromatique ou un groupe aromatique substitué ;
t vaut de zéro à trois ;
u vaut de zéro à trois ;
p vaut de un à cinq ; et
R⁶⁰ est choisi dans l'ensemble constitué par 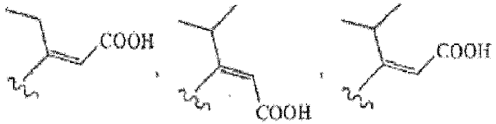 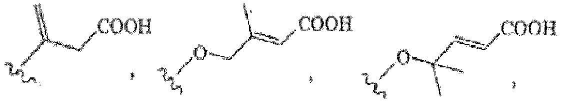 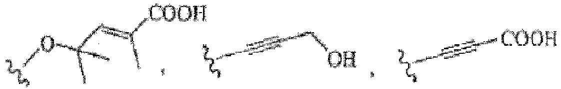 et

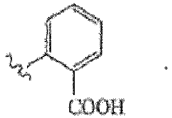

2. Composé selon la revendication 1, dans lequel
R¹ est -H ou -OH ; et
R² est un groupe aromatique substitué.

3. Composé selon la revendication 2, dans lequel R² est un groupe phényle substitué par un atome d'halogène.

4. Composé selon la revendication 3, dans lequel R² est le groupe 4-chlorophényle.

5. Composé selon la revendication 4, dans lequel n vaut 2, X₁ est -CH₂O-et R¹ est -OH.

6. Composition pharmaceutique comprenant un composé selon l'une quelconque des revendications 1 à 5 et un véhicule pharmaceutiquement acceptable.

7. Composé selon l'une quelconque des revendications 1 à 5, pour utilisation dans le traitement d'un maladie choisie dans l'ensemble constitué par l'arthrite, l'athérosclérose, l'artériosclérose, la resténose, des lésions dues à une ischémie/reperfusion, le diabète sucré, la psoriasis, la sclérose en plaques, les maladies intestinales inflammatoires, le rejet d'un organe ou tissu transplanté, le syndrome de réaction du greffon contre l'hôte, l'allergie et l'asthme.
